# EUROPÄISCHE PATENTSCHRIFT

(11) **EP 2 400 962 B1**
(45) Veröffentlichungstag und Bekanntmachung des Hinweises auf die Patenterteilung: **08.11.2017**
(21) Anmeldenummer: 10704932.2
(22) Anmeldetag: 18.02.2010
(51) Int. Cl.: A61K 31/196, A61K 31/44, A61K 31/519, A61K 45/06, A61P 1/00, A61P 11/00, A61P 17/00, A61P 19/00, A61P 25/00, A61P 29/00, A61P 35/00

(54) **ARZNEIMITTELKOMBINATIONEN ENTHALTEND PDE4-INHIBITOREN UND NSAIDS**
Medicinal combinations containing PDE4 inhibitors and NSAIDS
Combinaisons de médicaments contenant des inhibiteurs PDE4 et NSAID

(30) Priorität: 27.02.2009 EP 09153855; 22.07.2009 EP 09166131
(43) Veröffentlichungstag der Anmeldung: 04.01.2012
(73) Patentinhaber: Boehringer Ingelheim International GmbH, 55216 Ingelheim am Rhein (DE)
(72) Erfinder: NICKOLAUS, Peter, 55216 Ingelheim Am Rhein (DE); GOEGGEL, Rolf, 55216 Ingelheim Am Rhein (DE); PETER, Daniel, 55216 Ingelheim Am Rhein (DE)
(74) Vertreter: Simon, Elke Anna Maria
(86) Internationale Anmeldenummer: PCT/EP2010/052079
(87) Internationale Veröffentlichungsnummer: WO 2010/097334

(56) Entgegenhaltungen:
- WO-A-03/024489
- WO-A-2006/111549
- WO-A-2009/050248
- D Spina: "REVIEW PDE4 inhibitors: current status", British Journal of Pharmacology, vol. 155, 1 January 2008 (2008-01-01), pages 308-315, XP055254077,
- "6 Unwanted Side Effects" In: "Handbook of Experimental Pharmacology", 1 January 2011 (2011-01-01), XP055254088, vol. 204, pages 391-392,

## Beschreibung

Die vorliegende Erfindung betrifft neue Arzneimittelkombinationen, die neben einem oder mehreren PDE4-Inhibitoren wenigstens ein NSAID (= non-steroidal anti-inflammatory drug) (**2**) umfassen, Verfahren zu deren Herstellung sowie deren Verwendung zur Therapie von insbesondere Atemwegserkrankungen wie beispielsweise COPD, chronische Sinusitis und Asthma.

Die Erfindung betrifft insbesondere solche Arzneimittelkombinationen, die neben einem oder mehreren, bevorzugt einem PDE4-lnhibitor der allgemeinen Formel **1** 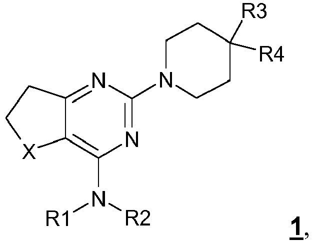
worin **X** SO ist und worin
R¹, R², R³ und R⁴ die in Anspruch 1 genannten Bedeutungen haben,
wenigstens ein NSAID (= non-steroidal anti-inflammatory drug) (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen, deren Herstellung und Verwendung zur Therapie von Atemwegserkrankungen.

### STAND DER TECHNIK

WO 2003/024489 offenbart Kombinationen von PDE4-Inhibitoren, insbesondere Roflumilast, mit NSAIDs, insbesondere Diclofenac. Dennoch sind die in WO 2003/024489 genannten PDE4-Inhibitoren strukturell völlig unterschiedlich zu den erfindungsgemäßen Dihydrothienopyrimidinen der Formel 1.

WO 2006/111549 offenbart Dihydrothienopyrimidine als PDE4-Inhibitoren, die sich von den erfindungsgemäßen Dihydrothienopyrimidinen der Formel 1 jedoch darin unterscheiden, dass sie mit einem Piperidin-Substituenten anstelle mit einem Piperazin-Substituenten substituiert sind, sowie Kombinationen derer mit anderen Wirkstoffen wie beispielsweise MRP4-Inhibitoren wie Diclofenac. WO 2009/050248 offenbart substituierte Piperidino-Dihydrothienopyrimidine der Formel **1** als PDE4-Inhibitoren, deren Herstellung sowie deren Verwendung zur Therapie von Atemwegserkrangungen, sowie Kombinationen derer mit anderen Wirkstoffen wie beispielweise MRP4-Inhibitoren wie Diclofenac, Ibuprofen oder Indomethacin. Weiterhin ist bekannt, dass viele PDE4-Inhibitoren der "1. Generation" wie beispielsweise Rolipram zu unerwünschten Nebenwirkungen führen. Es war folglich Aufgabe der vorliegenden Erfindung, ein Arzneimittel bzw. eine Arzneimittelkombination enthaltend einen PDE4-Inhibitor bereitzustellen, welches über ein geringes Nebenwirkungsprofil verfügt.

### BESCHREIBUNG DER ERFINDUNG

Überraschenderweise konnte nun gefunden werden, dass Arzneimittelkombinationen die neben einem PDE4-Inhibitor - insbesondere neben den als PDE4-Inhibitoren bekannten Piperidino-Dihydrothienopyrimidinsulfoxide der Formel **1**, in denen R¹, R², R³ und R⁴ die in Anspruch 1 genannten Bedeutungen haben (**1**) - auch wenigstens ein NSAID (**2**) enthalten, ein deutlich verringertes PDE4-vermitteltes Nebenwirkungsprofil aufweisen gegenüber einem Arzneimittel, das den entsprechenden PDE**4**-Inhibitor bzw. das entsprechende Piperidino-Dihydrothienopyrimidinsulfoxids der Formel **1** alleine enthält. Infolgedessen kann die Dosierung des entsprechenden Dihydrothienopyrimidinsulfoxids der Formel **1** (als PDE**4-**Inhibitor) deutlich höher ausfallen, wodurch sich dessen Wirksamkeit zur Therapie von beispielsweise Atemwegserkrankungen wie insbesondere COPD, chronischer Sinusitis und Asthma bei gleichzeitig niedrig bleibendem Nebenwirkungsprofil erhöht. Dies bedeutet demnach, dass sich das therapeutische Fenster für den verwendeten PDE4-Inhibitor vergrößert.

Die vorliegende Erfindung betrifft daher eine neuartige Arzneimittelkombination, die neben einem oder mehreren PDE4-Inhibitoren wenigstens ein NSAID (=non-steroidal anti-inflammatory drug) (**2**) umfasst.

Die vorliegende Erfindung betrifft vorzugsweise solche Arzneimittelkombinationen, die neben einer oder mehrerer, bevorzugt einer Verbindung der allgemeinen Formel **1** als PDE**4-**Inhibitor 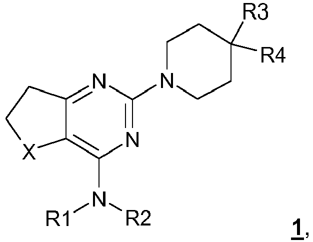 worin
- **X**: SO,
- **R¹**: H, C₁₋₆-Alkyl,
- **R²**: H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, C₆₋₁₀-Aryl, -Het, Hetaryl, einem monooder bicyclischem -C₃₋₁₀-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
- **Het**: ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält,
und wobei
- **Hetaryl**: ein fünf- bis zehngliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
- **Cycloalkyl**: gesättigt oder teilweise gesättigt sein kann,
wobei
**R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀-Aryl-C₁₋₆-alkylen, Hetaryl-C₁₋₆-alkylen-, Het-C₁₋₆-alkylen-, C₃₋₁₀-Cycloalkyl-C₁₋₆-alkylen-, ein mono- oder bicyclisches C₆₋₁₀-Aryl, Heteroaryl und ein -Het,
der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann, wobei
**R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀-Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃,-CO-N(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
oder
- **R²**: ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, -SO₂-NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -NH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl), -N(C₁₋₃-Alkyl)-CO-(C₁₋₆-Alkyl), -N(C₁₋₃-Alkyl)-CO-O-(C₆₋₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀-Aryl),-N(C₁₋₃-Alkyl)-CO-(C₁₋₃-Alkylen)-(C₆₋₁₀-Aryl), C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, Het, C₁₋₆-Alkyl, C₁₋₃-Fluoroalkyl, CF₃, CHF₂, CH₂F, C₆₋₁₀-Aryl-C₁₋₆-alkylen,
Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
- **NR¹R²**: gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
- **R³**: ein C₆₋₁₀-Aryl ist,
welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het,-CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁₀-Aryl-C₁₋₂-alkylen, Het-C₁₋₂-alkylen, -Het, -CO-Het, , CO-N(CH₃)-C₃₋₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist,
wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
- **R³**: ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1},-O-R^{2.1}, -COOR^{2.1}*,* SO₂-(CH₃), SO₂-(CH₂-CH₃), C₆₋₁₀-Aryl, Het, C₃₋₇-Cycloalkyl und Hetaryl substituiert sein kann,
der wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
oder worin
- **R³**: -O-R^{3.1},
wobei
**R^{3.1}** ein Rest ausgewählt aus der Gruppe bestehend aus -C₁₋₆-Alkyl, -C₆₋₁₀-Aryl,-C₁₋₃-Alkylen-C₆₋₁₀-Aryl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁₋₆-Alkylen)-Hetaryl,
-CO-N(C₁₋₃-Alkyl)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, C₆₋₁₀-Aryl, C₆₋₁₀-Aryl-C₁₋₂-alkylen, Het-C₁₋₂-alkylen, -CO-Het, Het, -CO-C₃₋₇-Cycloalkyl, -CO-N(C₁₋₃-Alkyl)-C₃₋₇-Cycloalkyl
C₃₋₇-Cycloalkyl, C₃₋₇-Cycloalkyl-C₁₋₂-alkylen, Hetaryl-C₁₋₂-alkylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann.
und worin
- **R⁴**: H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(C₁₋₃-Alkyl)-SO₂-(C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl), -C₁₋₃-Alkylen-NH-CO-(C₁₋₃-Alkylen)-O-(C₁₋₃-Alkyl), -CO-(C₆₋₁₀-Aryl), -(C₁₋₂Alkylen)-N(C₁₋₃-Alkyl)-CO-(C₁₋₂Alkylen)-O-(C₁₋₃-Alkyl),
wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆-Alkyl, -O-R^{2.1},-COOR^{2.1}, SO-R^{2.1}, SO₂-R^{2.1}, -C₁₋₃-alkylen-NR^{2.2}R^{2.3}, -NR^{2.2}R^{2.3}, C₆₋₁₀-Aryl, C₃₋₇-Cycloalkyl, Het und Hetaryl substituiert sein kann,
bedeuten, wenigstens ein NSAID (= non-steroidal anti-inflammatory drug) (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Bevorzugt sind Arzneimittelkombinationen, die neben einer oder mehrerer, bevorzugt einer Verbindung der Formel **1** als PDE4-Inhibitor, wobei
- **X**: SO,
- **R¹**: H
- **R²**: H ist oder C₁₋₆-Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
- **Het**: ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
wobei **R^{2.1}** H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂-alkylen,
-Het-C₁₋₂-alkylen, C₃₋₇-Cycloalkyl-C₁₋₂-alkylen, Phenyl, Hetaryl und ein Het, der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Alkyl), CO-R^{2.1} und COOR^{2.1},
der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
- **R²**: ein monocyclisches C₃₋₇ Cycloalkyl, das gegebenenfalls mit einem Rest ausgewählt aus der Gruppe bestehend aus C₁₋₂-Alkanol,
C₁₋₃-Fluoroalkyl, C₁₋₃-alkylen-OR^{2.1}, OR^{2.1}, COOR^{2.1}, SO₂-NR^{2.2}R^{2.3}, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
- **R²**: ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
- **R²**: ein Rest ausgewählt aus einer Gruppe bestehend aus Het und Hetaryl,
der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂-alkylen, -Het, -Hetaryl und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Butyl, Isobutyl, Phenyl und NR^{2.2}R^{2.3} substituiert sein kann,
und worin
- **R³**: ein Naphthalin oder Phenyl ist,
welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃; SO₂-CH₃, SO-CH₃, COOCH₃, COOCH₂CH₃, -CO-NH-(Methylen)-Hetaryl, -CO-NH-(Ethylen)-Hetaryl, -CO-NH-Hetaryl, -CO-N(CH₃)-Het,-CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het,
-NH-CO-Methyl , NCH₃-CO-Methyl, -NH-CO-Ethyl, NCH₃-CO-Ethyl, -NH-CO-Propyl, NCH₃-CO-Propyl, -NH-CO-Isopropyl, NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO-N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₃) und -N(CH₃)₂ substituiert sein kann,
wobei dieser Rest gegebenenfalls durch einen oder mehrere Reste ausgewählt aus der Gruppe bestehend aus OH, F, Cl, -CF₃, CHF₂, CH₂F, Oxo, Methyl und Phenyl substituiert sein kann
oder worin
- **R³**: ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₃)₂, -NH₂,-NH(CH₃), -N(CH₃)₂, Het und Hetaryl substituiert sein kann,
der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
oder worin
- **R³**: -O-R^{3.1},
wobei
**R^{3.1}** ein Rest ausgewählt aus der Gruppe bestehend aus -C₁₋₃-Alkyl, -Phenyl,-C₁₋₃-Alkylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl,
-CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N(C₅₋₇-Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂,
-Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl, , SO₂-Methyl, -SO₂-Ethyl, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇-Cycloalkyl, -CO-N(CH₃)-Cyclopropyl,
C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
- **R⁴**: H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl,
-CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇-Cycloalkyl, Het und Hetaryl substituiert sein kann,
bedeuten, wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Besonders bevorzugt sind weiterhin die obigen Arzneimittelkombinationen, die neben einer oder mehrerer, bevorzugt einer Verbindung der Formel **1** als PDE4-Inhibitor, wobei
- **R²**: ein Rest nach Formel **3** 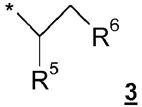
ist, worin
- **R⁶**: OH oder NH₂ ist und
worin
- **R⁵**: ein Rest ausgewählt aus der Gruppe bestehend aus C₁₋₄-Alkyl, einem fünf- bis sechsgliedrigen Heteroaryl mit 1, 2 oder 3 Heteroatomen aus der Gruppe S, O und N und Phenyl, welcher gegebenenfalls mit einem oder mehreren Resten ausgewählt aus der Gruppe bestehend aus OH, F, Br, OR^{2.1}, Oxo, Methyl, Ethyl, Methanol, Ethanol, Phenyl, COOR^{2.1}, CH₂-NR^{2.2}R^{2.3} und NR^{2.2}R^{2.3} substituiert sein kann,
bedeuten, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind die oben genannten Arzneimittelkombinationen, die neben einer oder mehrerer, bevorzugt einer Verbindung der Formel **1** als PDE4-Inhibitor, wobei
- **R²**: ein Rest nach Formel **3** 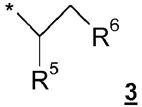
ist, worin
- **R⁶**: OH oder NH₂ ist und
worin
- **R⁵**: Methyl, Ethyl, Propyl, Isopropyl ist
bedeutet,
wenigstens ein NSAID (2) umfassen.

Ein weiterer besonders bevorzugter Gegenstand der Erfindung sind die obigen Arzneimittelkombinationen, die neben einer oder mehrerer, bevorzugt einer Verbindung der Formel 1 als PDE4-Inhibitor, wobei
- **R²**: ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, wobei
- R^{2.1}: ausgewählt ist aus der Gruppe bestehend aus Methyl, Ethyl, Propyl, Isopropyl, Butyl, Isobutyl,
bedeuten, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Außerdem sind solche der obigen Arzneimittelkombinationen besonders bevorzugt, die neben einer oder mehrerer, bevorzugt einer Verbindung der Formel **1** als PDE4-Inhibitor, wobei
**R²** ein Cyclopropyl ist, das gegebenenfalls mit einem weiteren Rest ausgewählt aus der Gruppe bestehend aus -NH₂, CH2- NH₂, -NH(CH₃), -N(CH₃)₂, Methyl, Ethyl, Propyl, Isopropyl, -NH-CO-(tert-Butyl), -NH-CO-O-(tert-Butyl), -N(CH₃)-CO-(tert-Butyl), -N(CH₃)-CO-O-(tert-Butyl), -CF₃, -CHF₂, CH₂F, F, Cl und Br substituiert sein,
bedeuten, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind solche der obigen Arzneimittelkombinationen, die neben einer oder mehrerer, bevorzugt einer Verbindung der Formel 1 als PDE4-Inhibitor, wobei
**R²** ein Cyclopropyl
   - **R²**: ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei **R^{2.1}** H, Methyl oder Ethyl, sein kann,
bedeuten, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Ein weiterer besonders bevorzugter Gegenstand der Erfindung sind solche der obigen Arzneimittelkombinationen, die neben einer oder mehrerer, bevorzugt einer Verbindung der Formel **1** als PDE4-Inhibitor wobei
- **R²**: einen Rest ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, CF₃, CHF₂, CH₂F, OH und Oxo oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
bedeuten und
wobei **R^{2.1}, R^{2.2}** und **R^{2.3}** wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Außerdem sind solche Arzneimittelkombinationen besonders bevorzugt, die neben einer oder mehrerer, bevorzugt einer Verbindung der Formel 1 als PDE4-Inhibitor,
wobei
- **R²**: einen Rest ausgewählt aus einer Gruppe bestehend aus einem monocyclischen, gesättigten sechsgliedrigem Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃ und N(CH₃)₂, Methyl, Ethyl, Propyl, Isopropyl, Cyclopropyl, Methoxy und Ethoxy substituiert sein kann,
bedeutet, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Ein weiterer besonders bevorzugter Gegenstand der vorliegenden Erfindung ist die oben benannte Arzneimittelkombination, die neben einer oder mehrerer, bevorzugt einer Verbindung der Formel **1** als PDE4-Inhibitor,
wobei
- **R²**: einen Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
bedeutet, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind die obigen Arzneimittelkombinationen, die neben einer oder mehrerer, bevorzugt einer Verbindung der Formel **1** als PDE4-Inhibitor,
wobei
- **R³**: ein Naphthalin oder Phenyl,
welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F,-OCH₃, OCH₂CH₃; SO₂-CH₃, SO₂-CH₂CH₃, COOCH₃ und CO-O-CH₂CH₃ substituiert sein kann,
bedeutet, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Ebenfalls besonders bevorzugt sind im Rahmen der Erfindung die obigen Arzneimittelkombinationen, die neben einer oder mehrerer, bevorzugt einer Verbindung der Formel **1** als PDE4-Inhibitor,
wobei
- **R³**: ein Rest ausgewählt aus der Gruppe bestehend aus Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₅₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl, -COO-Propyl, -COO-Isopropyl und O-Methyl, O-Ethyl, O-Propyl und O-Isopropyl substituiert sein kann,
und worin
- **R⁴**: H, CN, OH, CF₃, CHF₂, CH₂F, F, Methyl, Ethyl, O-Methyl oder O-Ethyl,
und wobei
- **Het**: ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Besonders bevorzugt sind weiterhin die obigen Arzneimittelkombinationen, dadurch gekennzeichnet, dass sie neben einer oder mehrerer, bevorzugt einer Verbindung der Formel **1** als PDE4-Inhibitor, wobei
- **R³**: ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann,
der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl,-COO-Ethyl und O-Methyl, O-Ethyl substituiert sein kann,
bedeuten, und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind die obigen Arzneimittelkombinationen, die neben einer oder mehrerer, bevorzugt einer Verbindung der Formel **1** als PDE4-Inhibitoren,
wobei
- **R³**: ein Rest ausgewählt aus einem monocyclischen, gesättigten oder teilweise gesättigten, drei- bis siebengliedrigem Heterocyclus oder einem monocyclischen fünfbis sechsgliedrigem Heteroaryl ist,
der ausgewählt ist aus der Gruppe bestehend aus Imidazol, Dihydroimidazol, Oxadiazol, Oxadiazolidin, Pyrazol, Pyridin und Dihydropyrazol,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, --O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
bedeuten,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Einen besonders bevorzugten Gegenstand der vorliegenden Erfindung stellen die obigen Arzneimittelkombinationen dar, die neben einer oder mehrerer, bevorzugt einer Verbindung der Formel **1** als PDE4-Inhibitor, wobei
- **R³** und **R⁴**: gemeinsam einen mono- oder bicyclischen, ungesättigten oder
teilweise gesättigten, drei- bis elfgliedrigen Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
bedeuten,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind die obigen Arzneimittelkombinationen, die neben einer oder mehrerer, bevorzugt einer Verbindung der Formel **1** als PDE4-Inhbitor, wobei
- **R³** und **R⁴**: gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder
mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂,
einem gesättigtem oder teilweise gesättigten, fünf- oder sechsgliedrigem Heterocyclus und einem fünf- oder sechsgliedrigem Heteroaryl substituiert sein kann,
bedeuten,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Außerdem sind die oben genannten Arzneimittelkombinationen im Rahmen der Erfindung besonders bevorzugt, die neben einer oder mehrerer, bevorzugt einer Verbindung der Formel **1** als PDE4-Inhibitor,
wobei
- **R³**: -O-R^{3.1} ist,
- **R**^{3.1}: ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, -CF₃, CHF₂, CH₂F, CO-(Methyl), CO-(Ethyl), CO-(Propyl), CO-(Isopropyl), CO-(Butyl), CO-(Isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂,-O-Methylen-N(Ethyl)₂, -O-Ethylen-N(Ethyl)₂, CO-NH₂, CO-NHCH₃, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇-Cycloalkyl, -CO-Cyclopropyl, -CO-N(CH₃)-Cyclopropyl, -CO-N(CH₃)-C₄₋₇-Cycloalkyl, C₄₋₇-Cycloalkyl, Cyclopropyl, C₄₋₇-Cycloalkyl-Methylen-, Cyclopropyl-Methylen-, C₄₋₇-Cycloalkyl-Ethylen-, Cyclopropyl-Ethylen-, Hetaryl-Methylen-, Hetaryl-Ethylen- und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O- Methyl, Ethyl, O-Ethyl, OH, Oxo und CF₃ substituiert sein kann,
bedeuten,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Ein weiterer besonders bevorzugter Gegenstand der Erfindung sind die oben genannten Arzneimittelkombinationen, dadurch gekennzeichnet, die neben einer oder mehrerer, bevorzugt einer Verbindung der Formel **1** als PDE4-Inhibitor,
worin
- **R⁴**: H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl,
-CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
bedeuten,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Weiterhin besonders bevorzugt sind die obigen Arzneimittelkombinationen, die neben einer oder mehrerer, bevorzugt einer Verbindung der Formel **1** als PDE4-Inhibitor,
worin
**R³** ein Rest ausgewählt aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl, O-Methyl, O-Ethyl, O-Propyl, O-Isopropyl, OH, F, Cl, Br, CF₃, Phenyl, Hetaryl und C₃₋₆-Cycloalkyl substituiert sein kann,
bedeutet,
und worin die übrigen Reste wie vorstehend definiert sind,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Im Rahmen der vorliegenden Erfindung sind die oben genannten Arzneimittelkombinationen besonders bevorzugt, die neben einer oder mehrerer, bevorzugt einer Verbindung der Formel **1** als PDE4-Inhibitor,
worin
- **X**: SO₂ ist,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Besonders bevorzugt sind weiterhin die obigen Arzneimittelkombinationen, die neben einer oder mehrerer, bevorzugt einer Verbindung der Formel **1** als PDE4-Inhibitor ausgewählt aus der Gruppe bestehend aus:
1.1 (R)-2-{2-[4-(4-Chlorphenyl)-piperidin-1-yl]-5-oxo-6,7-dihydro-5H-5λ4-thieno[3,2-d]pyrimidin -4-ylamino}-3-methylbutan-1-ol
1.2 (1-{2-[4-(4-Chlorphenyl)-piperidin-1-yl]-5-oxo-6,7-dihydro-5H-5M-thieno[3,2-d]pyrimidin-4-ylamino}-cyclopropyl)-methanol
1.3 (R)-2-{2-[4-(4-Chlorphenyl)-piperidin-1-yl]-5-oxo-6,7-dihydro-5H-5λ4-thieno[3,2-d]pyrimidin-4-ylamino}-pentan-1-ol
1.4 (R)-1-{2-[4-(4-Chlorphenyl)-piperidin-1-yl]-5-oxo-6,7-dihydro-5H-5λ4-thieno[3,2-d]pyrimidin-4-ylamino}-1-(4-fluorphenyl)-2-methylpropan-2-ol
1.5 (S)-5-{2-[4-(4-Chlorphenyl)-piperidin-1-yl]-5-oxo-6,7-dihydro-5H-5λ4-thieno[3,2-d]pyrimidin-4-ylamino}-1-methylpiperidin-2-on
1.6 {2-[4-(4-Chlorphenyl)-piperidin-1-yl]-5-oxo-6,7-dihydro-5H-5λ4-thieno[3,2-d]pyrimidin-4-yl}-(tetrahydropyran-4-yl)-amin
1.7 1-(4-(1-Hydroxymethylcyclopropylamino)-5-oxo-6,7-dihydro-5H-5λ4-thieno[3,2-d]pyrimidin-2-yl)-3'-methyl-1'H-spiro[piperidin-4,4'-chinazolin]-2'(3'H)-on
1.8 {1-[2-(4-Benzo[d]isoxazol-3-yl-piperidin-1-yl)-5-oxo-6,7-dihydro-5H-5λ4-thieno[3,2-d]pyrimidin-4-ylamino]-cyclopropyl}-methanol
1.9 (1-{2-[4-(2-Ethyl-5-fluor-1H-indol-3-yl)-piperidin-1-yl]-5-oxo-6,7-dihydro-5H-5λ4-thieno[3,2-d]pyrimidin-4-ylamino}-cyclopropyl)-methanol
1.10 1-[4-((S)-1-Methyl-6-oxopiperidin-3-ylamino)-5-oxo-6,7-dihydro-5H-5λ4-thieno[3,2-d]pyrimidin-2-yl]-4-phenylpiperidin-4-carbonitril
1.11 3'-methyl-1-(4-(tetrahydro-2H-pyran-4-ylamino)-5-oxo-6,7-dihydro-5H-5λ4-thieno[3,2-d]pyrimidin-2-yl)-1'H-spiro[piperidin-4,4'-chinazolin]-2'(3'H)-on
1.12 (3-Fluorphenyl)-[5-oxo-2-(3,4,5,6-tetrahydro-2H-[4,4']bipyridinyl-1-yl)-6,7-dihydro-5H-5λ4-thieno[3,2-d]pyrimidin-4-yl]-amin
1.13 {2-[4-(2-Ethyl-5-fluor-1H-indol-3-yl)-piperidin-1-yl]-5-oxo-6,7-dihydro-5H-5λ4-thieno[3,2-d]pyrimidin-4-yl}-(3-fluorphenyl)-amin
1.14 (1-{2-[4-(2,4-Difluorphenyl)-piperidin-1-yl]-5-oxo-6,7-dihydro-5H-5λ4-thieno[3,2-d]pyrimidin-4-ylamino}-cyclopropyl)-methanol
1.15 {2-[4-(2,4-Difluorphenyl)-piperidin-1-yl]-5-oxo-6,7-dihydro-5H-5λ4-thieno[3,2-d]pyrimidin-4-yl}-(tetrahydropyran-4-yl)-amin
1.16 (S)-5-[2-(4-Benzoxazol-2-yl-piperidin-1-yl)-5-oxo-6,7-dihydro-5H-5λ4-thieno[3,2-d]pyrimidin-4-ylamino]-1-methylpiperidin-2-on
1.17 (1-{2-[4-(6-Fluorbenzo[d]isoxazol-3-yl)-piperidin-1-yl]-5-oxo-6,7-dihydro-5H-5λ4-thieno[3,2-d]pyrimidin-4-ylamino}-cyclopropyl)-methanol
1.18 (1-{2-[4-(5-Fluorbenzo[d]isoxazol-3-yl)-piperidin-1-yl]-5-oxo-6,7-dihydro-5H-5λ4-thieno[3,2-d]pyrimidin-4-ylamino}-cyclopropyl)-methanol
1.19 {2-[4-(5-Furan-2-yl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.20 (3-Fluorphenyl)-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-yl}-amin
1.21 (R)-3-Methyl-2-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-ylamino}-butan-1-ol
1.22 (S)-5-{2-[4-(4-Fluorphenoxy)-piperidin-1-yl]-5-oxo-6,7-dihydro-5H-5λ4-thieno[3,2-d]pyrimidin-4-ylamino}-1-methylpiperidin-2-on
1.23 (2-{4-[4-(4,5-Dihydrooxazol-2-yl)-phenoxy]-piperidin-1-yl}-5-oxo-6,7-dihydro-5H-5λ4-thieno[3,2-d]pyrimidin-4-yl)-(tetrahydropyran-4-yl)-amin
1.24 4-{1-[5-Oxo-4-(tetrahydropyran-4-ylamino)-6,7-dihydro-5H-5λ4-thieno[3,2-d]pyrimidin-2-yl]-piperidin-4-yloxy}-benzoesäure
1.25 2-(1-{2-[4-(4-Chlorphenyl)-piperidin-1-yl]-5-oxo-6,7-dihydro-5H-5λ4-thieno[3,2-d]pyrimidin-4-ylamino}-cyclopropyl)-propan-2-ol
1.26 {2-[4-(5-tert-Butyl-1-methyl-1H-indol-3-yl)-piperidin-1-yl]-5-oxo-6,7-dihydro-5H-5λ4-thieno[3,2-d]pyrimidin-4-yl}-(tetrahydropyran-4-yl)-amin
1.27 2-[4-(5-Furan-2-yl-1-methyl-1H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.28 (S)-5-(2-{4-[4-(4,5-Dihydrooxazol-2-yl)-phenoxy]-piperidin-1-yl}-5-oxo-6,7-dihydro-5H-5λ4-thieno[3,2-d]pyrimidin-4-ylamino)-1-methylpiperidin-2-on
1.29 {2-[4-(5-Furan-2-yl-2-methyl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.30 {2-[4-(1-Methyl-1H-imidazo[4,5-c]pyridin-2-yl)-piperidin-1-yl]-5-oxo-6,7-dihydro-5H-5λ4-thieno[3,2-d]pyrimidin-4-yl}-(tetrahydropyran-4-yl)-amin
1.31 2-Methoxy-N-{1-[5-oxo-4-(tetrahydropyran-4-ylamino)-6,7-dihydro-5H-5λ4-thieno[3,2-d]pyrimidin-2-yl]-4-phenylpiperidin-4-ylmethyl}-acetamid
1.32 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yl}-benzamid
1.33 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yloxy}-benzamid
1.34 {5-Oxo-2-[4-(pyridin-4-yloxy)-piperidin-1-yl]-6,7-dihydro-5H-5λ4-thieno[3,2-d]pyrimidin-4-yl}-(tetrahydropyran-4-yl)-amin
1.35 {2-[4-(4-Chlorphenoxy)-piperidin-1-yl]-5-oxo-6,7-dihydro-5H-5λ4-thieno[3,2-d]pyrimidin-4-yl}-(tetrahydropyran-4-yl)-amin
1.36 (S)-1-Methyl-5-{2-[4-(5-methyl-4-phenyloxazol-2-yl)-piperidin-1-yl]-5-oxo-6,7-dihydro-5H-5λ4-thieno[3,2-d]pyrimidin-4-ylamino}-piperidin-2-on
1.37 (1-{2-[4-(5-Methyl-4-phenyloxazol-2-yl)-piperidin-1-yl]-5-oxo-6,7-dihydro-5H-5λ4-thieno[3,2-d]pyrimidin-4-ylamino}-cyclopropyl)-methanol
1.38 (S)-5-{2-[4-(4,5-Diphenyloxazol-2-yl)-piperidin-1-yl]-5-oxo-6,7-dihydro-5H-5λ4-thieno[3,2-d]pyrimidin-4-ylamino}-1-methylpiperidin-2-on
1.39 {4-(4-Chlorphenyl)-1-[5-oxo-4-(tetrahydropyran-4-ylamino)-6,7-dihydro-5H-5λ4-thieno[3,2-d]pyrimidin-2-yl]-piperidin-4-yl}-methanol
1.40 [1-(2-{4-[5-(4-Chlorphenyl)-4-methyloxazol-2-yl]-piperidin-1-yl}-5-oxo-6,7-dihydro-5H-5λ4-thieno[3,2-d]pyrimidin-4-ylamino)-cyclopropyl]-methanol
1.41 4-(4-Chlorphenyl)-1-[5-oxo-4-(tetrahydropyran-4-ylamino)-6,7-dihydro-5H-5λ4-thieno[3,2-d]pyrimidin-2-yl]-piperidin-4-ol
1.42 {2-[4-(4-Chlorphenyl)-4-methoxypiperidin-1-yl]-5-oxo-6,7-dihydro-5H-5λ4-thieno[3,2-d]pyrimidin-4-yl}-(tetrahydropyran-4-yl)-amin
1.43 4-{1-[4-(1-Hydroxymethylcyclopropylamino)-5-oxo-6,7-dihydro-5H-5λ4-thieno[3,2-d]pyrimidin-2-yl]-piperidin-4-yloxy}-benzonitril
1.44 5-Oxo-2-[4-(4,5,6,7-tetrahydrobenzoxazol-2-yl)-piperidin-1-yl]-6,7-dihydro-5H-5λ4-thieno[3,2-d]pyrimidin-4-yl}-(tetrahydropyran-4-yl)-amin
1.45 (S)-5-{2-[4-(4-Chlorphenyl)-piperidin-1-yl]-5,5-dioxo-6,7-dihydro-5H-5λ6-thieno[3,2-d]pyrimidin-4-ylamino}-1-methylpiperidin-2-on
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen.

Die oben genannten Verbindungen der Formel **1** werden wie in den Synthesevorschriften im Detail beschrieben hergestellt.

Insbesondere sind solche der obigen Arzneimittelkombinationen Gegenstand der Erfindung, in denen der PDE4-Inhibitor der allgemeinen Formel **1** in einer Einzeldosis von 0,01 mg bis 50 mg, vorzugsweise von 0,05 bis 30 mg, bevorzugter von 0,1 bis 20 mg, insbesondere von 0,5 bis 10 mg verabreicht wird.

Insbesondere betrifft die Erfindung die oben genannten Arzneimittelkombinationen, bei denen die oder zumindest eine oder mehrere der PDE4-Inhibitor-vermittelten Nebenwirkungen in erheblichem Ausmaß reduziert oder völlig vermieden wird im Vergleich zu einer alleinigen Applikation des in der Arzneimittelkombination verwendeten PDE4-Inhibitors. Diese PDE4-Inhibitor-vermittelten Nebenwirkungen sind vorzugsweise ausgewählt aus Körpergewichtsverlust, Leukozytose, Neutrophilie, Übelkeit, Erbrechen, Diarrhoe (einschließlich des Auftretens von Entzündungsparametern und der Fibroblasten-Proliferation im Mesenterium). Diese PDE4-Inhibitor-vermittelten Nebenwirkungen sind stärker bevorzugt ausgewählt aus Körpergewichtsverlust, Leukozytose, Neutrophilie und Diarrhoe. Diese PDE4-Inhibitor-vermittelten Nebenwirkungen betreffen insbesondere das Auftreten von Diarrhoe.

Ein weiterer Gegenstand der vorliegenden Erfindung ist die Verwendung eines NSAIDs (**2**) zur Reduzierung der Nebenwirkungen eines oder mehrerer PDE4-Inhibitoren bei der Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des peripheren oder zentralen Nervensystems.

Ein anderer Gegenstand der vorliegenden Erfindung ist die Verwendung einer Kombination enthaltend einen oder mehrere PDE4-Inhibitoren undwenigstens ein NSAID (**2**) zur Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

Weiterhin bevorzugt ist die Verwendung der Kombination eines oder mehrerer, bevorzugt eines PDE4-Inhibitors der allgemeinen Formel **1**, 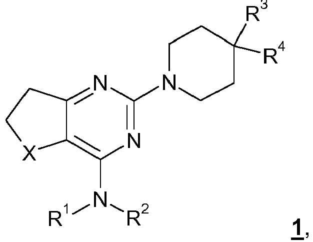 worin X, R¹, R²,R³ und R⁴ wie vorstehend definiert und bevorzugt definiert sind,
und wenigstens eines NSAIDs (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) zur Herstellung einer Arzneimittelkombination zur Behandlung einer der Erkrankungen ausgewählt aus Atemwegserkrankungen, Lungenerkrankungen, gastrointestinale Beschwerden, Erkrankungen und auch entzündliche Erkrankungen der Gelenke, der Haut oder der Augen, Krebserkrankungen, Erkrankungen des periphären oder zentralen Nervensystems, insbesondere jedoch zur Behandlung von entzündlichen und obstruktiven Erkrankungen wie COPD, chronischer Sinusitis, Asthma, Morbus Crohn, Colitis ulcerosa.

Bevorzugter Gegenstand der Erfindung ist hierbei die Verwendung der Kombination enthaltend einer einen oder mehrere PDE4-Inhibitoren - insbesondere einen oder mehrere der PDE4-Inhibitoren nach Formel **1** - und des wenigstens einen NSAIDs (**2**) zur Herstellung einer Arzneimittelkombination zur Behandlung der oben genannten Erkrankungen, dadurch gekennzeichnet, dass der PDE4-Inhibitor - insbesondere der PDE4-Inhibitor der Formel **1** - und das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) in einer einzigen Formulierung gemeinsam und gleichzeitig verabreicht werden, wobei diese einzige Formulierung vorzugsweise eine orale Formulierung ist wie beispielsweise eine Tablette, Kapsel o.ä...

Weiterhin bevorzugt ist hierbei die Verwendung der Kombination enthaltend einen oder mehrere PDE4-Inhibitoren - insbesondere einen oder mehrere PDE4-Inhibitoren nach Formel **1** - und des wenigstens einen NSAIDs (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) zur Herstellung einer Arzneimittelkombination zur Behandlung der oben genannten Erkrankungen, dadurch gekennzeichnet, dass der PDE4-Inhibitor - insbesondere der PDE4-Inhibitor der Formel **1** - und das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) in zwei separaten Formulierungen getrennt voneinander innerhalb eines zeitlichen Abstandes von 0 bis 6 Stunden verabreicht werden. Bei dieser getrennten Applikation in zwei separaten Formulierungen kann die Formulierung enthaltend den PDE4-Inhibitor - insbesondere den PDE4-Inhibitor der Formel **1** - eine orale oder inhalative Formulierung sein, ist aber vorzugsweise eine orale Formulierung, und die Formulierung enthaltend das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) ist vorzugsweise eine orale Formulierung. Weiterhin wird bei der Verwendung der Kombination in getrennten Formulierungen zur Herstellung einer Arzneimittelkombination zur Behandlung der oben genannten Erkrankungen die Formulierung enthaltend den PDE4-Inhibitor - insbesondere den PDE4-Inhibitor der Formel **1** - vorzugsweise einmal täglich und die Formulierung enthaltend das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) vorzugsweise entweder einmal oder zweimal täglich verabreicht.

Bei dieser oben genannten Verwendung der Kombination sind solche PDE4-Inhibitoren der allgemeinen Formel **1** besonders bevorzugt, die ausgewählt sind aus der Gruppe bestehend aus:
1.1 (R)-2-{2-[4-(4-Chlorphenyl)-piperidin-1-yl]-5-oxo-6,7-dihydro-5H-5λ4-thieno[3,2-d]pyrimidin-4-ylamino}-3-methylbutan-1-ol
1.2 (1-{2-[4-(4-Chlorphenyl)-piperidin-1-yl]-5-oxo-6,7-dihydro-5H-5λ4-thieno[3,2-d]pyrimidin-4-ylamino}-cyclopropyl)-methanol
1.3 (R)-2-{2-[4-(4-Chlorphenyl)-piperidin-1-yl]-5-oxo-6,7-dihydro-5H-5λ4-thieno[3,2-d]pyrimidin-4-ylamino}-pentan-1-ol
1.4 (R)-1-{2-[4-(4-Chlorphenyl)-piperidin-1-yl]-5-oxo-6,7-dihydro-5H-5λ4-thieno[3,2-d]pyrimidin-4-ylamino}-1-(4-fluorphenyl)-2-methylpropan-2-ol
1.5 (S)-5-{2-[4-(4-Chlorphenyl)-piperidin-1-yl]-5-oxo-6,7-dihydro-5H-5λ4-thieno[3,2-d]pyrimidin-4-ylamino}-1-methylpiperidin-2-on
1.6 {2-[4-(4-Chlorphenyl)-piperidin-1-yl]-5-oxo-6,7-dihydro-5H-5λ4-thieno[3,2-d]pyrimidin-4-yl}-(tetrahydropyran-4-yl)-amin
1.7 1-(4-(1-Hydroxymethylcyclopropylamino)-5-oxo-6,7-dihydro-5H-5λ4-thieno[3,2-d]pyrimidin-2-yl)-3'-methyl-1'H-spiro[piperidin-4,4'-chinazolin]-2'(3'H)-on
1.8 {1-[2-(4-Benzo[d]isoxazol-3-yl-piperidin-1-yl)-5-oxo-6,7-dihydro-5H-5λ4-thieno[3,2-d]pyrimidin-4-ylamino]-cyclopropyl}-methanol
1.9 (1-{2-[4-(2-Ethyl-5-fluor-1H-indol-3-yl)-piperidin-1-yl]-5-oxo-6,7-dihydro-5H-5λ4-thieno[3,2-d]pyrimidin-4-ylamino}-cyclopropyl)-methanol
1.10 1-[4-((S)-1-Methyl-6-oxopiperidin-3-ylamino)-5-oxo-6,7-dihydro-5H-5λ4-thieno[3,2-d]pyrimidin-2-yl]-4-phenylpiperidin-4-carbonitril
1.11 3'-methyl-1-(4-(tetrahydro-2H-pyran-4-ylamino)-5-oxo-6,7-dihydro-5H-5λ4-thieno[3,2-d]pyrimidin-2-yl)-1'H-spiro[piperidin-4,4'-chinazolin]-2'(3'H)-on
1.12 (3-Fluorphenyl)-[5-oxo-2-(3,4,5,6-tetrahydro-2H-[4,4']bipyridinyl-1-yl)-6,7-dihydro-5H-5λ4-thieno[3,2-d]pyrimidin-4-yl]-amin
1.13 {2-[4-(2-Ethyl-5-fluor-1H-indol-3-yl)-piperidin-1-yl]-5-oxo-6,7-dihydro-5H-5λ4-thieno[3,2-d]pyrimidin-4-yl}-(3-fluorphenyl)-amin
1.14 (1-{2-[4-(2,4-Difluorphenyl)-piperidin-1-yl]-5-oxo-6,7-dihydro-5H-5λ4-thieno[3,2-d]pyrimidin-4-ylamino}-cyclopropyl)-methanol
1.15 {2-[4-(2,4-Difluorphenyl)-piperidin-1-yl]-5-oxo-6,7-dihydro-5H-5λ4-thieno[3,2-d]pyrimidin-4-yl}-(tetrahydropyran-4-yl)-amin
1.16 (S)-5-[2-(4-Benzoxazol-2-yl-piperidin-1-yl)-5-oxo-6,7-dihydro-5H-5λ4-thieno[3,2-d]pyrimidin-4-ylamino]-1-methylpiperidin-2-on
1.17 (1-{2-[4-(6-Fluorbenzo[d]isoxazol-3-yl)-piperidin-1-yl]-5-oxo-6,7-dihydro-5H-5λ4-thieno[3,2-d]pyrimidin-4-ylamino}-cyclopropyl)-methanol
1.18 (1-{2-[4-(5-Fluorbenzo[d]isoxazol-3-yl)-piperidin-1-yl]-5-oxo-6,7-dihydro-5H-5λ4-thieno[3,2-d]pyrimidin-4-ylamino}-cyclopropyl)-methanol
1.19 {2-[4-(5-Furan-2-yl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.20 (3-Fluorphenyl)-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-yl}-amin
1.21 (R)-3-Methyl-2-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-ylamino}-butan-1-ol
1.22 (S)-5-{2-[4-(4-Fluorphenoxy)-piperidin-1-yl]-5-oxo-6,7-dihydro-5H-5λ4-thieno[3,2-d]pyrimidin-4-ylamino}-1-methylpiperidin-2-on
1.23 (2-{4-[4-(4,5-Dihydrooxazol-2-yl)-phenoxy]-piperidin-1-yl}-5-oxo-6,7-dihydro-5H-5λ4-thieno[3,2-d]pyrimidin-4-yl)-(tetrahydropyran-4-yl)-amin
1.24 4-{1-[5-Oxo-4-(tetrahydropyran-4-ylamino)-6,7-dihydro-5H-5λ4-thieno[3,2-d]pyrimidin-2-yl]-piperidin-4-yloxy}-benzoesäure
1.25 2-(1-{2-[4-(4-Chlorphenyl)-piperidin-1-yl]-5-oxo-6,7-dihydro-5H-5λ4-thieno[3,2-d]pyrimidin-4-ylamino}-cyclopropyl)-propan-2-ol
1.26 {2-[4-(5-tert-Butyl-1-methyl-1H-indol-3-yl)-piperidin-1-yl]-5-oxo-6,7-dihydro-5H-5λ4-thieno[3,2-d]pyrimidin-4-yl}-(tetrahydropyran-4-yl)-amin
1.27 2-[4-(5-Furan-2-yl-1-methyl-1H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.28 (S)-5-(2-{4-[4-(4,5-Dihydrooxazol-2-yl)-phenoxy]-piperidin-1-yl}-5-oxo-6,7-dihydro-5H-5λ4-thieno[3,2-d]pyrimidin-4-ylamino)-1-methylpiperidin-2-on
1.29 {2-[4-(5-Furan-2-yl-2-methyl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.30 {2-[4-(1-Methyl-1H-imidazo[4,5-c]pyridin-2-yl)-piperidin-1-yl]-5-oxo-6,7-dihydro-5H-5λ4-thieno[3,2-d]pyrimidin-4-yl}-(tetrahydropyran-4-yl)-amin
1.31 2-Methoxy-N-{1-[5-oxo-4-(tetrahydropyran-4-ylamino)-6,7-dihydro-5H-5λ4-thieno[3,2-d]pyrimidin-2-yl]-4-phenylpiperidin-4-ylmethyl}-acetamid
1.32 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yl}-benzamid
1.33 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yloxy}-benzamid
1.34 {5-Oxo-2-[4-(pyridin-4-yloxy)-piperidin-1-yl]-6,7-dihydro-5H-5λ4-thieno[3,2-d]pyrimidin-4-yl}-(tetrahydropyran-4-yl)-amin
1.35 {2-[4-(4-Chlorphenoxy)-piperidin-1-yl]-5-oxo-6,7-dihydro-5H-5λ4-thieno[3,2-d]pyrimidin-4-yl}-(tetrahydropyran-4-yl)-amin
1.36 (S)-1-Methyl-5-{2-[4-(5-methyl-4-phenyloxazol-2-yl)-piperidin-1-yl]-5-oxo-6,7-dihydro-5H-5λ4-thieno[3,2-d]pyrimidin-4-ylamino}-piperidin-2-on
1.37 (1-{2-[4-(5-Methyl-4-phenyloxazol-2-yl)-piperidin-1-yl]-5-oxo-6,7-dihydro-5H-5λ4-thieno[3,2-d]pyrimidin-4-ylamino}-cyclopropyl)-methanol
1.38 (S)-5-{2-[4-(4,5-Diphenyloxazol-2-yl)-piperidin-1-yl]-5-oxo-6,7-dihydro-5H-5λ4-thieno[3,2-d]pyrimidin-4-ylamino}-1-methylpiperidin-2-on
1.39 {4-(4-Chlorphenyl)-1-[5-oxo-4-(tetrahydropyran-4-ylamino)-6,7-dihydro-5H-5M-thieno[3,2-d]pyrimidin-2-yl]-piperidin-4-yl}-methanol
1.40 [1-(2-{4-[5-(4-Chlorphenyl)-4-methyloxazol-2-yl]-piperidin-1-yl}-5-oxo-6,7-dihydro-5H-5λ4-thieno[3,2-d]pyrimidin-4-ylamino)-cyclopropyl]-methanol
1.41 4-(4-Chlorphenyl)-1-[5-oxo-4-(tetrahydropyran-4-ylamino)-6,7-dihydro-5H-5λ4-thieno[3,2-d]pyrimidin-2-yl]-piperidin-4-ol
1.42 {2-[4-(4-Chlorphenyl)-4-methoxypiperidin-1-yl]-5-oxo-6,7-dihydro-5H-5λ4-thieno[3,2-d]pyrimidin-4-yl}-(tetrahydropyran-4-yl)-amin
1.43 4-{1-[4-(1-Hydroxymethylcyclopropylamino)-5-oxo-6,7-dihydro-5H-5λ4-thieno[3,2-d]pyrimidin-2-yl]-piperidin-4-yloxy}-benzonitril
1.44 5-Oxo-2-[4-(4,5,6,7-tetrahydrobenzoxazol-2-yl)-piperidin-1-yl]-6,7-dihydro-5H-5λ4-thieno[3,2-d]pyrimidin-4-yl}-(tetrahydropyran-4-yl)-amin
1.45 (S)-5-{2-[4-(4-Chlorphenyl)-piperidin-1-yl]-5,5-dioxo-6,7-dihydro-5H-5λ6-thieno[3,2-d]pyrimidin-4-ylamino}-1-methylpiperidin-2-on.

Stärker bevorzugt wird bei dieser oben genannten Verwendung ein solches NSAID (**2**) eingesetzt, das ausgewählt ist aus der Gruppe bestehend aus Celecoxib (2.25 Lumiracoxib (2.74), Meloxicam (2.77), Naproxen (2.82) und Priroxicam (2.93).

Noch stärker bevorzugt wird bei dieser oben genannten Verwendung ein solches NSAID (**2**) eingesetzt, das ausgewählt ist aus der Gruppe bestehend aus Meloxicam (2.77) und Naproxen (2.82)).

Insbesondere wird bei dieser oben genannten Verwendung ein solches NSAID eingesetzt, das ausgewählt ist aus der Gruppe bestehend aus
- Meloxicam (2.77), bevorzugt in einer Einzeldosis von 7,5 bis 30 mg, stärker bevorzugt von 10 bis 20 mg
- Naproxen (2.82), bevorzugt in einer Einzeldosis von 250 bis 1000 mg, stärker bevorzugt von 250 bis 750 mg, und
wobei diese Einzeldosis ein- oder zweimal täglich verabreicht werden kann.

Insbesondere wird bei den oben genannten Verwendungen der Kombination zur Therapie der oben genannten Erkrankungen der PDE4-Inhibitor der allgemeinen Formel **1** in einer Einzeldosis von 0,01 mg bis 50 mg, vorzugsweise von 0,05 bis 30 mg, stärker bevorzugt von 0,1 bis 20 mg, insbesondere von 0,5 bis 10 mg verabreicht.

Insbesondere betrifft die Erfindung die oben genannten Verwendungen, bei denen die oder zumindest eine oder mehrere der PDE4-Inhibitor-vermittelten Nebenwirkungen in erheblichem Ausmaß reduziert oder völlig vermieden wird im Vergleich zu einer alleinigen Applikation des in der Arzneimittelkombination verwendeten PDE4-Inhibitors.

Insbesondere betrifft die Erfindung weiterhin die Verwendung von NSAIDs, vorzugsweise wie vorstehend definiert und bevorzugt definiert, zur Reduktion oder Vermeidung einer oder mehrerer PDE4-Inhibitor-vermittelter Nebenwirkungen.

Diese PDE4-Inhibitor-vermittelten Nebenwirkungen sind vorzugsweise ausgewählt aus Körpergewichtsverlust, Leukozytose, Neutrophilie, Übelkeit, Erbrechen, Diarrhoe (einschließlich des Auftretens von Entzündungsparametern und der Fibroblasten-Proliferation im Mesenterium). Diese PDE4-Inhibitor-vermittelten Nebenwirkungen sind stärker bevorzugt ausgewählt aus Körpergewichtsverlust, Leukozytose, Neutrophilie und Diarrhoe. Diese PDE4-Inhibitor-vermittelten Nebenwirkungen betreffen insbesondere das Auftreten von Diarrhoe. Gegenstände, die in der vorliegenden Beschreibung offenbart werden, jedoch nicht durch den Umfang der Ansprüche abgedeckt sind, stellen nicht Teil der beanspruchten Erfindung dar.

### SYNTHESEVORSCHRIFTEN

Die Verbindungen der allgemeinen Formel (I) können nach folgendem allgemeinen Syntheseschema hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken.

### ALLGEMEINES SYNTHESE SCHEMA 1

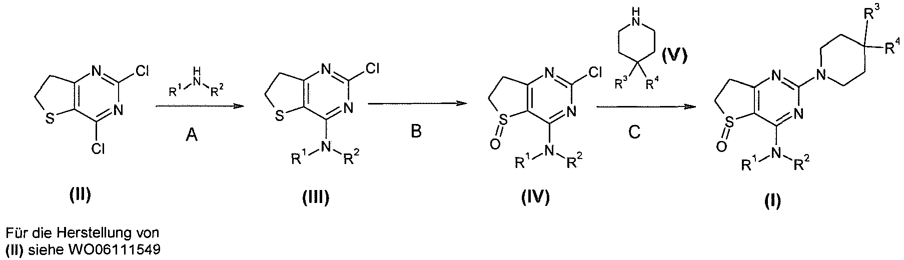

### 1. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN -4-YLAMINO}-3-METHYLBUTAN-1-OL (BEISPIEL 1.1)

### 1.1 (R)-2-(2-Chlor-6,7-dihydrothieno[3,2-d]pyrimidin-4-ylamino)-3-methylbutan-1-ol (III-1):

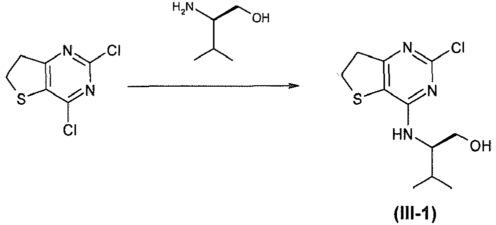

7,2 g 2,4-Dichlor-6,7-dihydrothieno[3,2-*d*]pyrimidin (**II**) werden in 36 ml Dioxan vorgelegt, erst werden 18 ml Diisopropylethylamin, dann 6,1 g (R)-(-)-2-Amino-3-methyl-1-butanol zugegeben. Das Reaktionsgemisch wird bei 100°C, bis keine weitere Umsetzung erfolgt, erhitzt und nach Abkühlen eingedampft. Der Rückstand wird mit Petrolether/Essigester (9:1) im Ultraschallbad behandelt und der Feststoff abgesaugt und getrocknet. 8,3 g **(III-1)** werden als Feststoff erhalten. Analytische HPLC (Methode A): RT = 2,75 min

### 1.2 (R)-2-(2-Chlor-5-oxo-6,7-dihydro-5H-5λ⁴-thieno[3,2-d]pyrimidin-4-ylamino)-3-methylbutan-1-ol (IV-1):

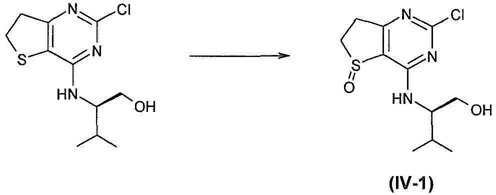

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschließend wird eine Suspension von 4,1 g **(III-1)** in 107 ml Dichlormethan zugegeben. Das Reaktionsgemisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 86/14) gereinigt. 2,45 g **(IV-1)** werden als Feststoff erhalten. Analytische HPLC (Methode A): RT = 2,37 min

### 1.3 (R)-2-{2-[4-(4-Chlorphenyl)-piperidin-1-yl]-5-oxo-6,7-dihydro-5H-5λ⁴-thieno[3,2-d]pyrimidin -4-ylamino}-3-methylbutan-1-ol (Beispiel 1.1)

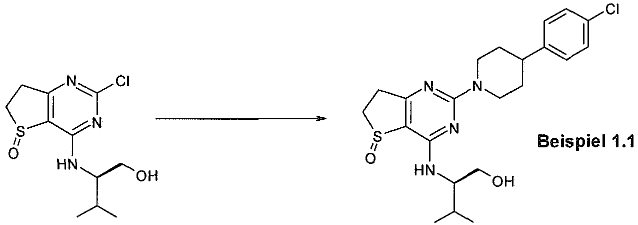

0,2 g **(IV-1)** wird in 3 ml Dioxan und 360 µl Diisopropylethylamin vorgelegt, mit 0,16 g 4-(4-Chlorphenyl)-piperidin versetzt und bei 120°C, bis keine weitere Umsetzung erfolgt, in der Mikrowelle erhitzt. Das Reaktionsgemisch wird mit Wasser versetzt, mit Dichlormethan extrahiert und das Produkt chromatographisch (Kieselgel, Dichlormethan/Methanol 100/0 bis 92/8) gereinigt. 0,33 g **Beispiel 1.1** werden als Feststoff erhalten. Analytische HPLC-MS (Methode A): RT = 1,24 min.

### 2. SYNTHESE VON (1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.2)

### 2.1 (1-Hydroxymethylcyclopropyl)-carbamidsäure tert-butylester:

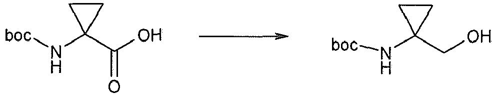

1 g 1-(BOC-amino)-cyclopropancarbonsäure wird in 20 ml Dimethoxyethan gelöst und auf - 70°C abgekühlt. Dann werden 0,65 ml N-Methylmorpholin zugegeben und 0,71 ml Isobutylchloroformiat in 5 ml Dimethoxyethan zugetropft. Das Reaktionsgemisch wird auf - 5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 2.2 1-Aminocyclopropanmethanol:

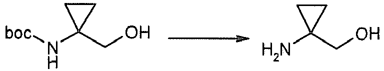

1,04 g (1-Hydroxymethyicyclopropyl)-carbamidsäure *tert*-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1 H, t); 0,91 (2H, t); 0,71 (2H, t).

### 2.3 [1-(2-Chlor-6,7-dihydrothieno[3,2-d]pyrimidin-4-ylamino)-cyclopropyl]-methanol (III-2):

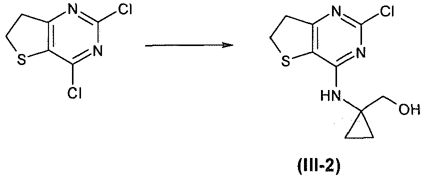

1,4 g **(II)** werden in 10 ml Dioxan vorgelegt, anschliessend werden 3,6 ml Diisopropylethylamin und dann 1 g 1-Aminocyclopropanmethanol (siehe 2.2) zugegeben. Das Reaktionsgemisch wird bei 160°C, bis keine weitere Umsetzung erfolgt, erhitzt und nach Abkühlen eingedampft. Der Rückstand wird mit Cyclohexan/Essigester (4:1) im Ultraschallbad behandelt, der Feststoff abgesaugt und getrocknet. 1,24 g **(III-2)** werden als Feststoff erhalten. Analytische HPLC-MS (Methode A): RT = 1,01 min.

### 2.4 [1-(2-Chlor-5-oxo-6,7-dihydro-5H-5λ⁴-thieno[3,2-d]pyrimidin-4-ylamino)-cyclopropyl]-methanol (IV-2):

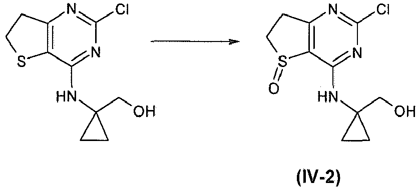

0,28 g S-(-)-1,1'-Bi-2-naphtol werden in 20 ml Chloroform unter Argon vorgelegt, dann 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g **(III-2)** in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Die Wasserphase wird mit Dichlormethan gewaschen und gefriergetrocknet. 1 g **(IV-2)** wird als Feststoff erhalten. Analytische HPLC-MS (Methode A) RT = 0,85 min

### 2.5 (1-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-methanol (Beispiel 1.2)

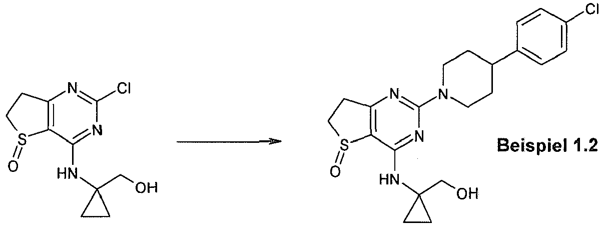

Ausgehend von 0,17 g **(IV-2)** und 0,15 g 4-(4-Chlorphenyl)-piperidin werden 0,14 g **Beispiel 1.2** analog zu Beispiel 1.1 (siehe 1.3) hergestellt und aufgereinigt. Analytische HPLC-MS (Methode B): RT = 1,32 min.

### 3. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-PENTAN-1-OL (BEISPIEL 1.3)

### 3.1 (R)-2-(2-Chlor-6,7-dihydrothieno[3,2-d]pyrimidin-4-ylamino)-pentan-1-ol (III-3):

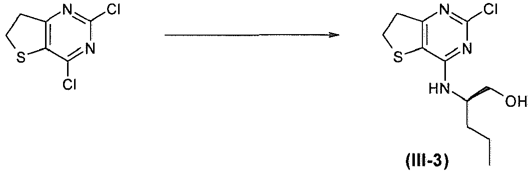

1,4 g 2,4-Dichlor-6,7-dihydrothieno[3,2-*d*]pyrimidin **(II)** werden in 9 ml Dioxan vorgelegt, erst werden 3,5 ml Diisopropylethylamin, dann 0,9 g D-norvalinol zugegeben. Das Reaktionsgemisch wird bei 120°C, bis keine weitere Umsetzung erfolgt, in der Mikrowelle erhitzt und nach Abkühlen eingedampft. Der Rückstand wird mit Petrolether/Essigester 9:1 im Ultraschallbad behandelt, der Feststoff wird abgesaugt und getrocknet. 1,5 g **(III-3)** werden als Feststoff erhalten. ¹H NMR (400 MHz, DMSO): 4,67 (1H, t); 0,86 (3H, t).

### 3.2 (R)-2-(2-Chlor-5-oxo-6,7-dihydro-5H-5λ⁴-thieno[3,2-d]pyrimidin-4-ylamino)-pentan-1-ol (IV-3):

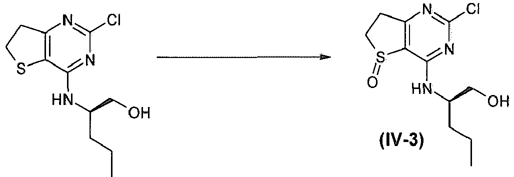

0,3 g S-(-)-1,1'-Bi-2-naphtol werden in 5 ml Chloroform unter Argon vorgelegt, dann 0,15 ml Titan(IV)-isopropylat und 0,19 ml Wasser zugegeben. Das Reaktionsgemisch wird 1 Stunde bei Raumtemperatur gerührt. Anschließend wird eine Suspension von 1,4 g **(III-3)** in 20 ml Dichlormethan zugegeben. Das Reaktionsgemisch wird auf -5°C abgekühlt und nach 30 Minuten werden 0,95 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird mit Dichlormethan extrahiert und chromatographisch (Essigester/Methanol 100/0 bis 80/20) gereinigt. 1,17 g **(IV-3)** werden als Feststoff erhalten. Analytische HPLC (Methode A): RT = 2,41 min

### 3.3 (R)-2-{2-[4-(4-Chlorphenyl)-piperidin-1-yl]-5-oxo-6,7-dihydro-5H-5λ⁴-thieno[3,2-d]pyrimidin-4-ylamino}-pentan-1-ol (Beispiel 1.3)

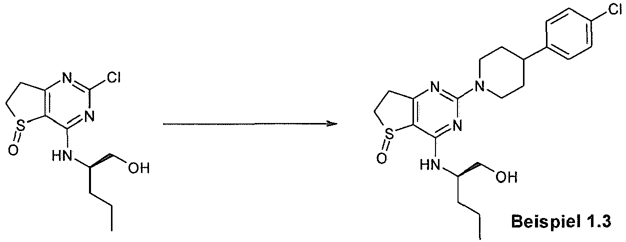

0,2 g **(IV-3)** werden in 4 ml Dioxan und 237 µl Diisopropylethylamin vorgelegt, mit 0,149 g 4-(4-Chlorphenyl)-piperidin versetzt und 30 min bei 130°C in der Mikrowelle erhitzt. Das Reaktionsgemisch wird mit Wasser versetzt und das Produkt mit Dichlormethan extrahiert. Der Rückstand wird mit Acetonitril im Ultraschallbad behandelt und der Feststoff abgesaugt. 0,104 g **Beispiel 1.3** werden als Feststoff erhalten. Analytische HPLC-MS (Methode A): RT = 1,29 min.

### 4. SYNTHESE VON (R)-1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-(4-FLUORPHENYL)-2-METHYLPROPAN-2-OL (BEISPIEL 1.4)

### 4.1 (R)-Amino-(4-fluorphenyl)-essigsäuremethylester:

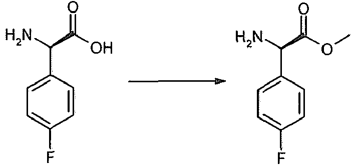

4 g (R)-4-fluorphenylglycin werden in 80 ml Methanol suspendiert. Unter Eisbadkühlung werden 3,28 ml Thionylchlorid langsam zugetropft, so dass die Temperatur zwischen 15°C und 20°C gehalten wird. Das Reaktionsgemisch wird 12 Stunden bei Raumtemperatur gerührt und anschließend zur Trockne eingedampft. 5,1 g des Produkts werden als Hydrochlorid erhalten. Analytische HPLC-MS (Methode A): RT = 0,8 min.

### 4.2 (R)-(4-Fluorphenyl)-(2,2,2-trifluoracetylamino)-essigsäuremethylester:

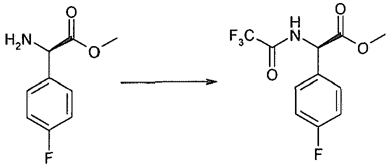

5,1 g (R)-Amino-(4-fluorphenyl)-essigsäuremethylester werden in 36,5 ml abs. Tetrahydrofuran vorgelegt, dann 3,9 ml Triethylamin zugegeben. Das Reaktionsgemisch wird auf -70°C abgekühlt. 3,9 ml Trifluoressiganhydrid werden dann langsam zugetropft, so dass die Temperatur nicht -60°C übersteigt. Das Reaktionsgemisch wird 12 Stunden bei Raumtemperatur gerührt und anschließend mit Wasser versetzt. Dann wird Kaliumhydrogencarbonat, bis keine Schaumbildung mehr zu beobachten ist, zugegeben und das Produkt mit Essigester extrahiert. 6,2 g des Produkts werden als Öl erhalten. Analytische HPLC-MS (Methode A): RT = 1,28 min.

### 4.3 2,2,2-Trifluor-N-[(R)-1-(4-fluorphenyl)-2-hydroxy-2-methylpropyl]-acetamid:

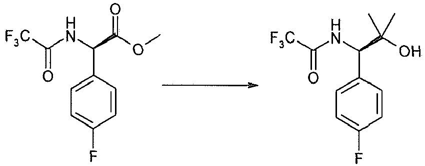

6,2 g (R)-(4-Fluorphenyl)-(2,2,2-trifluoracetylamino)-essigsäuremethylester werden in 195 ml abs. Tetrahydrofuran vorgelegt und das Reaktionsgemisch auf +3°C gekühlt. 37,2 ml einer Methylmagnesiumiodid Lösung (3 M) werden langsam zugetropft, so dass die Temperatur nicht +10°C übersteigt. Das Reaktionsgemisch wird 12 Stunden bei Raumtemperatur gerührt und anschließend in Eiswasser gerührt. Ammoniumchlorid wird, bis der Niederschlag gelöst ist, zugegeben und das Produkt mit Essigester extrahiert. 5,6 g des Produkts werden als Öl erhalten. Analytische HPLC-MS (Methode A): RT = 1,19 min

### 4.4 (R)-1-Amino-1-(4-fluorphenyl)-2-methylpropan-2-ol:

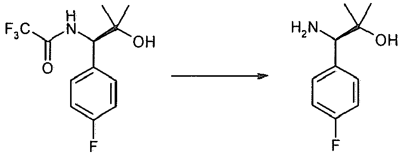

5,6 g 2,2,2-Trifluor-*N*-[(R)-1-(4-fluorphenyl)-2-hydroxy-2-methylpropyl]-acetamid und 2,27 g KOH werden in 60 ml Methanol suspendiert. Das Reaktionsgemisch wird 20 Stunden bei 60°C gerührt, anschließend mit Wasser versetzt und das Produkt mit Dichlormethan extrahiert. 3,2 g Produkt werden als Öl erhalten. Analytische HPLC-MS (Methode A): RT = 0,79 min.

### 4.5 (R)-1-(2-Chlor-6,7-dihydrothieno[3,2-d]pyrimidin-4-ylamino)-1-(4-fluorphenyl)-2-methylpropan-2-ol (III-4):

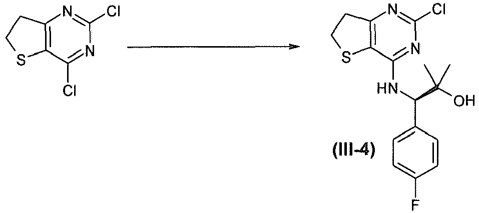

0,533 g **(II)**, 0,850 g (R)-1-Amino-1-(4-fluorphenyl)-2-methylpropan-2-ol und 1,3 ml Diisopropylethylamin werden in 9,8 ml Dioxan suspendiert. Das Reaktionsgemisch wird in der Mikrowelle 2 Stunden bei 80°C erhitzt und anschießend zur Trockne eingedampft. Der Rückstand wird mit Wasser versetzt. Der ausgefallene Niederschlag wird abgesaugt und chromatographisch (Kieselgel, Petrolether/Essigester 100/0 bis 60/40) gereinigt. 0,260 g **(III-4)** werden als Feststoff erhalten. Analytische HPLC-MS (Methode A): 1,39 min.

### 4.6 (R)-1-(2-Chlor-5-oxo-6,7-dihydro-5H-5λ⁴-thieno[3,2-d]pyrimidin-4-ylamino)-1-(4-fluorphenyl)-2-methylpropan-2-ol (IV-4):

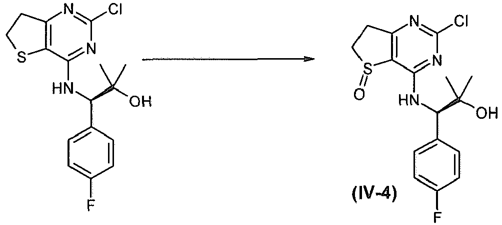

0,24 g S-(-)-1,1'-Bi-2-naphtol werden in 4 ml Chloroform unter Argon vorgelegt, dann 0,125 ml Titan(IV)-isopropylat und 0,15 ml Wasser zugegeben. Das Reaktionsgemisch wird 1 Stunde bei Raumtemperatur gerührt. Anschließend wird eine Suspension von 1,51 g (III-4) in 26 ml Chloroform zugegeben. Das Reaktionsgemisch wird auf -6°C abgekühlt und nach 30 Minuten werden 0,78 ml tert-Butylhydroperoxid 5-6 M in Decan zugetropft. Das Reaktionsgemisch wird bei -6 °C, bis keine weitere Umsetzung erfolgt, weitergerührt und mit NH₄OH basisch gestellt. Das Produkt wird mit Dichlormethan extrahiert und chromatographisch (Dichlormethan/Methanol 100/0 bis 95/5) gereinigt. 0,62 g **(IV-4)** werden als Feststoff erhalten. Analytische HPLC-MS (Methode A): RT = 1,19 min.

### 4.7 (R)-1-{2-[4-(4-Chlorphenyl)-piperidin-1-yl]-5-oxo-6,7-dihydro-5H-5λ⁴-thieno[3,2-d]pyrimidin-4-ylamino}-1-(4-fluorphenyl)-2-methylpropan-2-ol (Beispiel 1.4)

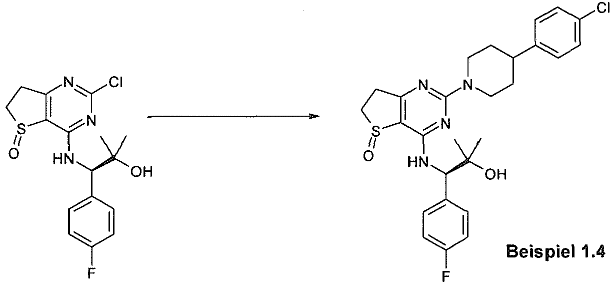

Ausgehend von 0,24 g **(IV-4)** und 0,15 g 4-(4-Chlorphenyl)-piperidin werden 0,19 g **Beispiel 1.4** analog zu Beispiel 1.1 (siehe 1.3) hergestellt. Das Produkt wird chromatographisch (Dichlormethan/Methanoi 100/0 bis 96/4) gereinigt. Analytische HPLC-MS (Methode A): RT = 1,36 min.

### 5. SYNTHESE VON (S)-5-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.5)

### 5.1 (S)-5-Dibenzylaminopiperidin-2-on:

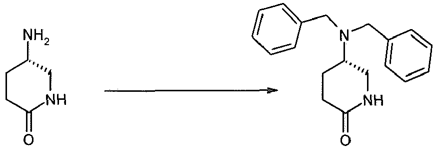

0,600 g 4-(S)-Amino-delta-valerolactam Hydrochlorid, 0,970 ml Benzylbromid und 1,5 g Natriumhydrogencarbonat werden in 30 ml Ethanol suspendiert. Das Reaktionsgemisch wird dann 8 Stunden bei 80°C gerührt und anschließend zur Trockne eingedampft. Der Rückstand wird in Wasser suspendiert und das Produkt mit Dichlormethan extrahiert und chromatographisch (Kieselgel, Dichlormethan/Methanol 100/0 bis 95/5) gereinigt. 0,500 g Produkt werden als Öl erhalten. Analytische HPLC-MS (Methode A): RT = 1,01 min.

### 5.2 (S)-5-Dibenzylamino-1-methylpiperidin-2-on:

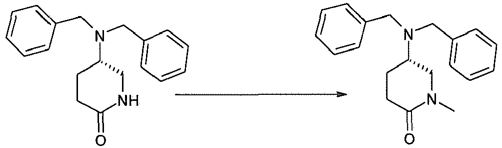

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g Produkt werden als Öl erhalten. Analytische HPLC-MS (Methode A): RT = 1,07 min.

### 5.3 (S)-5-Amino-1-methylpiperidin-2-on:

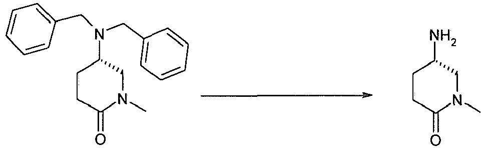

0,450 g (S)-5-Dibenzylamino-1-methylpiperidin-2-on werden in 25 ml Methanol suspendiert und mit 0,150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 5.4 (S)-5-(2-Ch)or-6,7-dihydrothieno[3,2-d]pyrimidin-4-ylamino)-1-methylpiperidin-2-on (III-5):

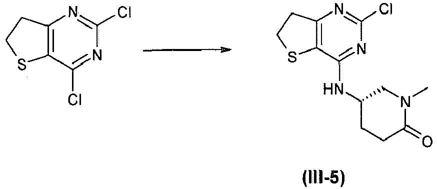

0,27 g **(II)** werden in 3 ml Dioxan vorgelegt, erst werden 0,45 ml Diisopropylethylamin, dann 0,25 g (S)-5-Amino-1-methylpiperidin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode A) gereinigt. 0,26 g **(III-5)** werden als Feststoff erhalten. Analytische HPLC-MS (Methode A): RT = 1,06 min.

### 5.5 (S)-5-(2-Chlor-5-oxo-6,7-dihydro-5H-5λ⁴-thieno[3,2-d]pyrimidin-4-ylamino)-1-methylpiperidin-2-on (IV-5):

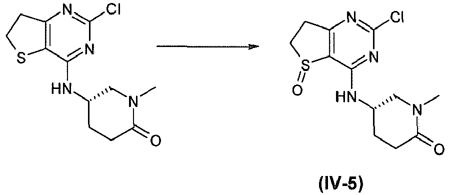

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g **(III-5)** in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird chromatographisch (Kieselgel, Essigester/Methanol 100/0 bis 60/40) gereinigt. 0,09 g **(IV-5)** werden als Feststoff erhalten. Analytische HPLC-MS (Methode A): RT = 0,83 min.

### 5.6 (S)-5-{2-[4-(4-Chlorphenyl)-piperidin-1-yl]-5-oxo-6,7-dihydro-5H-5λ⁴-thieno[3,2-d]pyrimidin-4-ylamino}-1-methylpiperidin-2-on (Beispiel 1.5)

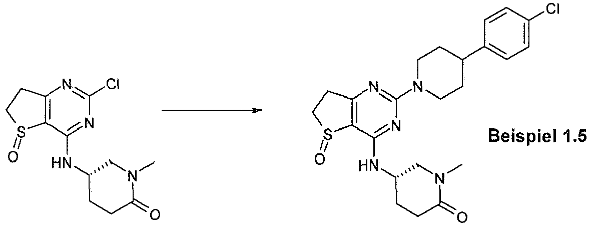

Ausgehend von 0,2 g **(IV-5)** und 0,18 g 4-(4-Chlorphenyl)-piperidin werden 0,17 g **Beispiel 1.5** analog zu Beispiel 1.1 (siehe 1.3) hergestellt. Das Produkt wird chromatographisch (präparative HPLC, Methode A) gereinigt. Die Produktfraktionen werden mit Ammoniak basisch gestellt und gefriergetrocknet. Analytische HPLC-MS (Methode A): RT = 1,18 min

### 6. SYNTHESE VON {2-[4-(4-CHLORPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.6)

### 6.1 (2-Chlor-6,7-dihydrothieno[3,2-d]pyrimidin-4-yl)-(tetrahydropyran-4-yl)-amin (III-6):

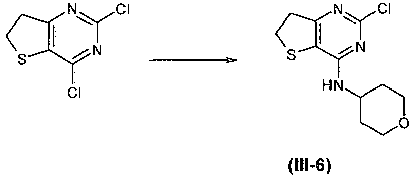

0,68 g **(II)** werden in 6 ml Dioxan vorgelegt, erst werden 1,72 ml Diisopropylethylamin, dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt, dann abgesaugt und getrocknet. 0,66 g **(III-6)** werden als Feststoff erhalten. Analytische HPLC-MS (Methode C): RT = 1,08 min.

### 6.2 (2-Chlor-5-oxo-6,7-dihydro-5H-5λ⁴-thieno[3,2-d]pyrimidin-4-yl)-(tetrahydropyran-4-yl)-amin (IV-6):

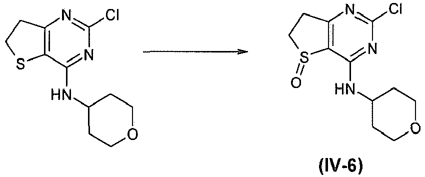

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g **(III-6)** in 25 ml Chloroform zugegeben. Das Reaktionsgemisch wird auf -10°C abgekühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g **(IV-6)** werden als Feststoff erhalten. Analytische HPLC-MS (Methode A): RT = 0,94 min.

### 6.3 {2-[4-(4-Chlorphenyl)-piperidin-1-yl]-5-oxo-6,7-dihydro-5H-5λ⁴-thieno[3,2-d]pyrimidin-4-yl}-(tetrahydropyran-4-yl)-amin (Beispiel 1.6)

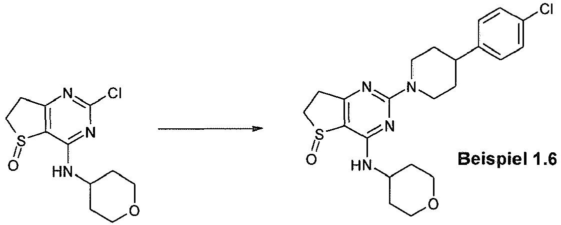

Ausgehend von 0,18 g **(IV-6)** und 0,17 g 4-(4-Chlorphenyl)-piperidin werden 0,23 g **Beispiel 1.6** analog zu Beispiel 1.1 (siehe 1.3) hergestellt. Das Produkt wird mit Wasser im Ultraschallbad behandelt und der Feststoff abgesaugt. Analytische HPLC-MS (Methode A): RT = 1,24 min

### 7. SYNTHESE VON 1-(4-(1-HYDROXYMETHYLCYCLOPROPYLAMINO)-5-OXO-6,7-DIHYDRO-5H-5λ4-THIENO[3,2-d]PYRIMIDIN-2-YL)-3'-METHYL-1'H-SPIRO[PIPERIDIN-4,4'-CHINAZOLIN]-2'(3'H)-ON (BEISPIEL 1.7)

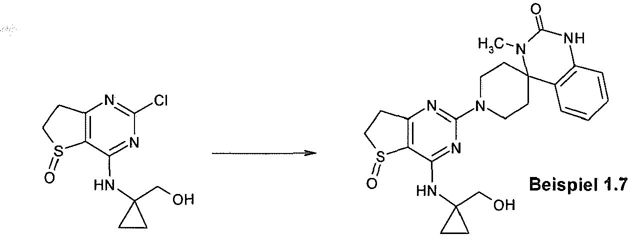

Ausgehend von **(IV-2)** (siehe 2.4) und 3'-methyl-1'H-spiro[piperidin-4,4'-chinazolin]-2'(3'*H*)-on (Chem. Pharm. Bull. 1988, 4659) (0,1 mmol) in 400µl NMP versetzt und 30 min bei 120°C in der Mikrowelle erhitzt. Anschliessend werden 600 µL DMF zugesetzt, die Reaktionslösung über präparative HPLC-MS (Methode A) aufgereinigt und die Produktfraktionen gefriergetrocknet. Analytische HPLC-MS (Methode C): RT = 1,52 min.

### 8. SYNTHESE VON {1-[2-(4-BENZO[d]ISOXAZOL-3-YL-PIPERIDIN-1-YL)-5-OXO-6,7-DIHYDRO-5H-5λ4-THIENO[3,2-d]PYRIMIDIN-4-YLAMINO]-CYCLOPROPYL}-METHANOL (BEISPIEL 1.8)

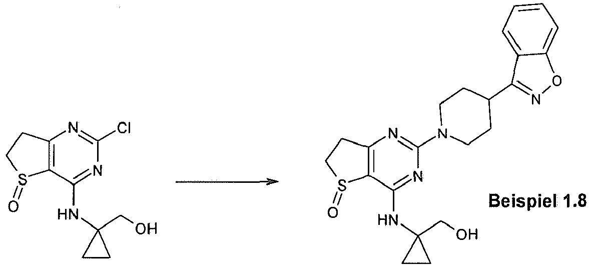

Ausgehend von **(IV-2)** (siehe 2.4) und 3-Piperidin-4-yl-benzo[*d*]isoxazol kann **Beispiel 1.8** analog zu Beispiel 1.7 (siehe 7.) hergestellt und aufgereinigt werden. Analytische HPLC-MS (Methode C): RT = 1,7 min.

### 9. SYNTHESE VON (1-{2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YLAMINO)-CYCLOPROPYL)-METHANOL (BEISPIEL 1.9)

### 9.1 2-But-1-ynyl-4-fluorphenylamin

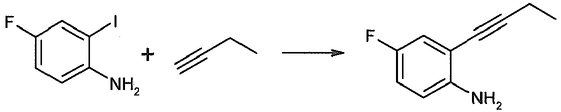

80 ml Tetrahydrofuran wird unter Argon vorgelegt. 5 g 4-Fluor-2-iodphenylamin, 0,74 g Dichlorbis(triphenylphosphin) palladium(II), 0,2 g Kupferiodid und 8,8 ml Triethylamin werden zugegeben. 4 g gasförmiges 1-Butin werden durch die Suspension geleitet. Das Reaktionsgemisch wird unter Argon 15 Stunden bei Raumtemperatur gerührt, dann über Celite filtriert und zur Trockne eingedampft. 3,4 g Produkt werden als Feststoff erhalten. ¹H NMR (400 MHz, DMSO): 2,45 (2H, q); 1,18 (3H, t).

### 9.2 2-Ethyl-5-fluor-1H-indol

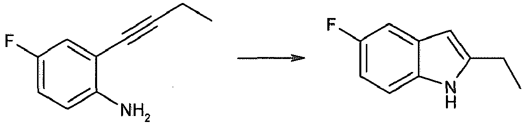

Unter Argon werden 4,9 g Kalium-*tert*-butylat in 25 ml N-Methyl-2-pyrrolidinon suspendiert und eine Suspension von 3,4 g 2-But-1-ynyl-4-fluorphenylamin in 25 ml N-Methyl-2-pyrrolidinon dazu getropft. Das Reaktionsgemisch wird 3 Stunden bei Raumtemperatur gerührt und mit Wasser versetzt. Das Produkt wird mit Diethylether extrahiert und chromatographisch (Kieselgel, Cyclohexan/Essigester 100/0 - 90/10) gereinigt. 2,83 g Produkt werden als Feststoff erhalten. ¹H NMR (400 MHz, DMSO): 2,72 (2H, q); 1,27 (3H, t).

### 9.3 2-Ethyl-5-fluor-3-(1,2,3,6-tetrahydropyridin-4-yl)-1H-indol

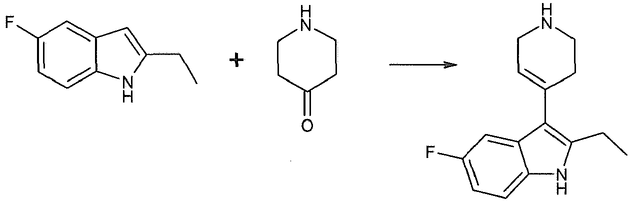

2,83 g 2-Ethyl-5-fluor-1H-indol werden in 50 ml Essigsäure suspendiert und auf 90°C erwärmt. Eine Suspension von 6,66 g 4-Piperidon in 15 ml Phosphorsäure 2N wird zugegeben. Das Reaktionsgemisch wird 3 Stunden bei 90°C gerührt, mit Natronlauge versetzt und das Produkt mit Essigester extrahiert. 2,85 g Produkt werden als Feststoff erhalten. ¹H NMR (400 MHz, DMSO): 5,63 (1H, s); 2,73 (2H, q); 1,23 (3H, t).

### 9.4 2-Ethyl-5-fluor-3-piperidin-4-yl-1H-indol (V-1)

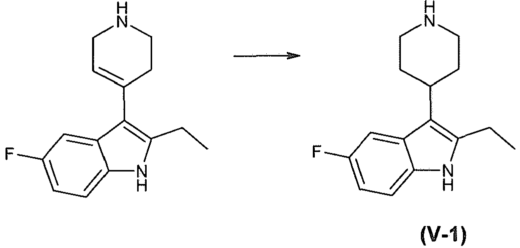

2,83 g 2-Ethyl-5-fluor-3-(1,2,3,6-tetrahydropyridin-4-yl)-1*H*-indol werden in 50 ml Methanol suspendiert und mit 0,3 g Pd/C 10% bei Normaldruck und Raumtemperatur hydriert. Der Katalysator wird abgesaugt und das Filtrat zur Trockne eingedampft. 2,3 g **(V-1)** werden als Feststoff erhalten. ¹H NMR (400 MHz, DMSO): 2,70 (2H, q); 1,19 (3H, t).

### 9.5 (1-{2-[4-(2-Ethyl-5-fluor-1H-indol-3-yl)-piperidin-1-yl]-5-oxo-6,7-dihydro-5H-5λ⁴-thieno[3,2-d]pyrimidin-4-ylamino}-cyclopropyl)-methanol (Beispiel 1.9)

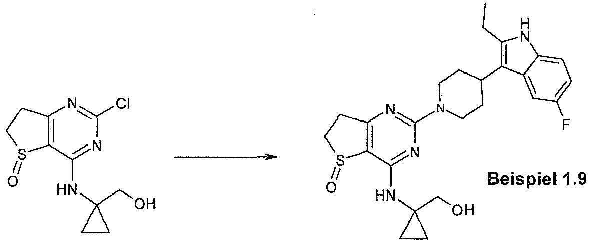

Ausgehend von **(IV-2)** (siehe 2.4) und **(V-1)** kann **Beispiel 1.9** analog zu Beispiel 1.7 (siehe 7.) hergestellt und aufgereinigt werden. Analytische HPLC-MS (Methode C): RT = 1,78 min.

### 10. SYNTHESE VON 1-[4-((S)-1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-CARBONITRIL (BEISPIEL 1.10)

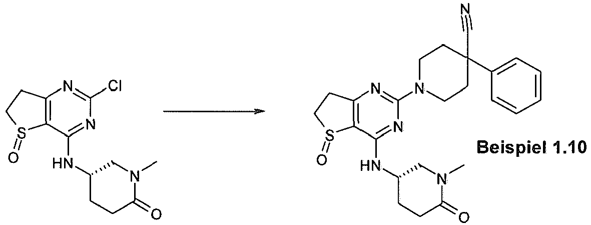

Ausgehend von **(IV-5)** (siehe 5.5) und 4-Phenylpiperidin-4-carbonitril
kann **Beispiel 1.10** analog zu Beispiel 1.7 (siehe 7) hergestellt und aufgereinigt werden. Analytische HPLC-MS (Methode C): RT = 1,71 min.

### 11. SYNTHESE VON 3'-METHYL-1-(4-(TETRAHYDRO-2H-PYRAN-4-YLAMINO)-5-OXO-6,7-DIHYDRO-5H-5λ⁴-THIENO[3,2-d]PYRIMIDIN-2-YL)-1'H-SPIRO[PIPERIDIN-4,4'-CHINAZOLIN]-2'(3'H)-ON (BEISPIEL 1.11)

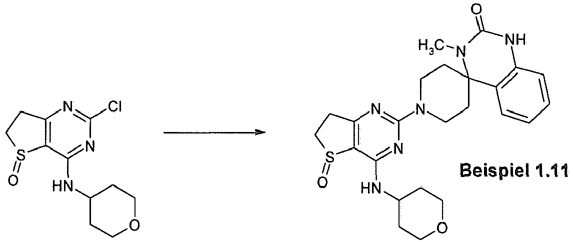

Ausgehend von **(IV-6)** (siehe 6.2) und 3'-methyl-1'*H*-spiro[piperidin-4,4'-chinazolin]-2'(3'H)-on (Chem. Pharm. Bull. 1988, 4659) kann **Beispiel 1.11** analog zu Beispiel 1.7 (siehe 7.) hergestellt und aufgereinigt werden. Analytische HPLC-MS (Methode C): RT = 1,56 min.

### 12. SYNTHESE VON (3-FLUORPHENYL)-[5-OXO-2-(3,4,5,6-TETRAHYDRO-2H-[4,4']BIPYRIDINYL-1-YL)-6,7-DIHYDRO-5H-5λ⁴-THIENO[3,2-d]PYRIMIDIN-4-YL]-AMIN (BEISPIEL 1.12)

### 12.1 (2-Chlor-6,7-dihydrothieno[3,2-d]pyrimidin-4-yl)-(3-fluorphenyl)-amin (III-7):

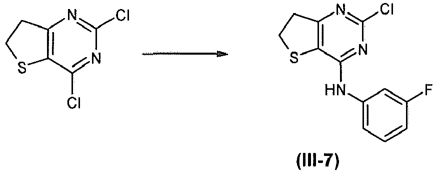

4 g **(II)** werden in 15 ml Dimethylformamid vorgelegt, anschliessend werden 4,5 ml Diisopropylethylamin und dann 2,5 ml 3-Fluorphenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g **(III-7)** werden als Feststoff erhalten. Analytische HPLC (Methode A): RT = 3,27 min

### 12.2 2-Chlor-5-oxo-6,7-dihydro-5H-5λ⁴-thieno[3,2-d]pyrimidin-4-yl)-(3-fluorphenyl)-amin (IV-7):

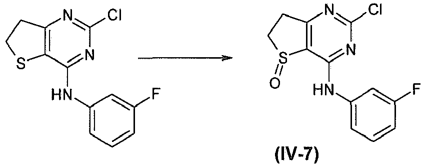

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g **(III-7)** in 25 ml Chloroform zugegeben. Das Reaktionsgemisch wird auf -2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g **(IV-7)** werden als Feststoff erhalten. Analytische HPLC-MS (Methode A): RT = 1,15 min.

### 12.3 (3-Fluorphenyl)-[5-oxo-2-(3,4,5,6-tetrahydro-2H-[4,4']bipyridinyl-1-yl)-6,7-dihydro-5H-5λ⁴-thieno[3,2-d]pyrimidin-4-yl]-amin (Beispiel 1.12)

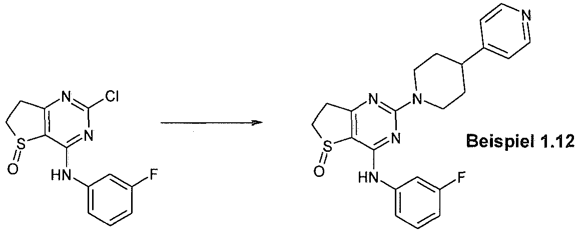

Ausgehend von **(IV-7)** (siehe 12.2) und 1,2,3,4,5,6-Hexahydro-[4,4']bipyridinyl kann **Beispiel 1.12** als Trifluoracetat analog zu Beispiel 1.7 (siehe 7.) hergestellt und aufgereinigt werden. Analytische HPLC-MS (Methode C): RT = 1,55 min.

### 13. SYNTHESE VON {2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 1.13)

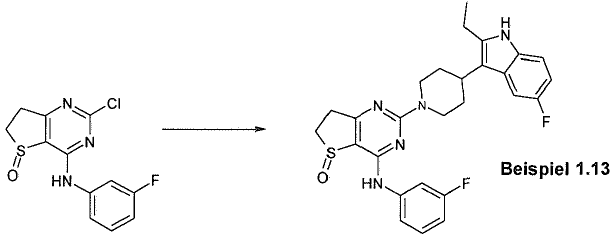

Ausgehend von **(IV-7)** (siehe 12.2) und **(V-1)** (siehe 9.4) kann **Beispiel 1.13** analog zu Beispiel 1.7 (siehe 7.) hergestellt und aufgereinigt werden. Analytische HPLC-MS (Methode C): RT = 2,12 min.

### 14. SYNTHESE VON (1-{2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.14)

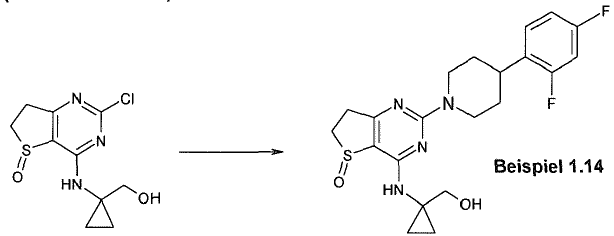

Ausgehend von **(IV-2)** (siehe 2.4) und 4-(2,4-Difluorphenyl)-piperidin kann **Beispiel 1.14** analog zu Beispiel 1.7 (siehe 7.) hergestellt werden. Das Produkt kann chromatographisch (präparative HPLC, Methode B) gereinigt werden. Analytische HPLC-MS (Methode D): RT = 1,18 min.

### 15. SYNTHESE VON {2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.15)

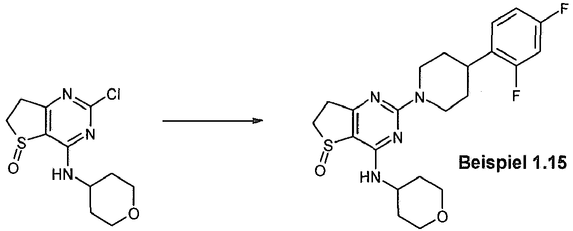

Ausgehend von **(IV-6)** (siehe 6.2) und 4-(2,4-Difluorphenyl)-piperidin kann **Beispiel 1.15** analog zu Beispiel 1.14 (siehe 14.) hergestellt und aufgereinigt werden. Analytische HPLC-MS (Methode D): RT = 1,23 min.

### 16. SYNTHESE VON (S)-5-[2-(4-BENZOXAZOL-2-YL-PIPERIDIN-1-YL)-5-OXO-6,7-DIHYDRO-5H-5λ⁴-THIENO[3,2-d]PYRIMIDIN-4-YLAMINO]-1-METHYLPIPERIDIN-2-ON (BEISPIEL 1.16)

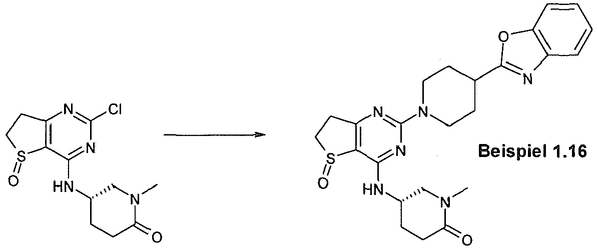

Ausgehend von **(IV-5)** (siehe 5.5) und 2-Piperidin-4-yl-benzoxazol kann **Beispiel 1.16** analog zu Beispiel 1.14 (siehe 14.) hergestellt und aufgereinigt werden. Analytische HPLC-MS (Methode B): RT = 1,18 min.

### 17. SYNTHESE VON (1-{2-[4-(6-FLUORBENZO[d]ISOXAZOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.17)

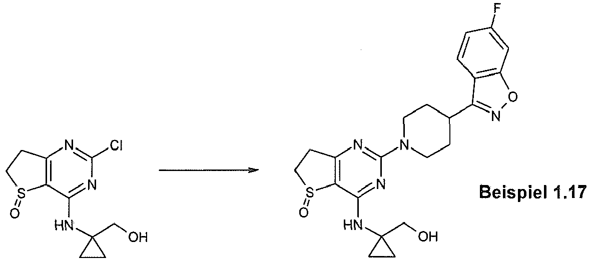

Ausgehend von **(IV-2)** (siehe 2.4) und 6-Fluor-3-piperidin-4-yl-benzo[d]isoxazol
kann **Beispiel 1.17** analog zu Beispiel 1.7 (siehe 7.) hergestellt und aufgereinigt werden. Analytische HPLC-MS (Methode C): RT = 1,76 min.

### 18. SYNTHESE VON (1-{2-[4-(5-FLUORBENZO[d]ISOXAZOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.18)

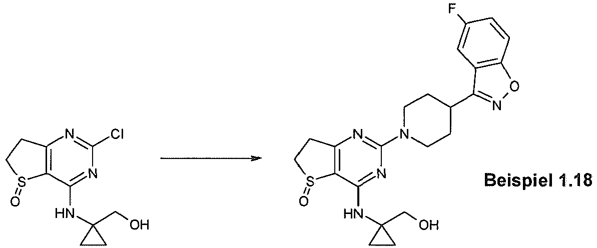

Ausgehend von **(IV-2)** (siehe 2.4) und 5-Fluor-3-piperidin-4-yl-benzo[*d*]isoxazol
kann **Beispiel 1.17** analog zu Beispiel 1.7 (siehe 7.) hergestellt und aufgereinigt werden. Analytische HPLC-MS (Methode C): RT = 1,74 min.

### 19. SYNTHESE VON {2-[4-(5-FURAN-2-YL-2H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.19)

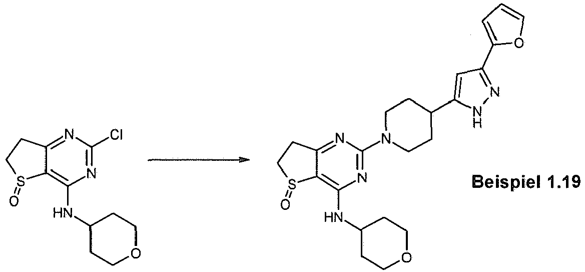

Ausgehend von **(IV-6)** (siehe 6.2) und 4-(5-Furan-2-yl-2H-pyrazol-3-yl)-piperidin kann **Beispiel 1.19** analog zu Beispiel 1.11 (siehe 11.) hergestellt und aufgereinigt werden. Analytische HPLC-MS (Methode C): RT = 1,64 min.

### 20. SYNTHESE VON (3-FLUORPHENYL)-{5-OXO-2-[4-(3-PYRIDIN-4-YL-[1,2,4] OXADIAZOL-5-YL)-PIPERIDIN-1-YL]-6,7-DIHYDRO-5H-5λ⁴-THIENO[3,2-d]PYRIMIDIN-4-YL}-AMIN (BEISPIEL 1.20)

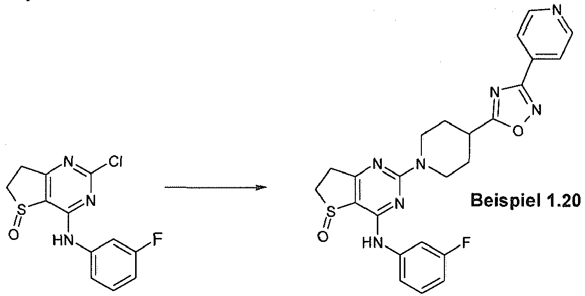

Ausgehend von **(IV-7)** (siehe 12.2) und 4-(5-Piperidin-4-yl-[1,2,4]oxadiazol-3-yl)-pyridin kann Beispiel 1.20 als Trifluoracetat analog zu Beispiel 1.7 (siehe 7.) hergestellt und aufgereinigt werden. Analytische HPLC-MS (Methode C): RT = 1,72 min.

### 21. SYNTHESE VON (R)-3-METHYL-2-{5-OXO-2-[4-(3-PYRIDIN-4-YL-[1,2,4] OXADIAZOL-5-YL)-PIPERIDIN-1-YL]-6,7-DIHYDRO-5H-5λ⁴-THIENO[3,2-d]PYRIMIDIN-4-YLAMINO}-BUTAN-1-OL (BEISPIEL 1.21)

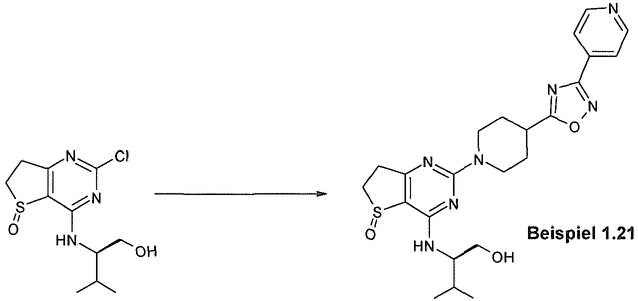

Ausgehend von **(IV-1)** (siehe 1.2) und 4-(5-Piperidin-4-yl-[1,2,4]oxadiazol-3-yl)-pyridin kann **Beispiel 1.21** als Trifluoracetat analog zu Beispiel 1.7 (siehe 7.) hergestellt und aufgereinigt werden. Analytische HPLC-MS (Methode C): RT = 1,48 min.

### 22. SYNTHESE VON: (S)-5-{2-[4-(4-FLUORPHENOXY)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.22)

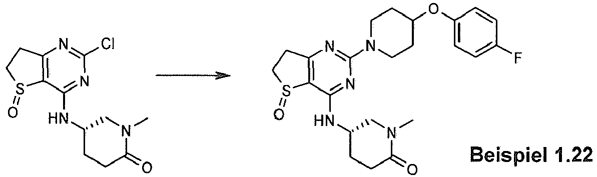

Ausgehend von **(IV-5)** (siehe 5.5) und 4-(4-Fluorphenoxy)-piperidin kann **Beispiel 1.22** analog zu Beispiel 1.16 (siehe 16.) hergestellt und aufgereinigt werden. Analytische HPLC-MS (Methode A): RT = 1,15 min.

### 23. SYNTHESE VON: (2-{4-[4-(4,5-DIHYDROOXAZOL-2-YL)-PHENOXY]-PIPERIDIN-1-YL}-5-OXO-6,7-DIHYDRO-5H-5λ⁴-THIENO[3,2-d]PYRIMIDIN-4-YL)-(TETRAHYDRO PYRAN-4-YL)-AMIN (BEISPIEL 1.23)

### 23.1 4-(Toluol-4-sulfonyloxy)-piperidin-1-carbonsäure-tert-butylester:

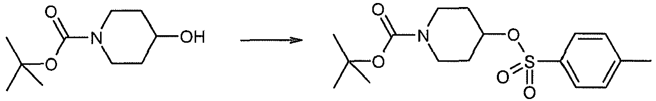

5 g 4-Hydroxypiperidin-1-carbonsäure-*tert*-butylester werden in 15 ml Pyridin vorgelegt, dann 4,7 g p-Toluolsulfonylchlorid portionsweise zugegeben. Das Reaktionsgemisch wird bei Raumtemperatur gerührt, nach 12 Stunden auf Eiswasser gegossen und die erhaltene Mischung eine weitere Stunde bei Raumtemperatur gerührt. Der ausgefallene Feststoff wird abgesaugt und getrocknet. 7,5 g Produkt werden erhalten.

### 23.2 4-[4-(4,5-Dihydrooxazol-2-yl)-phenoxy]-piperidin-1-carbonsäure-tert-butylester:

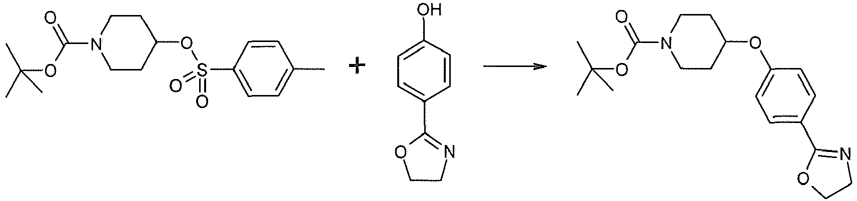

2,0 g 4-(4,5-Dihydrooxazol-2-yl)-phenol (siehe US5491201) werden in 30 ml Dimethylformamid vorgelegt, dann 3,3 g Kaliumcarbonat und 4,2 g 4-(Toluol-4-sulfonyloxy)-piperidin-l-carbonsäure-tert-butylester zugegeben. Das Reaktionsgemisch wird bei 75°C gerührt, nach 12 Stunden mit Wasser versetzt und der ausgefallene Feststoff abgesaugt und getrocknet. 2,8 g Produkt werden erhalten.

### 23.3 4-[4-(4,5-Dihydrooxazol-2-yl)-phenoxy]-piperidin (V-2):

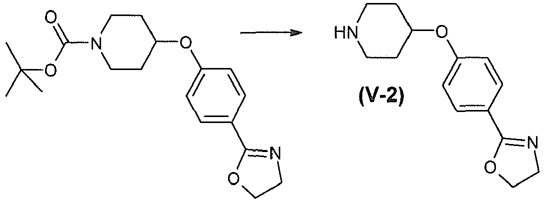

50 mg 4-[4-(4,5-Dihydrooxazol-2-yl)-phenoxy]-piperidin-1-carbonsäure-*tert*-butylester werden vorgelegt und mit 6 ml einer (5/1) Dichlormethan/Trifluoressigsäure Mischung versetzt. Das Reaktionsgemisch wird bei Raumtemperatur gerührt und nach 15 min vorsichtig mit einer gesättigten NaHCO₃ Lösung versetzt. Die organische Phase wird getrocknet und zur Trockne eingedampft. 20 mg **(V-2)** werden erhalten.

### 23.4 (2-{4-[4-(4,5-Dihydrooxazol-2-yl)-phenoxy]-piperidin-1-yl}-5-oxo-6,7-dihydro-5H-5λ⁴-thieno[3,2-d]pyrimidin-4-yl)-(tetrahydropyran-4-yl)-amin (Beispiel 1.23)

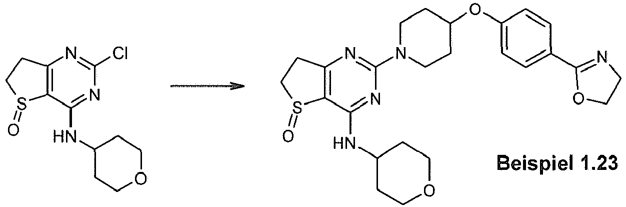

Ausgehend von **(IV-6)** (siehe 6.2) und **(V-2)** kann **Beispiel 1.23** analog zu Beispiel 1.14 (siehe 14.) hergestellt und aufgereinigt werden. Analytische HPLC-MS (Methode A): RT = 0,99 min.

### 24. SYNTHESE VON: 4-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-BENZOESÄURE (BEISPIEL 1.24)

### 24.1 4-{1-[5-Oxo-4-(tetrahydropyran-4-ylamino)-6,7-dihydro-5H-5λ⁴-thieno[3,2-d]pyrimidin-2-yl]-piperidin-4-yloxy}-benzoesäuremethylester

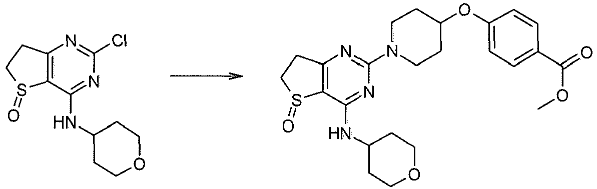

Ausgehend von **(IV-6)** (siehe 6.2) und 4-(Piperidin-4-yloxy)-benzoesäuremethylester (J. Med. Chem. 2002, 3406) kann 4-{1-[5-Oxo-4-(tetrahydropyran-4-ylamino)-6,7-dihydro-5*H*-5λ⁴-thieno[3,2-d]pyrimidin-2-yl]-piperidin-4-yloxy}-benzoesäuremethylester analog zu Beispiel 1.15 (siehe 15.) hergestellt und aufgereinigt werden. Analytische HPLC-MS (Methode A): RT = 1.17 min.

### 24.2 4-{1-[5-Oxo-4-(tetrahydropyran-4-ylamino)-6,7-dihydro-5H-5λ⁴-thieno[3,2-d]pyrimidin-2-yl]-piperidin-4-yloxy}-benzoesäure (Beispiel 1.24)

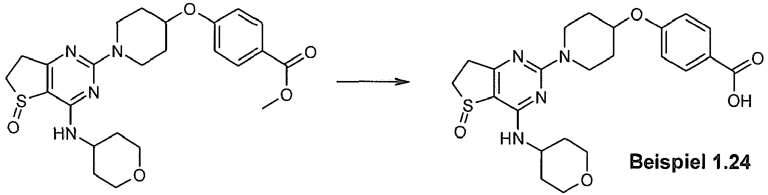

80 mg von 4-{1-[5-Oxo-4-(tetrahydropyran-4-ylamino)-6,7-dihydro-5*H*-5λ⁴-thieno[3,2-*d*]pyrimidin-2-yl]-piperidin-4-yloxy}-benzoesäuremethylester werden in 1,5 ml Methanol vorgelegt, dann 560 µl einer 1 N NaOH Lösung zugegebenDas Reaktionsgemisch wird bei 50°C, bis keine weitere Umsetzung erfolgt, gerührt, dann mit einer 1 M HCl Lösung versetzt. Das Produkt wird mit Dichlormethan extrahiert. 77 mg **Beispiel 1.24** werden als Feststoff erhalten. Analytische HPLC-MS (Methode B): RT = 1,19 min.

### 25. SYNTHESE VON 2-(1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-PROPAN-2-OL (BEISPIEL 1.25)

### 25.1 2-[1-(2-Chlor-6,7-dihydrothieno[3,2-d]pyrimidin-4-ylamino)-cyclopropyl]-propan-2-ol (III-8):

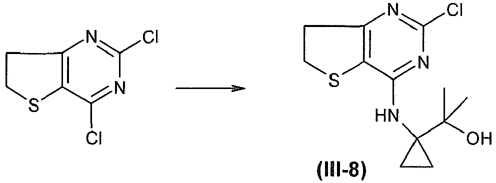

2,7 g **(II)** werden in 30 ml Dioxan vorgelegt, dann 6,8 ml Diisopropyl-ethylamin und 1,8 g 2-(1-Aminocyclopropyl)-propan-2-ol (siehe Liebigs Ann. Chem. 1978,1194) zugegeben. Das Reaktionsgemisch wird, bis keine weitere Umsetzung erfolgt, bei 160°C erhitzt und nach Abkühlen zur Trockne eingedampft. Der Rückstand wird mit Eiswasser versetzt. Das Produkt wird mit Dichlormethan extrahiert und chromatographisch gereinigt. 125 mg **(III-8)** werden als Feststoff erhalten. Analytische HPLC-MS (Methode A): RT = 1,08 min.

### 25.2 2-[1-(2-Chlor-5-oxo-6,7-dihydro-5H-5λ⁴--thieno[3,2-d]pyrimidin-4-ylamino)-cyclopropyl]-propan-2-ol (IV-8):

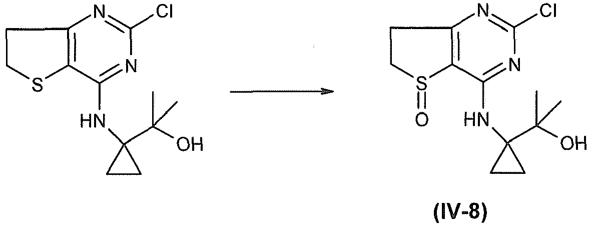

21,6 mg S-(-)-1,1'-Bi-2-naphtol werden in 1 ml Chloroform unter Argon vorgelegt, dann 11 µl Titan(IV)-isopropylat und 14 µl Wasser zugegeben. Das Reaktionsgemisch wird 1 Stunde bei Raumtemperatur gerührt. Anschließend wird eine Mischung von 120 mg **(III-8)** in 4 ml Dichlormethan zugegeben. Das Reaktionsgemisch wird auf -5°C abgekühlt und nach 30 Minuten werden 69,5 µl *tert*-Butylhydroperoxid 5-6 M in Decan zugetropft. Das Reaktionsgemisch wird bei -5 °C gerührt. Nach 2 Tage werden nochmal die gleichen Mengen von S-(-)-1,1'-Bi-2-naphtol, Titan(IV)-isopropylat, Wasser und tert-Butylhydroperoxid zugegeben. Das Reaktionsgemisch wird bei -5°C bis 5°C weitergerührt bis keine weitere Umsetzung erfolgt, mit Wasser versetzt und mit NH₄OH basisch gestellt. Die organische Phase wird zur Trockne eingedampft und das Produkt chromatographisch (präparative HPLC, Methode B) gereinigt. 105 mg **(IV-8)** werden als erhalten. Analytische HPLC-MS (Methode A): RT = 0,96 min.

### 25.3 2-(1 1-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-propan-2-ol (Beispiel 1.25)

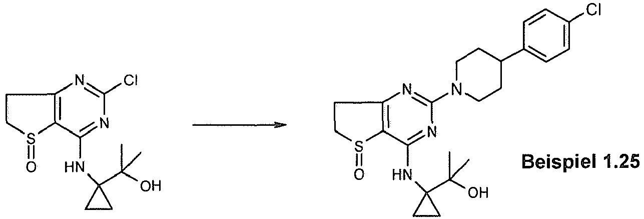

Ausgehend von **(IV-8)** und 4-(4-Chlorphenyl)-piperidin Hydrochlorid kann **Beispiel 1.25** analog zu Beispiel 1.14 (siehe 14.) hergestellt und aufgereinigt werden. Analytische HPLC-MS (Methode B): RT = 1,37 min.

### 26. SYNTHESE VON: {2-[4-(5-tert-BUTYL-1-METHYL-1H-INDOL-3-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 1.26)

### 26.1 4-(1H-Indol-3-yl)-piperidin-1-carbonsäure-tert-butylester:

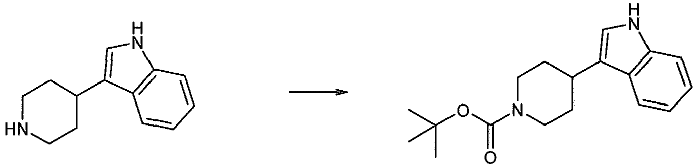

10 g 3-Piperidin-4-yl-1H-indol werden in 300 mL THF vorgelegt und 10,9 g Di-*tert*-butyldicarbonat zugegeben. Das Reaktionsgemisch wird über Nacht bei Raumtemperatur gerührt und zur Trockne eingedampft. Der Rückstand wird mit Wasser versetzt und das Produkt mit Diethylether extrahiert und chromatographisch gereinigt. 9 g des Produkts werden als Feststoff erhalten.

### 26.2 4-(1-Methyl-1H-indol-3-yl)-piperidin-1-carbonsäure-tert-butylester:

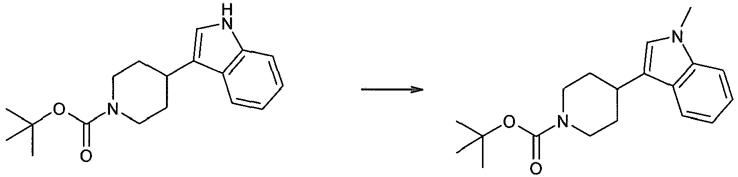

500 mg 4-(1H-Indol-3-yl)-piperidin-1-carbonsäure-*tert*-butylester werden in 8 ml Dimethylformamid vorgelegt und 73,3 mg Natriumhydrid (60% in Mineralöl) zugegeben. Nach 15 min werden 175 µl Methyliodid zugegeben. Das Reaktionsgemisch wird bei Raumtemperatur gerührt. Nach vollständiger Umsetzung wird das Produkt direkt über präparative HPLC (Methode C) gereinigt. 302 mg des Produkts werden als Öl erhalten. Analytische HPLC-MS (Methode A): RT = 1,65 min.

### 26.3 5-tert-Butyl-1-methyl-3-piperidin-4-yl-1H-indol (V-3)

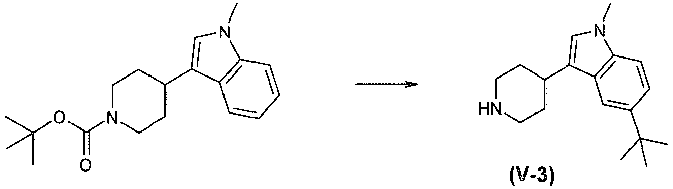

365 mg 4-(1-Methyl-1H-indol-3-yl)-piperidin-1-carbonsäure-tert-butylester werden in 1 ml Dichlormethan vorgelegt und mit 1,03 ml Trifluoressigsäure versetzt. Das Reaktionsgemisch wird bei Raumtemperatur gerührt. Nach 12 und 16 h werden nochmal 1,03 ml Trifluoressigsäure zugegeben. Nach weiteren 12 h wird das Reaktionsgemisch zur Trockne eingedampft. Der Rückstand wird mit Toluol versetzt und zur Trockne eingedampft. Der Rückstand wird mit Diethylether verrieben, der Niederschlag abgesaugt und getrocknet. 154 mg **(V-3)** werden als Feststoff erhalten. Analytische HPLC-MS (Methode A): RT = 1,34 min.

### 26.4 {2-[4-(5-tert-Butyl-1-methyl-1H-indol-3-yl)-piperidin-1-yl]-5-oxo-6,7-dihydro-5H-5λ⁴-thieno[3,2-d]pyrimidin-4-yl)-(tetrahydropyran-4-yl)-amin (Beispiel 1.26)

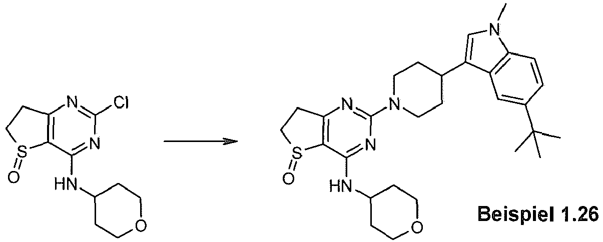

Ausgehend von **(IV-6)** (siehe 6.2) und **(V-3)** kann **Beispiel 1.26** analog zu Beispiel 1.14 (siehe 14.) hergestellt und aufgereinigt werden. Analytische HPLC-MS (Methode B): RT = 1,16 min.

### 27. SYNTHESE VON: {2-[4-(5-FURAN-2-YL-1-METHYL-1H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.27)

### 27.1 14-(5-Furan-2-yl-2H-pyrazol-3-yl)-piperidin-1-carbonsäure-tert-butylester:

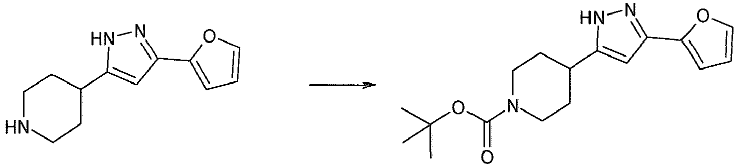

200 mg 4-(5-Furan-2-yl-2H-pyrazol-3-yl)-piperidin werden in 2 ml Dioxan vorgelegt. Anschließend werden 0,34 ml Wasser und 155 mg Natriumcarbonat zugegeben. Das Reaktionsgemisch wird beim Raumtemperatur gerührt. Nach 5 min werden 204 mg Di-tert-butyl-dicarbonat zugegeben. Nach 3 h wird das Reaktionsgemisch mit Wasser versetzt und das Produkt mit Dichlormethan extrahiert. 300 mg Produkt werden als Öl erhalten. Analytische HPLC-MS (Methode B): RT = 1,54 min.

### 27.2 4-(5-Furan-2-yl-2-methyl-2H-pyrazol-3-yl)-piperidin-1-carbonsäure-tert-butylester und 4-(5-Furan-2-yl-1-methyl-1H-pyrazol-3-yl)-piperidin-1-carbonsäure-tert-butylester:

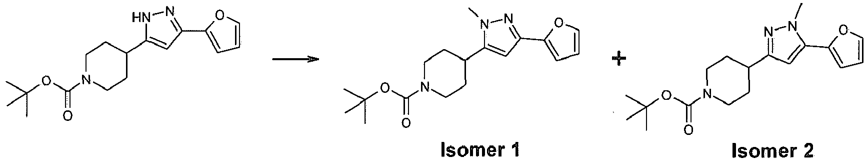

250 mg 14-(5-Furan-2-yl-2H-pyrazol-3-yl)-piperidin-1-carbonsäure-*tert*-butylester werden in 1,5 ml Dimethylformamid vorgelegt. Das Reaktionsgemisch wird im Eisbad abgekühlt und 40 mg Natriumhydrid (60% in Mineralöl) werden zugegeben. Nach 10 min werden 60 µl Methyliodid zugegeben. Das Reaktionsgemisch wird 30 min bei 5°C und anschliessend 4 h bei Raumtemperatur gerührt. Das Produkt wird dann direkt über präparative HPLC (Methode D) aufgereinigt. 90 mg **Isomer 1** und 50 mg **Isomer 2** werden als Feststoff erhalten. Analytische HPLC-MS (Methode D): RT = 1,33 min (Isomer 1); RT = 1,28 (Isomer 2).

### 27.3 4-(5-Furan-2-yl-1-methyl-1H-pyrazol-3-yl)-piperidin (V-4):

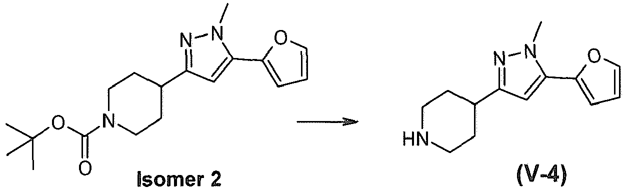

47 mg **Isomer 2** werden in 1 ml Dichlormethan vorgelegt und 120 µl Trifluoressigsäure zugegeben. Das Reaktionsgemisch wird 2h bei Raumtemperatur gerührt, dann zur Trockne eingedampft. Der Rückstand wird mit Toluol versetzt und zur Trockne eingedampft. Der Rückstand wird mit Wasser versetzt, mit konz. Ammoniak basisch gestellt und das Produkt mit Dichlormethan extrahiert. 23 mg **(V-4)** werden als Feststoff erhalten. Analytische HPLC-MS (Methode B): RT = 0,85 min

### 27. 4 {2-[4-(5-Furan-2-yl-1-methyl-1H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.27)

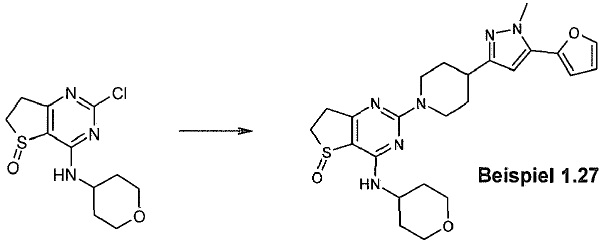

Ausgehend von **(IV-6)** (siehe 6.2) und **(V-4)** kann **Beispiel 1.27** analog zu Beispiel 1.14 (siehe 14) hergestellt und aufgereinigt werden. Analytische HPLC-MS (Methode B): RT = 1,21 min.

### 28. SYNTHESE VON: (S)-5-(2-{4-[4-(4,5-DIHYDROOXAZOL-2-YL)-PHENOXY]-PIPERIDIN-1-YL}-5-OXO-6,7-DIHYDRO-5H-5λ⁴-THIENO[3,2-d]PYRIMIDIN-4-YLAMINO)-1-METHYLPIPERIDIN-2-ON (BEISPIEL 1.28)

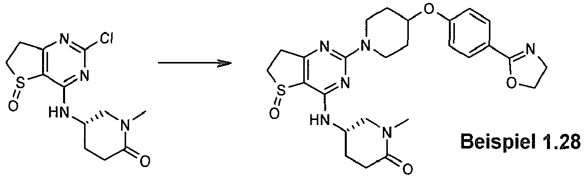

Ausgehend von **(IV-5)** (siehe 5.5) und **(V-2)** (siehe 23.3) kann **Beispiel 1.28** analog zu Beispiel 1.16 (siehe 16.) hergestellt und aufgereinigt werden. Analytische HPLC-MS (Methode B): RT = 1.07 min.

### 29. SYNTHESE VON: {2-[4-(5-FURAN-2-YL-2-METHYL-2H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.29)

### 29.1 4-(5-Furan-2-yl-2-methyl-2H-pyrazol-3-yl)-piperidin (V-5):

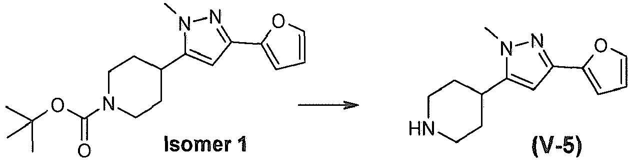

Ausgehend von **Isomer 1** (siehe 27.2), kann **(V-5)** analog zu **(V-4)** (siehe 27.3) hergestellt werden. Analytische HPLC-MS (Methode D): RT = 0,89 min.

### 29. 2 Synthese von: {2-[4-(5-Furan-2-yl-2-methyl-2H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.29)

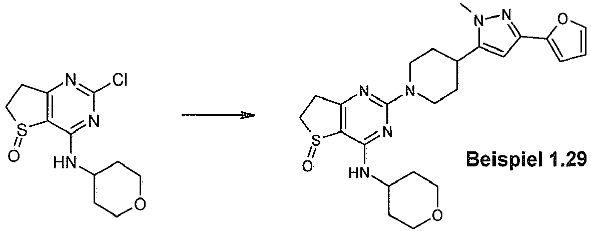

Ausgehend von **(IV-6)** (siehe 6.2) und **(V-5)** kann **Beispiel 1.29** analog zu Beispiel 1.14 (siehe 14.) hergestellt und aufgereinigt werden. Analytische HPLC-MS (Methode B): RT = 1,26 min.

### 30. SYNTHESE VON: {2-[4-(1-METHYL-1H-IMIDAZO[4,5-c]PYRIDIN-2-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 1.30)

### 30.1 Methyl-(3-nitropyridin-4-yl)-amin:

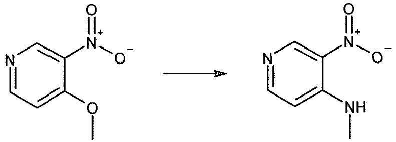

2,36 g 4-Methoxy-3-nitro-pyridin und 2,33 ml Methylamin (40%ig in Wasser) werden in 25 ml Ethanol 3 h unter Rückfluss gekocht. Anschließend wird das Reaktionsgemisch zur Trockne eingedampft. 2,3 g Produkt werden als Feststoff erhalten.

### 30.2 N⁴-Methylpyridin-3,4-diamin:

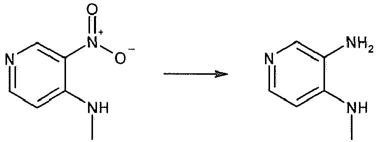

2,3 g Methyl-(3-nitropyridin-4-yl)-amin werden in 50 ml Methanol für und mit 0,8 g Raney-Nickel 2,5 h bei 50°C und 50 psi Wasserstoffdruck hydriert. Der Katalysator wird abfiltriert und das Filtrat zur Trockne eingedampft. Das Produkt wird chromatographisch (Alox, Dichlormethan/Methanol von 99/1 bis 19/1) gereinigt. 1,55 g Produkt werden als Feststoff erhalten. Smp: 163-165°C.

### 30.3 1-Methyl-2-piperidin-4-yl-1H-imidazo[4,5-c]pyridin (V-6):

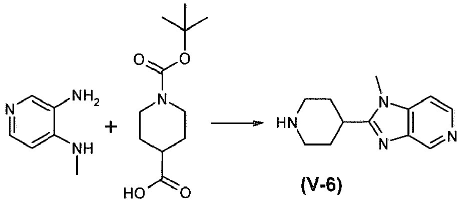

450 mg *N*⁴-Methylpyridin-3,4-diamin und 838 mg Piperidin-1,4-Dicarbonsäure-mono-*tert-*butylester werden in 8,6 g Polyphosphorsäure für 4 h bei 200°C erhitzt. Nach dem Abkühlen wird mit 4 N NaOH basisch gestellt und mit Trifluoressigsäure angesäuert. Das Gemisch wird über präparative HPLC (Methode C) aufgereinigt. 3,37 g (50%ig) **(V-6)** werden als Trifluoracetat erhalten. Analytische HPLC-MS (Methode B): RT = 0,30 min.

### 30.4 {2-[4-(1-Methyl-1H-imidazo[4,5-c]pyridin-2-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.30)

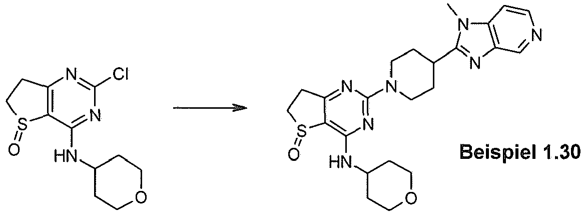

Ausgehend von **(IV-6)** (siehe 6.2) und **(V-6)** kann **Beispiel 1.30** analog zu Beispiel 1.14 (siehe 14) hergestellt und aufgereinigt werden. Analytische HPLC-MS (Methode D): RT = 0,86 min.

### 31. SYNTHESE VON 2-METHOXY-N-{1-[5-OXO-4-(TETRAHYDROPYRAN-4-YLAMINO)-6,7-DIHYDRO-5H-5λ⁴-THIENO[3,2-d]PYRIMIDIN-2-YL]-4-PHENYLPIPERIDIN-4-YLMETHYL}-ACETAMID (BEISPIEL 1.31)

### 31.1 4-[(2-Methoxyacetylamino)-methyl]-4-phenylpiperidin-1-carbonsäure-tert-butylester:

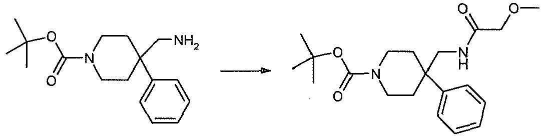

3,7 g des käuflichen 4-Aminomethyl-4-phenyl-piperidin-1-carbonsäure-*tert*-butylester und 3 ml Diisopropylethylamin werden in 30 ml Dichlormethan vorgelegt. Anschließend werden 2,25 ml Methoxyacetylchlorid langsam zugegeben. Das Reaktionsgemisch wird bei Raumtemperatur, bis keine weitere Umsetzung erfolgt, gerührt, dann mit Wasser versetzt. Die organische Phase wird zur Trockne eingedampft. 4,7 g Produkt werden als Öl erhalten.

### 31.2 2-Methoxy-N-(4-phenylpiperidin-4-ylmethyl)-acetamid (V-7):

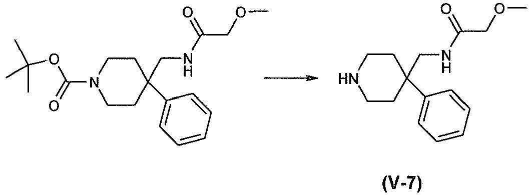

1 g 4-[(2-Methoxyacetylamino)-methyl]-4-phenylpiperidin-1-carbonsäure-*tert*-butylester werden in 4 ml Dichlormethan vorgelegt. Anschließend werden 1,7 ml Trifluoressigsäure zugegeben und über Nacht bei Raumtemperatur gerührt. Das Reaktionsgemisch wird mit Kaliumcarbonat basisch gestellt und die organische Phase zur Trockne eingedampft. 610 mg **(V-7)** werden als Öl erhalten.

### 31.3 Synthese von 2-Methoxy-N-{1-[5-oxo-4-(tetrahydropyran-4-ylamino)-6,7-dihydro-5H-5λ⁴-thieno[3,2-d]pyrimidin-2-yl]-4-phenylpiperidin-4-ylmethyl}-acetamid (Beispiel

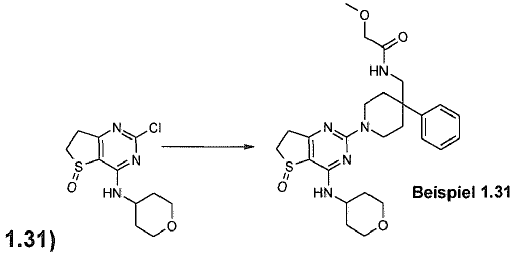

Ausgehend von **(IV-6)** (siehe 6.2) und **(V-7)** kann **Beispiel 1.31** analog zu Beispiel 1.15 (siehe 15.) hergestellt und aufgereinigt werden. Analytische HPLC-MS (Methode B): RT = 1,21 min.

### 32. SYNTHESE VON: N-CYCLOPROPYL-N-METHYL-4-{1-[5-OXO-4-(TETRAHYDRO PYRAN-4-YLAMINO)-6,7-DIHYDRO-5H-5λ⁴-THIENO[3,2-d]PYRIMIDIN-2-YL]-PIPERIDIN-4-YL}-BENZAMID (BEISPIEL 1.32)

### 32.1 4-[4-(Cyclopropylmethylcarbamoyl)-phenyl]-piperidin-1-carbonsäure-tert-butylester:

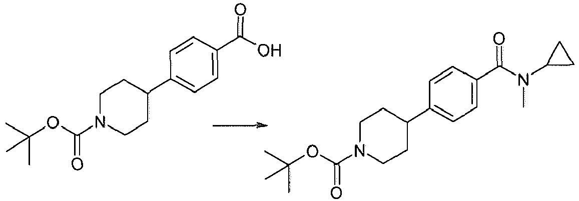

500 mg 4-(4-Carboxyphenyl)-piperidin-1-carbonsäure-*tert*-butylester werden in 28 ml Dimethylformamid vorgelegt, dann 1,14 ml Diisopropylethylamin und 747 mg HATU zugegeben. Das Reaktionsgemisch wird 15 min bei Raumtemperatur gerührt, dann werden 194 mg Cyclopropylmethylamin Hydrochlorid zugegeben. Das Reaktionsgemisch wird über Nacht bei Raumtemperatur gerührt. Anschließend wird das Produkt über präparative HPLC (Methode A) gereinigt. 480 mg Produkt werden als Öl erhalten. Analytische HPLC-MS (Methode B): RT = 1,64 min.

### 32.2 N-Cyclopropyl-N-methyl-4-piperidin-4-yl-benzamid (V-8):

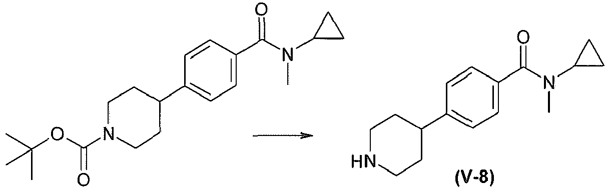

480 mg 4-[4-(Cyclopropylmethylcarbamoyl)-phenyl]-piperidin-1-carbonsäure-*tert*-butylester werden in 7,8 ml Dichlormethan vorgelegt und mit 1,09 ml Trifluoressigsäure versetzt. Das Reaktionsgemisch wird 1,5 h bei Raumtemperatur gerührt und anschließend zur Trockne eingedampft. Der Rückstand wird mit Toluol versetzt und wieder zur Trockne eingedampft. 444 mg **(V-8)** werden als Trifluoracetat erhalten. Analytische HPLC-MS (Methode B): RT = 1,11 min.

### 32.3 N-Cyclopropyl-N-methyl-4-{1-[5-oxo-4-(tetrahydropyran-4-ylamino)-6,7-dihydro-5H-5λ⁴-thieno[3,2-d]pyrimidin-2-yl]-piperidin-4-yl}-benzamid (Beispiel 1.32)

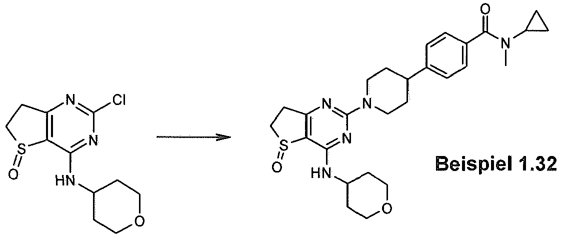

Ausgehend von **(IV-6)** (siehe 6.2) und **(V-8)** kann **Beispiel 1.32** analog zu Beispiel 1.14 (siehe 14.) hergestellt und aufgereinigt werden. Analytische HPLC-MS (Methode D): RT = 1,05 min.

### 33. SYNTHESE VON: N-CYCLOPROPYL-N-METHYL-4-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-BENZAMID (BEISPIEL 1.33)

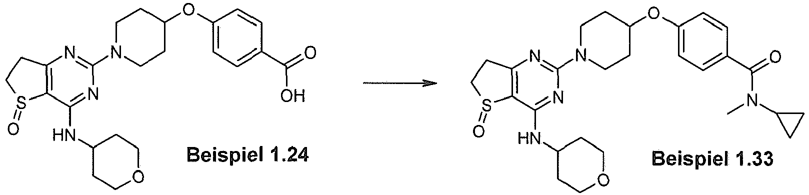

55 mg von **Beispiel 1.24** (siehe 24.2) werden in 2 ml Dimethylformamid vorgelegt, dann 81 µl Diisopropylethylamin und 53,1 mg O-(7-Azabenzotriazol-1-yl-)-N,N,N',N'-tetramethyluroniumhexafluorphosphat (HATU) zugegeben. Nach 15 min werden 13,8 mg cyclopropylmethylamin Hydrochlorid zugegeben. Das Reaktionsgemisch wird bei Raumtemperatur, bis keine weitere Reaktion erfolgt, gerührt und das Produkt direkt über präparative HPLC (Methode B) gereinigt. 30 mg **Beispiel 1.33** werden als Feststoff erhalten. Analytische HPLC-MS (Methode D): RT = 1,03 min.

### 34. SYNTHESE VON: {5-OXO-2-[4-(PYRIDIN-4-YLOXY)-PIPERIDIN-1-YL]-6,7-DIHYDRO-5H-5λ⁴-THIENO[3,2-d]PYRIMIDIN-4-YL}-(TETRAHYDROPYRAN-4-YL)-AMIN (BEISPIEL 1.34)

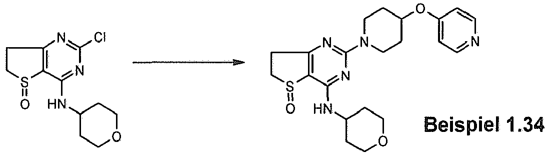

Ausgehend von **(IV-6)** (siehe 6.2) und 4-(Piperidin-4-yloxy)-pyridin kann **Beispiel 1.34** analog zu Beispiel 1.15 (siehe 15.) hergestellt und aufgereinigt werden. Analytische HPLC-MS (Methode B): RT = 0,99 min.

### 35. SYNTHESE VON: {2-[4-(4-CHLORPHENOXY)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.35)

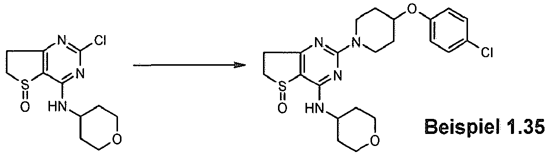

Ausgehend von **(IV-6)** (siehe 6.2) und 4-(4-Chlorphenoxy)-piperidin kann **Beispiel 1.35** analog zu Beispiel 1.15 (siehe 15.) hergestellt und aufgereinigt werden. Analytische HPLC-MS (Methode B): RT = 1,39 min.

### 36. SYNTHESE VON: (S)-1-METHYL-5-{2-[4-(5-METHYL-4-PHENYL-OXAZOL-2-YL)-PIPERIDIN-1-YL]-5-OXO-6,7-DIHYDRO-5H-5λ⁴-THIENO[3,2-d]PYRIMIDIN-4-YLAMINO}-PIPERIDIN-2-ON (BEISPIEL 1.36)

### 36.1 4-(5-Methyl-4-phenyloxazol-2-yl)-piperidin (V-9):

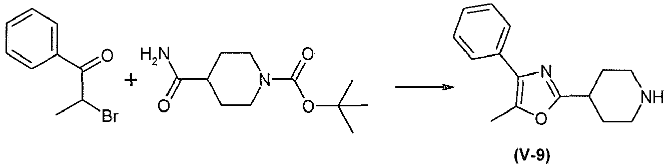

1,75 g 2-Brom-1-phenylpropan-1-on und 1,87 g 4-Carbamoylpiperidin-1-carbonsäure-*tert-*butylester werden in 0,5 ml NMP vorgelegt. Das Reaktionsgemisch wird bei 160°C für 20 min in der Mikrowelle und 35 min im Ölbad erhitzt, dann nach dem Abkühlen in Methanol aufgenommen und zur Trockne eingedampft. Der Rückstand wird mit Wasser versetzt, im Ultraschallbad behandelt und das unlösliche Öl abgesaugt. Die Mutterlauge wird über präparative HPLC (Methode C) gereinigt. 160 mg **(V-9)** werden als Trifluoracetat erhalten. Analytische HPLC-MS (Methode B): RT = 1,24 min.

### 36.2 (S)-1-Methyl-5-{2-[4-(5-methyl-4-phenyloxazol-2-yl)-piperidin-1-yl]-5-oxo-6,7-dihydro-5H-5λ⁴-thieno[3,2-d]pyrimidin-4-ylamino}-piperidin-2-on (Beispiel 1.36)

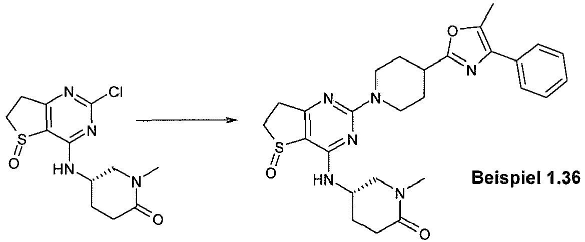

Ausgehend von **(IV-5)** (siehe 5.5) und **(V-9)** kann **Beispiel 1.36** analog zu Beispiel 1.14 (siehe 14.) hergestellt und aufgereinigt werden. Analytische HPLC-MS (Methode D): RT = 1,08 min.

### 37 SYNTHESE VON: (1-{2-[4-(5-METHYL-4-PHENYLOXAZOL-2-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.37)

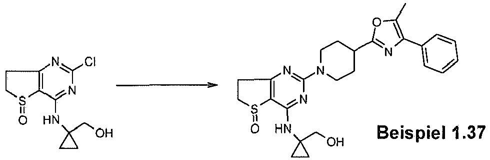

Ausgehend von **(IV-2)** (siehe 2.4) und **(V-9)** (siehe 36.1) kann **Beispiel 1.37** analog zu Beispiel 1.14 (siehe 14.) hergestellt und aufgereinigt werden. Analytische HPLC-MS (Methode B): RT = 1,33 min.

### 38. SYNTHESE VON: (S)-5-{2-[4-(4,5-DIPHENYLOXAZOL-2-YL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.38)

### 38.1 4-(4,5-Diphenyloxazol-2-yl)-piperidin-1-carbonsäure-tert-butylester

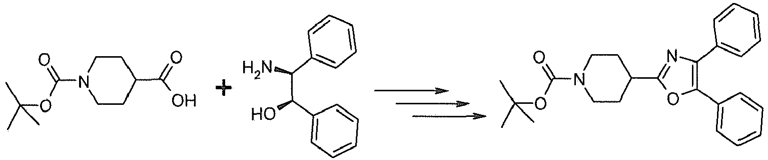

Ausgehend von 1,08 g Piperidin-1,4-dicarbonsäure-mono-*tert*-butylester und 1 g 2-Amino-1,2-diphenyl-ethanol kann das Produkt wie in der Literatur beschrieben (siehe Tet. 2001, 4867) hergestellt werden. Das Produkt wird chromatographisch (Methode B) gereinigt. 560 mg werden als Öl erhalten. Analytische HPLC-MS (Methode A): RT = 1,72 min.

### 38.2 4-(4,5-Diphenyloxazol-2-yl)-piperidin (V-10)

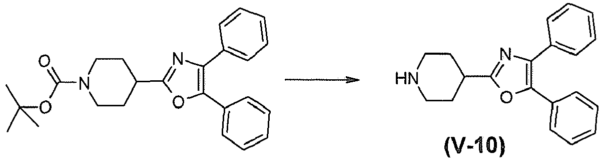

560 mg 4-(4,5-Diphenyloxazol-2-yl)-piperidin-1-carbonsäure-*tert*-butylester werden in 2 ml Dichlormethan vorgelegt, dann 1,1 ml Trifluoressigsäure zugegeben. Das Reaktionsgemisch wird 15 Stunden bei Raumtemperatur gerührt, dann zur Trockne eingedampft. Der Rückstand wird mit Toluol versetzt und wieder zur Trockne eingedampft. Der Rückstand wird mit Diethylether versetzt und der ausgefallene Feststoff abgesaugt und getrocknet. 510 mg **(V-10)** werden erhalten. Analytische HPLC-MS (Methode B): RT = 1,38 min.

### 38.3 (S)-5-{2-[4-(4,5-Diphenyloxazol-2-yl)-piperidin-1-yl]-5-oxo-6,7-dihydro-5H-5λ4-thieno[3,2-d]pyrimidin-4-ylamino}-1-methylpiperidin-2-on (Beispiel 1.38)

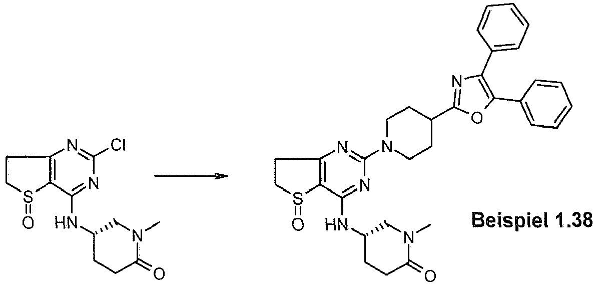

Ausgehend von **(IV-5)** (siehe 5.5) und **(V-10)** kann **Beispiel 1.38** analog zu Beispiel 1.14 (siehe 14.) hergestellt und aufgereinigt werden. Analytische HPLC-MS (Methode B): RT = 1,40 min.

### 39. SYNTHESE VON: {4-(4-CHLORPHENYL)-1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YL}-METHANOL (BEISPIEL 1.39)

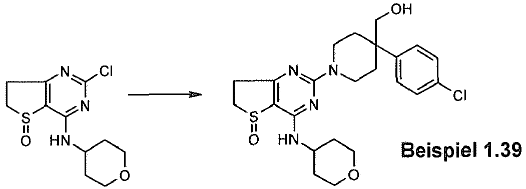

Ausgehend von **(IV-6)** (siehe 6.2) und [4-(4-Chlorphenyl)-piperidin-4-yl]-methanol (J. Med. Chem. 2004, 497) kann Beispiel 1.39 analog zu Beispiel 1.15 (siehe 15.) hergestellt und aufgereinigt werden. Analytische HPLC-MS (Methode B): RT = 1,24 min.

### 40. SYNTHESE VON: [1-(2-{4-[5-(4-CHLORPHENYL)-4-METHYLOXAZOL-2-YL]-PIPERIDIN-1-YL)-5-OXO-6,7-DIHYDRO-5H-5λ⁴-THIENO[3,2-d]PYRIMIDIN-4-YLAMINO)-CYCLOPROPYL]-METHANOL (BEISPIEL 1.40)

### 40.1 4-[5-(4-Chlorphenyl)-4-methyloxazol-2-yl]-piperidin (V-11):

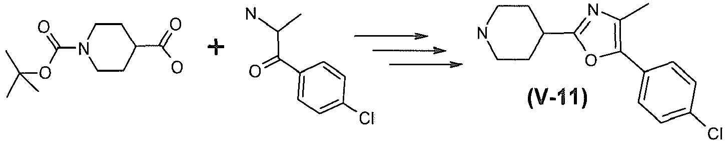

Ausgehend von Piperidin-1,4-dicarbonsäure-mono-tert-butylester und 2-Amino-1-(4-chlorphenyl)-propan-1-on (siehe J. Med. Chem. 1974, 416) kann **(V-11)** analog zu (V-10) (siehe 38.2) hergestellt werden. Analytische HPLC-MS (Methode B): RT = 1,30 min.

### 40.2 [1-(2-{4-[5-(4-Chlorphenyl)-4-methyloxazol-2-yl]-piperidin-1-yl}-5-oxo-6,7-dihydro-5H-5λ⁴-thieno(3,2-d]pyrimidin-4-ylamino)-cyclopropyl]-methanol (Beispiel 1.40)

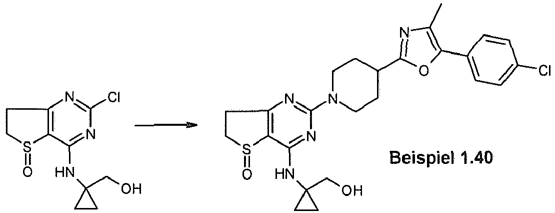

Ausgehend von **(IV-2)** (siehe 2.4) und **(V-11)** kann **Beispiel 1.40** analog zu Beispiel 1.14 (siehe 14.) hergestellt und aufgereinigt werden. Analytische HPLC-MS (Methode B): RT = 1,37 min.

### 41. SYNTHESE VON: 4-(4-CHLORPHENYL)-1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-OL (BEISPIEL 1.41)

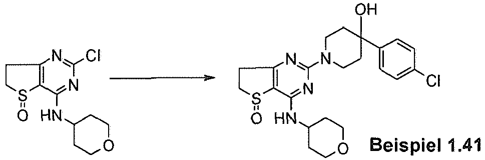

Ausgehend von **(IV-6)** (siehe 6.2) und 4-(4-Chlorphenyl)-piperidin-4-ol kann **Beispiel 1.41** analog zu Beispiel 1.15 (siehe 15.) hergestellt und aufgereinigt werden. Analytische HPLC-MS (Methode B): RT = 1,25 min.

### 42. SYNTHESE VON: {2-[4-(4-CHLORPHENYL)-4-METHOXYPIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.42)

### 42.1 4-(4-Chlorphenyl)-4-hydroxypiperidin-1-carbonsäure-tert-butylester:

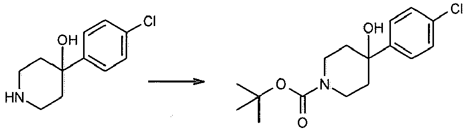

500 mg 4-(4-Chlorphenyl)-piperidin-4-ol werden in 6 ml Dioxan vorgelegt, dann 0,9 ml Wasser und 400 mg Natriumcarbonat zugegeben. Nach 5 min werden 530 mg Di-*tert*-butyldicarbonat zugegeben. Das Reaktionsgemisch wird 12 Stunden bei Raumtemperatur gerührt, anschließend mit Wasser versetzt und das Produkt mit Dichlormethan extrahiert.. 790 mg Produkt werden als Öl erhalten. Analytische HPLC-MS (Methode B): RT = 1,65 min.

### 42.2 4-(4-Chlorphenyl)-4-methoxypiperidin-1-carbonsäure-tert-butylester:

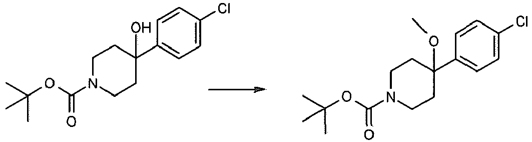

790 mg 4-(4-Chlorphenyl)-4-hydroxypiperidin-1-carbonsäure-*tert*-butylester werden in 5 ml Dimethylformamid vorgelegt und 193 mg Natriumhydrid (60% in Mineralöl) zugegeben. Das Reaktionsgemisch wird 30 min bei Raumtemperatur gerührt, dann 267 µl Methyliodid zugegeben. Nach 1 h wird das Reaktionsgemisch auf Eis gegossen und das Produkt mit Diethylether extrahiert. 650 mg Produkt werden als Öl erhalten. Analytische HPLC-MS (Methode B): RT = 1,88 min.

### 42.3 4-(4-Chlorphenyl)-4-methoxypiperidin (V-12):

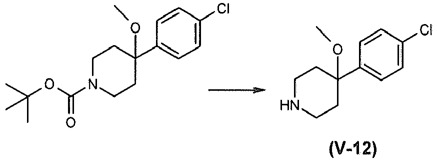

650 mg 4-(4-Chlorphenyl)-4-methoxypiperidin-1-carbonsäure-*tert*-butylester werden in 3 ml Dichlormethan vorgelegt, dann 1,46 ml Trifluoressigsäure zugegeben. Das Reaktionsgemisch wird über Nacht bei Raumtemperatur gerührt und zur Trockne eingedampft. Der Rückstand wird mit Toluol versetzt und wieder zur Trockne eingedampft. Der Rückstand wird mit Diethylether verrieben und der Feststoff abgesaugt. 450 mg (V-12) werden als Trifluoracetat erhalten. Analytische HPLC-MS (Methode B): RT = 1,22 min.42.4

### {2-[4-(4-Chlorphenyl)-4-methoxypiperidin-1-yl]-5-oxo-6,7-dihydro-5H-5λ⁴-thieno[3,2-d]pyrimidin-4-yl}-(tetrahydropyran-4-yl)-amin (Beispiel 1.42)

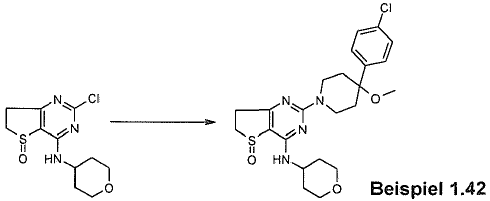

Ausgehend von **(IV-6)** (siehe 6.2) und **(V-12)** kann **Beispiel 1.42** analog zu Beispiel 1.15 (siehe 15.) hergestellt und aufgereinigt werden. Analytische HPLC-MS (Methode B): RT = 1,39 min.

### 43. SYNTHESE VON: 4-{1-[4-(1-HYDROXYMETHYLCYCLOPROPYLAMINO)-5-OXO-6,7-DIHYDRO-5H-5λ⁴-THIENO[3,2-d]PYRIMIDIN-2-YL]-PIPERIDIN-4-YLOXY}-BENZONITRIL (BEISPIEL 1.43)

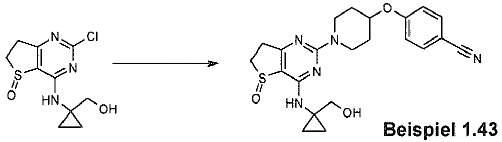

Ausgehend von **(IV-2)** (siehe 2.4) und 4-(Piperidin-4-yloxy)-benzonitril (WO2007/106705) kann **Beispiel 1.43** analog zu Beispiel 1.14 (siehe 14.) hergestellt und aufgereinigt werden. Analytische HPLC-MS (Methode B): RT = 1,24 min.

### 44. SYNTHESE VON: 5-OXO-2-[4-(4,5,6,7-TETRAHYDROBENZOXAZOL-2-YL)-PIPERIDIN-1-YL]-6,7-DIHYDRO-5H-5λ⁴-THIENO[3,2-d]PYRIMIDIN-4-YL}-(TETRAHYDRO PYRAN-4-YL)-AMIN (BEISPIEL 1.44)

### 44.1 2-(1-Benzylpiperidin-4-yl)-4,5,6,7-tetrahydrobenzoxazol:

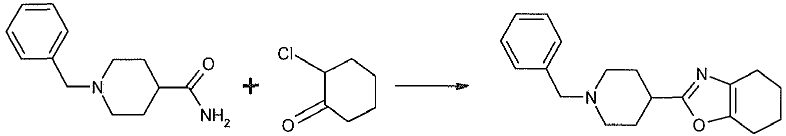

Ein Gemisch von 2,43 g 2-Chlorcyclohexanon und 1g 1-Benzylpiperidin-4-carbonsäureamid (WO2005/61483) wird bei 160°C in der Mikrowelle, bis keine weitere Umsetzung erfolg, erhitzt. Das Produkt wird chromatographisch gereinigt. 963 mg des Produkts werden erhalten. Analytische HPLC-MS (Methode B): RT = 1,28 min.

### 44.2 2-Piperidin-4-yl-4,5,6,7-tetrahydrobenzoxazol (V-13):

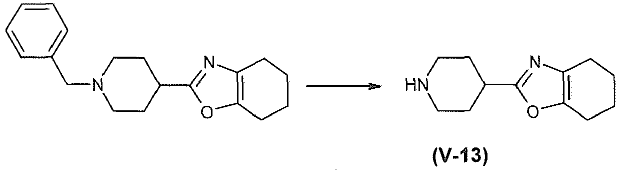

903 mg 2-(1-Benzyl-piperidin-4-yl)-4,5,6,7-tetrahydrobenzoxazol werden in 20 ml Methanol vorgelegt und mit 450 mg Pd/C 10% bei einem Druck von 3 bar und bei Raumtemperatur hydriert. Nach 12 Stunden wird der Katalysator abgesaugt und das Filtrat zur Trockne eingedampft. Das Produkt wird chromatographisch gereinigt. 469 mg **(V-13)** werden als Trifluoracetat erhalten. Analytische HPLC-MS (Methode B): RT = 1,09 min.

### 44.3 5-Oxo-2-[4-(4,5,6,7-tetrahydrobenzoxazol-2-yl)-piperidin-1-yl]-6,7-dihydro-5H-5λ⁴-thieno[3,2-d]pyrimidin-4-yl}-(tetrahydropyran-4-yl)-amin (Beispiel 1.44)

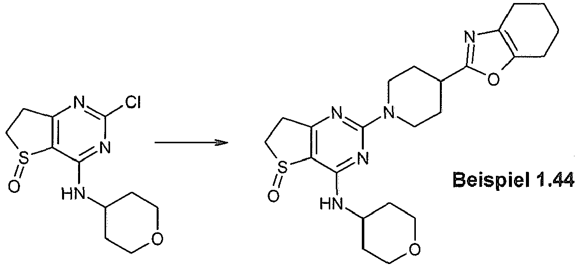

Ausgehend von **(IV-6)** (siehe 6.2) und **(V-13)** kann **Beispiel 1.44** analog zu Beispiel 1.14 (siehe 14.) hergestellt und aufgereinigt werden. Analytische HPLC-MS (Methode B): RT = 1,23 min.

### Synthese Schema 2

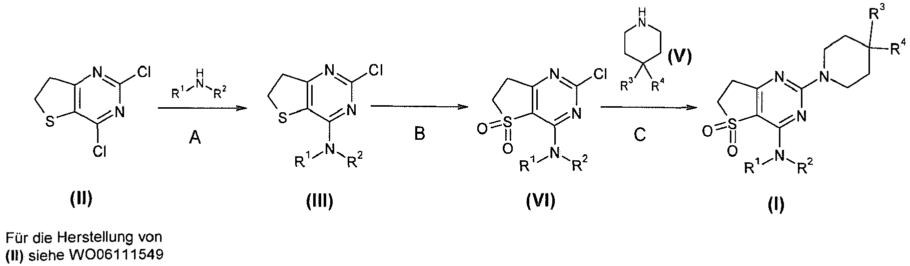

### 45. SYNTHESE VON: (S)-5-{2-[4-(4-CHLORPHENYL)-PIPERIDIN-1-YL]-5,5-DIOXO-6,7-DIHYDRO-5H-5λ⁶-THIENO[3,2-d]PYRIMIDIN-4-YLAMINO}-1-METHYLPIPERIDIN-2-ON (Beispiel 1.45)

### 45.1 5-(2-Chlor-5,5-dioxo-6,7-dihydro-5H-5)λ⁶-thieno[3,2-d]pyrimidin-4-ylamino)-1-methylpiperidin-2-on (VI-1):

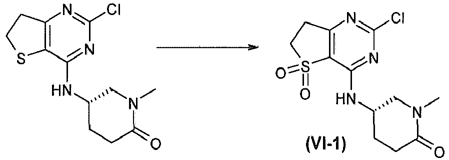

200 mg **(III-5)** (siehe 5.4) werden in 3 ml Trifluoressigsäure vorgelegt, dann 165 µl Wasserstoffperoxid (35%) langsam zugetropft. Eine exotherme Reaktion findet statt. Das Reaktionsgemisch wird 12 Stunden bei Raumtemperatur gerührt, dann mit Eiswasser versetzt und mit NH₄OH basisch gestellt. Das Produkt wird mit Dichlormethan extrahiert. 150 mg **(VI-1)** werden als Feststoff erhalten.

### 45.2 (S)-5-{2-[4-(4-Chlorphenyl)-piperidin-1-yl]-5,5-dioxo-6,7-dihydro-5H-5λ⁶-thieno[3,2-d]pyrimidin-4-ylamino}-1-methylpiperidin-2-on (Beispiel 1.45)

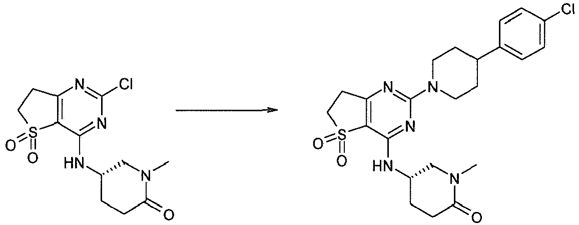

Ausgehend von **(VI-1)** und 4-(4-Chlorphenyl)-piperidin Hydrochlorid kann **Beispiel 1.45** analog zu Beispiel 1.14 (siehe 14.) hergestellt und aufgereinigt werden. Analytische HPLC-MS (Methode B): RT = 1,48 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in der Tabellen B, D und E angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith™ Flash RP-18e, 4.6 mm x 25 mm (Säulentemperatur: konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.80 |
| 0.30 | 95 | 5 | 2.80 |
| 1.60 | 2 | 98 | 2.80 |
| 1.90 | 2 | 98 | 2.80 |
| 2.00 | 95 | 5 | 2.50 |

Als stationäre Phase dient eine Säule Merck Chromolith™ Flash RP-18e, 3 mm x 100 mm (Säulentemperatur: konstant bei 25°C).

### Analytische HPLC-MS, Methode C

Waters ZQ2000 Massenspektrometer (positive Ionisation (ESI+)), HP1100 HPLC (DAD, Wellenlängenbereich: 210 to 500 nm), and Gilson 215 Autosampler.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC-MS, Methode D

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur.

### Analytische HPLC-MS, Methode E

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.20 |
| 0.30 | 95 | 5 | 1.20 |
| 9.00 | 2 | 98 | 1.20 |
| 9.40 | 2 | 98 | 1.20 |
| 9.50 | 95 | 5 | 2.80 |
| 9.90 | 95 | 5 | 2.80 |
| 10.00 | 95 | 5 | 0.20 |

Als stationäre Phase dient eine Säule Merck Chromolith™ Flash RP-18e, 4.6 mm x 25 mm (Säulentemperatur: konstant bei 25°C).

### Analytische HPLC, Methode A

Agilent 1100 (Diodenarraydetektion, Wellenlängenbereich: 210-380 nm).
A: Wasser mit 0.10% TFA
B: Acetonitril mit 0.13% TFA

| | | | |
|---|---|---|---|
| Zeit in min | %A | %B | Flussrate in ml/min |
| 0.00 | 95 | 5 | 1.50 |
| 0.60 | 95 | 5 | 1.50 |
| 3.40 | 2 | 98 | 1.50 |
| 3.90 | 2 | 98 | 1.50 |
| 4.20 | 95 | 5 | 1.50 |
| 4.90 | 95 | 5 | 1.50 |

Als stationäre Phase dient eine Säule Varian Microsorb, RP C18, 3 µm, 100 A, Raumtemperatur.

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur.

### Präparative HPLC, Methode B

Gilson HPLC mit Gilson UV-VIS-155 Detektor, Sampling Injektor 231 XL.

Als Wellenlänge wird das substanzspezifische UV-Maximum angegeben.
A: Wasser mit 0.1 % Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode C

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.50 | 95 | 5 | 180 |

Als stationäre Phase dient eine Säule Microsorb RP 18, 8 µm, 50 X 150 mm, Raumtemperatur.

### Präparative HPLC, Methode D

Gilson HPLC mit Gilson UV-VIS-155 Detektor, Sampling Injektor 231 XL.

Als Wellenlänge wird das substanzspezifische UV-Maximum angegeben.
A: Wasser mit 0.1 % Ammoniak 35%ig
B: Acetonitril

| Zeit in min | %A | %B | Flussrate in ml/min |
|---|---|---|---|
| 0.00 | 95 | 5 | 180 |
| 1.10 | 95 | 5 | 180 |
| 9.00 | 2 | 98 | 180 |
| 10.00 | 2 | 98 | 180 |
| 10.50 | 95 | 5 | 180 |
| 12.00 | 95 | 5 | 180 |

Als stationäre Phase dient eine Säule X-Bridge C18, 5 µm, 50 X 65 mm, Raumtemperatur.

### INDIKATIONSGEBIETE

Wie gefunden wurde, zeichnen sich die erfindungsgemäßen Kombinationen enthaltend eine Verbindung der Formel 1 und mindestens ein NSAID durch vielfältige Anwendungsmöglichkeiten auf therapeutischem Gebiet aus. Hervorzuheben sind solche Anwendungsmöglichkeiten, für welche die erfindungsgemäßen Kombinationen aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung der erfindungsgemäßen Kombinationen zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der erfindungsgemäßen Kombinationen zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der erfindungsgemäßen Kombinationen zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose, Amyotrophe Lateralsklerose (ALS) oder akute und chronische Schmerzzustände sowie Verletzungen des Gehirns hervorgerufen durch Schlaganfall, Hypoxie oder Schädel-Hirn-Trauma.

Ein herausragender Aspekt der erfindungsgemäßen Formulierungen enhaltend eine Kombination einer Verbindung der Formel **1** und mindestens ein NSAID ist deren reduziertes Profil an Nebenwirkungen im Vergleich zu Formulierungen, die die gleiche Verbindung der Formel **1** in gleicher Menge in Abwesenheit eines NSAIDs enthalten. Nebenwirkungen, die bei der Verabreichung eines PDE4-Inhibitors häufig auftreten, sind unter anderen vorzugsweise Diarrhoe, Übelkeit und Erbrechen. Im Rattenmodell konnten noch weitere Nebenwirkungen nach PDE4-Inhibitor-Verabreichung beobachtet werden wie beispielsweise Körpergewichtsverlust, Leukozytose und Neutrophilie, aber auch Diarrhoe.

Unter einem reduzierten Profil an Nebenwirkungen wird im Rahmen der Erfindung insbesondere verstanden, eine therapeutisch wirksame Dosis eines PDE4-Inhibitors in einer der erfindungsgemäßen pharmazeutischen Zusammensetzung verabrelchen zu können, ohne beim Patienten die oder zumindest eine der häufig bei der Verabreichung von PDE4-Inhibitoren beobachteten Nebenwirkungen in nennenswertem Ausmaß auszulösen. Besonders bevorzugt ist die Verabreichung einer therapeutisch wirksamen Substanzmenge eines PDE4-Inhibitors in der erfindungsgemäßen pharmazeutischen Zusammensetzung in jedem Stadium des Krankheitsverlaufs, ohne die typischen PDE4-Inhibitor-vermittelten Nebenwirkungen der Diarrhoe, des Körpergewichtsverlustes, der Leukozytose oder der Neutrophilie auszulösen. Ein besonderer Gegenstand der vorliegenden Erfindung betrifft die Verabreichung einer therapeutisch wirksamen Substanzmenge der erfindungsgemäßen pharmazeutischen Zusammensetzung in Jedem Stadium des Krankheitsverlaufs, ohne die typische PDE4-Inhibitor-vormittelte Nebenwirkung der Diarrhoe in nennenswertem Ausmaß auszulösen.

Im folgenden konnte experimentell am Rattenmodell gezeigt werden, dass die erfindungsgemäßen pharmazeutischen Zusammensetzungen enthaltend einen PDE4-Inhibitor und mindestens ein NSAID viele der Nebenwirkungen, die bei der Verabreichung des entsprechenden PDE4-Inhibitors alleine auftreten, erheblich reduzieren oder sogar völlig vermeiden.

### EXPERIMENTELLE DURCHFÜHRUNG:

### Versuch 1: Diclofenac schützt vor Roflumilast-vermittelten Effekten wie Körpergewichtsvertust, Leukozytose, und Neutrophilie: (Vergleichsbeispiele)

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 10 mg/kg Roflumilast (PDE4-Inhibitor) um 13 Uhr.
Gruppe 4 ("Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 0.5% Natrosol (Placebo) um 13 Uhr.

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung. Das gleiche traf für eine Ratte aus der Roflumilast-Gruppe zu, die zwischen Versuchstag 4 und 5 verstarb.

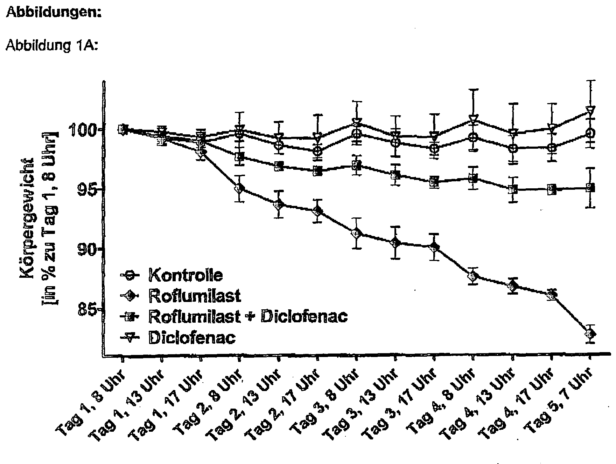

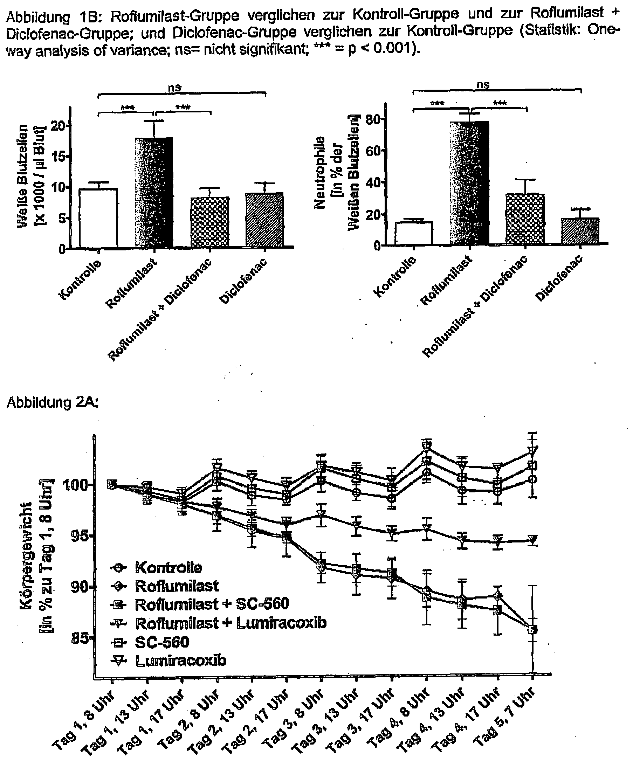

In Abbildung 1A werden die Körpergewichte der Ratten aus den verschiedenen Gruppen als Veränderung in % 1, Applikationszeitpunkt (= Tag 1, 8 Uhr (=Zeitpunkt t₀)) dargestellt. Der Mittelwert ± Standardabweichung der Körpergewichte zum Zeitpunkt t₀ betrug 355 ± 17 g, Am Versuchsende (95 Stunden nach t₀ ( = dem Zeitpunkt der ersten Applikation am Tag 1, 8 Uhr)) wurden aus dem Blut von 4 bzw. 5 der Ratten aus den einzelnen Gruppen der Anteil von Weißen Blutzellen (x 1000 Zellen/µl Blut, Abbildung 1B, linke Abbildung) und der Anteil der Neutrophilen (in % der Weißen Blutzellen, Abbildung 1B, rechte Abbildung) bestimmt

### Versuch 2: Diclofenac schützt vor Roflumilast-vermittelten Effekten wie Diarrhoe (Vergleichsbeispiele)

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roffumilast + Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 10 mg/kg Roflumilast (PDE4-Inhibitor) um 13 Uhr.
Gruppe 4 ("Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 0.5% Natrosol (Placebo) um 13 Uhr.

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung. Das gleiche traf für eine Ratte aus der Roflumilast-Gruppe zu, die zwischen Versuchstag 4 und 5 verstarb.

Am Versuchsende (95 Stunden nach t₀ ( = dem Zeitpunkt der ersten Applikation am Tag 1, 8 Uhr)) wurden die Ratten aus den einzelnen Gruppen auf das Auftreten multifokaler perivaskulärer mononukleärer Infiltration (=Entzündungsparameter) im Mesenterium und auf das Auftreten der Proliferation von Fibroblasten im Mesenterium hin phänotypisch und histopathologisch untersucht. Weiterhin wurde das Auftreten von Diarrhoe bei den Ratten der verschiedenen Gruppen festgehalten. Die Befunde sind in Tabelle 1 wie folgt zusammengefasst:

**Tabelle 1: Phänotypische und histopathologische Befunde**

| Parameter | Kontrolle (Gruppe 1) | Roflumilast (Gruppe 2) | Roflumilast + Diclofenac (Gruppe 3) | Diclofenac (Gruppe 4) |
|---|---|---|---|---|
| Diarrhoe | 0/6 (= 0 von 6 Tieren) | 5/6 | 0/6 | 0/6 |
| Mesenterium: | | | | |
| multifokale perivaskuläre mononukleäre Infiltration (= Entzündungsparameter) | 0/5 | 4/4 | 0/5 | 0/5 |
| Mesenterium: | 0/5 | 4/4 | 0/5 | 0/5 |
| Proliferation von Fibroblasten | | | | |

Zusammenfassend lässt sich sagen, dass die in der Roflumilast-Gruppe beobachteteten PDE4-Inhibitor-vermittelten Nebenwirkungen wie Körpergewichtsverlust (Abb. 1A), Leukozytose (Abb. 1B, links), Neutrophille (Abb. 1B, rechts) und Diarrhoe (einschließlich des Autretens von Entzündungsparametern und der Fibroblasten-Proliferation im Mesenterium) erheblich reduziert oder verhindert werden können (häufig sogar Reduktion auf den Wert der Kontroll-Gruppe), indem man gleichzeitig bzw. nur um einige Stunden versetzt, ein NSAID wie Diclofenac koappliziert (siehe Roflumilast + Diclofenac-Gruppe). Die, gemessenen Parameter nach Applikation von Diclofenac alleine zeigten sich sehr ähnlich zu den Kontrollgruppen.

### Versuch 3: Der COX-2 selektive Inhibitor Lumiracoxib, aber nicht der COX-1 selektive Inhibitor SC-560, schützt vor Roflumilast-vermittelten Effekten wie Körpergewichtsverlust, Leukozytose, und Neutrophilie: (Vergleichsbeispiele)

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + SC-560-Gruppe"): Sechs männliche Wistar-Ratten erhielten eine tägliche Applikation von 2 mg/kg SC-560 (NSAID, selektiv für COX-1) zu den Zeitpunkten 8 Uhr und 17 Uhr und von 10 mg/kg Roflumilast (PDE4-Inhibitor) um 13 Uhr,
Gruppe 4 ("Roflumilast + Lumiracoxib-Gruppe"): Sechs männliche Wistar-Ratten erhielten eine tägliche Applikation von 2 mg/kg Lumiracoxib (NSAID, selektiv für COX-2) zu den Zeitpunkten 8 Uhr und 17 Uhr und von 10 mg/kg Roflumilast (PDE4-Inhibitor) um 13 Uhr.
Gruppe 5 ("SC-560-Gruppe"): Sechs männliche Wistar-Ratten erhielten eine tägliche Applikation von 2 mg/kg SC-560 (NSAID, selektiv für Cox-1) zu den Zeitpunkten 8 Uhr und 17 Uhr und von 0.5% Natrosol um 13 Uhr.
Gruppe 6 ("Lumiracoxib-Gruppe"); Sechs männliche Wistar-Ratten erhielten eine tägliche Applikation von 2 mg/kg Lumiracoxib (NSAID, selektiv für Cox-2) zu den Zeitpunkten 8 Uhr und 17 Uhr und von 0,5% Natrosol um 13 Uhr,

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.

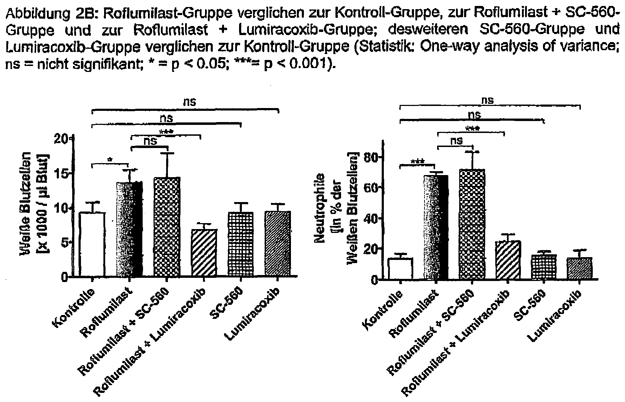

In Abbildung 2A werden die Körpergewichte der Ratten aus den verschiedenen Gruppen als Veränderung in % 1. Applikationszeitpunkt (= Tag 1, 8 Uhr (= Zeitpunkt t₀)) dargestellt. Der Mittelwert ± Standardabweichung der Körpergewichte zum Zeitpunkt t₀ betrug 306 ± 11 g. Am Versuchsende (95 Stunden nach t₀ ( = dem Zeitpunkt der ersten Applikation am Tag 1, 8 Uhr)) wurden aus dem Blut von 5 der Ratten aus den einzelnen Gruppen der Anteil von Weißen Blutzellen (x 1000 Zellen/ µl Blut, Abbildung 2B, linke Abbildung) und der Anteil der Neutrophilen (in % der Weißen Blutzelien, Abbildung 2B, rechte Abbildung) bestimmt

### Versuch 4: Der COX-2 selektive Inhibitor Lumiracoxib, aber nicht der COX-1 selektive Inhibitor SC-560, schützt vor Roflumilast-vermittelten Effekten wie Diarrhoe (Vergleichsbeispiele)

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + SC-560-Gruppe"): Sechs männliche Wistar-Ratten erhielten eine tägliche Applikation von 2 mg/kg SC-560 (NSAID, selektiv für COX-1) zu den Zeitpunkten 8 Uhr und 17 Uhr und von 10 mg/kg Roflumilast(PDE4-Inhibitor) um 13 Uhr.
Gruppe 4 ("Roflumilast + Lumiracoxib-Gruppe"): Sechs männliche Wistar-Ratten erhielten eine tägliche Applikation von 2 mg/kg Lumiracoxib (NSAID, selektiv für COX-2) zu den Zeitpunkten 8 Uhr und 17 Uhr und von 10 mg/kg Roflumilast (PDE4-Inhibitor) um 13 Uhr.
Gruppe 5 ("SC-560-Gruppe"): Sechs männliche Wistar-Ratten erhielten eine tägliche Applikation von 2 mg/kg SC-560 (NSAID, selektiv für Cox-1) zu den Zeitpunkten 8 Uhr und 17 Uhr und von 0.5% Natrosol um 13 Uhr.
Gruppe 6 ("Lumiracoxib-Gruppe"): Sechs männliche Wistar-Ratten erhielten eine tägliche Applikation von 2 mg/kg Lumiracoxib (NSAID, selektiv für Cox-2) zu den Zeitpunkten 8 Uhr und 17 Uhr und von 0.5% Natrosol um 13 Uhr.

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.

Am Versuchsende (95 Stunden nach t₀ ( = dem Zeitpunkt der ersten Applikation am Tag 1, 8 Uhr)) wurden die Ratten aus den einzelnen Gruppen auf das Auftreten multifokaler perivaskulärer mononukfeärer Infiltration (=Entzündungsparameter) im Mesenterium und auf das Auftreten der Proliferation von Fibroblasten im Mesenterium hin phänotypisch und histopathologisch untersucht. Weiterhin wurde das Auftreten von Diarrhoe bei den Ratten der verschiedenen Gruppen festgehalten. Die Befunde sind in Tabetfe 2 wie folgt zusammengefasst:

**Tabelle 2: Phänotypische und histopathotogische Befunde**

| Parameter | Kontrolle | Roflumilast | Roflumilast + SC-560 | Roflumilast+ Lumiracoxib | SC-560 | Lumiracoxib |
|---|---|---|---|---|---|---|
| | (Gruppe 1) | (Gruppe 2) | (Gruppe 3) | (Gruppe 4) | (Gruppe 5) | (Gruppe 6) |
| Diarrhoe | 0/6 (= 0 von 6 Tieren) | 2/6 | 0/6 | 0/6 | 0/6 | 0/6 |
| Mesenterium: | | | | | | |
| multifokale perivaskuläre mononukleäre Infiltration (= Entzündungsparameter) | 0/5 | 5/5 | 4/5 | 0/5 | 0/5 | 0/5 |
| Mesenterium: | 0/5 | 5/5 | 4/5 | 0/5 | 0/5 | 0/5 |
| Proliferation von Fibroblasten | | | | | | |

Zusammenfassend lässt sich sagen, dass die in der Roflumilast-Gruppe beobachteteten PDE4-Inhibitor-vermittelten Nebenwirkungen wie Körpergewichtsverlust (Abb. 2A), Leukozytose (Abb. 2B, links), Neutrophilie (Abb. 2B, rechts) und Diarrhoe (einschließlich des Auftretens von Entzündungsparametem und der Fibroblasten-Proliferation im Mesenterium) erheblich reduziert oder verhindert werden können (häufig sogar Reduktion auf den Wert der Kontroll-Gruppe), indem man gleichzeitig bzw. nur um einige Stunden versetzt, ein COX-2 selektives NSAID wie Lumiracoxib koappliziert (siehe Roflumilast + Lumiracoxib). Der COX-1 selektive NSAID SC-560 hat keinerlei protektive Wirkung auf Körpergewichtsverlust, Leukozytose, und Neutrophilie und nur eine sehr geringe protektive Wirkung auf die histopathologischen Befunde (multifokale perivaskuläre mononukleäre Infiltration oder Proliferation von Fibroblasten im Mesenterium). Eine Aussage bezüglich der Wirkung von SC-560 auf Diarrhoe wird dadurch erschwert, dass in diesem Versuch in der Roflumilast-Gruppe an für sich nur zwei Tiere Diarrhoe zeigten. Die gemessenen Parameter nach Applikation von SC-560 oder von Lumiracoxib alleine zeigten sich sehr ähnlich zu den Kontrollgruppen.

Insgesamt lässt sich schlussfolgern, dass die protektive Wirkung eines NSAIDs auf die PDE4-Inhibitor-vermlttelten Nebenwirkungen auf der Hemmung von COX-2 beruhen.

### DARREICHUNGSFORMEN

Die Applikation der erfindungsgemäßen Wirkstoffkombinationen aus 1 und **2** erfolgt bevorzugt auf oralem Wege. Hierzu müssen die Bestandteile (**1**) und (**2**) in geeigneten oralen Darreichungsformen bereitgestellt werden.

Geeignete orale Darreichungsformen sind beispielsweise Tabletten, Kapseln, Lösungen, Säfte, Emulsionen. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann In Form einer Tablette, als Pulver, als Pulver in einer Kapsel (z.B. Hartgelatinekapsel), als Lösung oder Suspension erfolgen.

Besonders bevorzugt ist es, wenn die Verabreichung ein oder zweimal täglich erfolgt Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat, mikrokristalliner Cellulose, Sorbitol, Mannitol, Isomaltose oder Milchzucker, Sprengmitteln, wie Maisstärke, quervernetztes Polyvinylpyrrolidon, quervemetzte Natrium Carboxymethylcellulose, Natriumstärkeglykolat oder Alginsäure, Bindemitteln, wie Stärke, Hydroxypropylmethylcellulose, Polyvinylpyrrolidon oder Gelatine, Schmiermitteln, wie Magnesiumstearat oder Talk, und/oder Mitteln zur Erzielung des Depoteffektes, wie Hydroxypropylcellulose, Hydroxypropylmethylcellulose, Ethylcellulose, Amminomethacrylat, Polyvinylpyrrolidon-Polyvinylacetat Copolymer, Carboxymethylcellulose oder Polyvinylacetat erhalten werden. Die Tabletten können auch aus mehreren Schichten bestehen.

Entsprechend können Dragees bzw. Filmtabletten durch Überziehen von analog den Tabletten hergestellten Kernen mit üblicherweise In Dragee- bzw. Filmüberzügen verwendeten Mitteln, beispielsweise Kollidon oder Schellack, Gummi arabicum, Talk, Titandioxid, Zucker, Hydroxypropylmethylcellulose, Ehthycellulose, Celluloseacetatphthalat, Polymethacrylate, Polyethylenglycol, Polyvinylalcohol, Polyvinylalkohol-Polyethylenglycol Copolymere oder Polyvinylacetat hergestellt werden. Zur Erzielung eines Depoteffektes oder zur Vermeldung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss-oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gefatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

### Formulierungsbeispiele:

Die folgenden Formulierungsbeispiele für Kombinationsformulierungen sollen der besseren Illustration der Erfindung dienen, ohne jedoch diese darauf zu beschränken, Insbesondere können die Wirkstoffe 1 und **2** auch in voneinander getrennten Formulierungen vorliegen und innerhalb eines Zeitfensters von maximal 6 Stunden versetzt voneinander getrennt verabreicht werden. Formulierungen, die Diclofenac oder Ibuprofen enthalten, sind Vergleichsbeispiele.
1)

| | |
|---|---|
| 0,05 mg | Wirkstoff **1** |
| 500 mg | Acetylsalicylsäure (Wirkstoff **2**) |
| 100 mg | Milchzucker |
| 329,95 mg | Microkristalline Cellulose |
| 30 mg | Polyvinylpyrrolidon |
| 30 mg | quervernetztes Polyvinylpyrrolidon |
| 10 mg | Magnesiumstearat |
| 1000 mg | |

2)

| | |
|---|---|
| 0,1 mg | Wirkstoffe **1** |
| 500 mg | Acetylsalicylsäure (Wirkstoff **2**) |
| 100 mg | Milchzucker |
| 329,9 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

3)

| | |
|---|---|
| 0,5 mg | Wirkstoff **1** |
| 500 mg | Acetylsalicylsäure (Wirkstoff **2**) |
| 100 mg | Milchzucker |
| 329,5 mg | Microkristalline Cellulose |
| 30 mg | quervemetztes Polyvinylpyrrolidon |
| 30 mg | Polyvinylpyrrolidon |
| 10 mg | Magnesiumstearat |
| 1000 mg | |

4)

| | |
|---|---|
| 5 mg | Wirkstoff **1** |
| 500 mg | Acetylsalicylsäure (Wirkstoff **2**) |
| 100 mg | Milchzucker |
| 325 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

5)

| | |
|---|---|
| 20 mg | Wirkstoff **1** |
| 500 mg | Acetylsalicylsäure (Wirkstoff **2**) |
| 100 mg | Milchzucker |
| 310 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

6)

| | |
|---|---|
| 0,05 mg | Wirkstoff **1** |
| 25 mg | Diclofenac (Wirkstoff **2**) |
| 170 mg | Milchzucker |
| 269,95 mg | Microkristalline Cellulose |
| 15 mg | quervemetztes Polyvinylpyrrolidon |
| 15 mg | Polyvinylpyrrolidon |
| 5 mg | Magnesiumstearat |
| 500 mg | |

7)

| | |
|---|---|
| 0,1 mg | Wirkstoff **1** |
| 25 mg | Diclofenac (Wirkstoff **2**) |
| 170 mg | Milchzucker |
| 269,9 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

8)

| | |
|---|---|
| 0,5 mg | Wirkstoff **1** |
| 25 mg | Diclofenac (Wirkstoff **2**) |
| 170 mg | Milchzucker |
| 269,5 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

9)

| | |
|---|---|
| 5 mg | Wirkstoff **1** |
| 25 mg | Diclofenac (Wirkstoff **2**) |
| 170 mg | Milchzucker |
| 265 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

10)

| | |
|---|---|
| 20 mg | Wirkstoff **1** |
| 25 mg | Diclofenac (Wirkstoff **2**) |
| 170 mg | Milchzucker |
| 240 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

11)

| | |
|---|---|
| 0,05 mg | Wirkstoff **1** |
| 15 mg | Meloxlcam (Wirkstoff **2**) |
| 170 mg | Milchzucker |
| 279,95 mg | Microkristalline Cellulose |
| 15 mg | quervemetztes Polyvinylpyrrolidon |
| 15 mg | Polyvinylpyrrolidon |
| 5 mg | Magnesiumstearat |
| 500 mg | |

12)

| | |
|---|---|
| 0,1 mg | Wirkstoff **1** |
| 15 mg | Meloxicam (Wirkstoff **2**) |
| 170 mg | Milchzucker |
| 279,9 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

13)

| | |
|---|---|
| 0,5 mg | Wirkstoff **1** |
| 15 mg | Meloxicam (Wirkstoff **2**) |
| 170 mg. | Milchzucker |
| 279,5 mg | Microkristalline Cellulose |
| 15 mg | quervernetztes Polyvinylpyrrolidon |
| 15 mg | Polyvinylpyrrolidon |
| 5 mg | Magnesiumstearat |
| 500 mg | |

14)

| | |
|---|---|
| 5 mg | Wirkstoff **1** |
| 15 mg | Meloxicam (Wirkstoff **2**) |
| 170 mg | Milchzucker |
| 275 mg | Microkristalline Cellulose |
| 15 mg | quervemetztes Polyvinylpyrrolidon |
| 15 mg | Polyvinylpyrrolidon |
| 5 mg | Magnesiumstearat |
| 500 mg | |

15)

| | |
|---|---|
| 20 mg | Wirkstoff **1** |
| 15 mg | Meloxicam(Wirkstoff **2**) |
| 170 mg | Milchzucker |
| 260 mg | Microkristalline Cellulose |
| 15 mg | quervemetztes Polyvinylpyrrolidon |
| 15 mg | Polyvinylpyrrolidon |
| 5 mg | Magnesiumstearat |
| 500 mg | |

16)

| | |
|---|---|
| 0,05 mg | Wirkstoff **1** |
| 500 mg | Naproxen (Wirkstoff **2**) |
| 100 mg | Milchzucker |
| 329,95 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

17)

| | |
|---|---|
| 0,1 mg | Wirkstoff **1** |
| 500 mg | Naproxen (Wirkstoff **2**) |
| 100 mg | Milchzucker |
| 329,9 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

18)

| | |
|---|---|
| 0,5 mg | Wirkstoff **1** |
| 500 mg | Naproxen (Wirkstoff **2**) |
| 100 mg | Milchzucker |
| 329,5 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

19)

| | |
|---|---|
| 5 mg | Wirkstoff **1** |
| 500 mg | Naproxen (Wirkstoff **2**) |
| 100 mg | Milchzucker |
| 325 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

20)

| | |
|---|---|
| 20 mg | Wirkstoff **1** |
| 500 mg | Naproxen (Wirkstoff **2**) |
| 100 mg | Milchzucker |
| 310 mg | Microkristalline Cellulose |
| 30 mg | quervernetztes Polyvinylpyrrolidon |
| 30 mg | Polyvinylpyrrolidon |
| 10 mg | Magnesiumstearat |
| 1000 mg | |

21)

| | |
|---|---|
| 0,05 mg | Wirkstoff **1** |
| 200 mg | Ibuprofen (Wirkstoff **2**) |
| 100 mg | Milchzucker |
| 258,95 mg | Microkristalline Cellulose |
| 18 mg | quervemetztes Polyvinylpyrrolidon |
| 18 mg | Polyvinylpyrrolidon |
| 5 mg | Magnesiumstearat |
| 600 mg | |

22)

| | |
|---|---|
| 0,1 mg | Wirkstoff **1** |
| 200 mg | Ibuprofen (Wirkstoff **2**) |
| 100 mg | Milchzucker |
| 258,9 mg | Microkristalline Cellulose |
| 18 mg | quervemetztes Polyvinylpyrrolidon |
| 18 mg | Polyvinylpyrrolidon |
| 5 mg | Magnesiumstearat |
| 600 mg | |

23)

| | |
|---|---|
| 0,5 mg | Wirkstoff **1** |
| 200 mg | Ibuprofen (Wirkstoff **2**) |
| 100 mg | Milchzucker |
| 258,5 mg | Microkristalline Cellulose |
| 18 mg | quervemetztes Polyvinylpyrrolidon |
| 18 mg | Polyvinylpyrrolidon |
| 5 mg | Magnesiumstearat |
| 600 mg | |

24)

| | |
|---|---|
| 5 mg | Wirkstoff **1** |
| 200 mg | Ibuprofen (Wirkstoff **2**) |
| 100 mg | Milchzucker |
| 254 mg | Microkristalline Cellulose |
| 18 mg | quervemetztes Polyvinylpyrrolidon |
| 18 mg | Polyvinylpyrrolidon |
| 5 mg | Magnesiumstearat |
| 600 mg | |

25)

| | |
|---|---|
| 20 mg | Wirkstoff **1** |
| 200 mg | Ibuprofen (Wirkstoff **2**) |
| 100 mg | Milchzucker |
| 239 mg | Microkristalline Cellulose |
| 18 mg | quervemetztes Polyvinylpyrrolidon |
| 18 mg | Polyvinylpyrrolidon |
| 5 mg | Magnesiumstearat |
| 600 mg | |

Der feingemahlene Wirkstoff, Milchzucker und ein Teil der Microkristallinen Cellulose werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Microkristallinen Cellulose und das quervernetzte Polyvinylpyrrolidon werden gesiebt und miteinander vermischt. Anschließend wird das Magnesiumstearat aufgesiebt und kurz untergemischt. Das Gemisch wird zu Tabletten geeigneter Form und Größe verpreßt.

## Patentansprüche

1. Arzneimittelkombination, **dadurch gekennzeichnet, dass** sie neben einem PDE4-Inhibitor der Formel **1**, 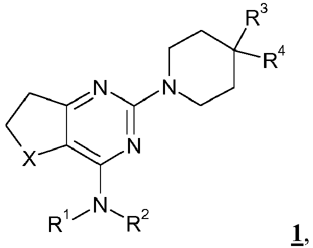 wobei
**X** SO,
**R¹** H
**R²** C₁₋₆-Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{*2*.3} substituiert sein kann, welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
**Het** ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂-alkylen,
-Het-C₁₋₂-alkylen, C₃₋₇-Cycloalkyl-C₁₋₂-alkylen, Phenyl, Hetaryl und ein Het,
der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Aikyi), CO-R^{2.1} und COOR^{2.1},
der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
**R²** ein monocyclisches C₃₋₇ Cycloalkyl, das gegebenenfalls mit einem Rest ausgewählt aus der Gruppe bestehend aus C₁₋₂-Alkanol,
C₁₋₃-Fluoroalkyl, C₁₋₃-alkylen-OR^{2.1}, OR^{2.1}, COOR^{2.1}, SO₂-NR²²R²³, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{*2*.3} substituiert sein kann,
der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{*2*.3} substituiert sein kann,
oder
**R²** ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂₋CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{*2*.3} substituiert sein kann,
oder
**R²** ein Rest ausgewählt aus einer Gruppe bestehend aus Het und Hetaryl,
der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂alkylen, -Het, -Hetaryl und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Butyl, Isobutyl, Phenyl und NR^{2.2}R^{*2*.3} substituiert sein kann,
und worin
**R³** ein Naphthalin oder Phenyl ist,
welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃; SO₂-CH₃, SO-CH₃, COOCH₃, COOCH₂CH₃ , -CO-NH-(Methylen)-Hetaryl, -CO-NH-(Ethylen)-Hetaryl, -CO-NH-Hetaryl, -CO-N(CH₃)-Het,-CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het,
-NH-CO-Methyl , NCH₃-CO-Methyl, -NH-CO-Ethyl, NCH₃-CO-Ethyl, -NH-CO-Propyl, NCH₃-CO-Propyl, -NH-CO-Isopropyl , NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO-N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₃) und -N(CH₃)₂ substituiert sein kann,
wobei dieser Rest gegebenenfalls durch einen oder mehrere Reste ausgewählt aus der Gruppe bestehend aus OH, F, Cl, -CF₃, CHF₂, CH₂F, Oxo, Methyl und Phenyl substituiert sein kann
oder worin
**R³** ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₃)₂, -NH₂,-NH(CH₃), -N(CH₃)₂, Het und Hetaryl substituiert sein kann,
der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
oder worin
**R³** -O-R^{3.1},
wobei
**R^{3.1}** ein Rest ausgewählt aus der Gruppe bestehend aus -C₁₋₃-Alkyl, -Phenyl,-C₁₋₃-Alkylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N( C₅₋₇Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂,
-Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl,, SO₂-Methyl, -SO₂-Ethyl, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇-Cycloalkyl, -CO-N(CH₃)-Cyclopropyl,
C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
**R⁴** H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl,
-CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇-Cycloalkyl, Het und Hetaryl substituiert sein kann,
bedeuten, wenigstens ein NSAID (= non-steroidal anti-inflammatory drug) (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93)
umfasst.

2. Arzneimittelkombination nach Anspruch 1, **dadurch gekennzeichnet, dass** sie neben einem PDE4-Inhibitor der Formel **1,** wobei
**R²** ein Rest nach Formel **3** 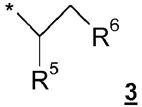
ist, worin
**R⁶** OH oder NH₂ ist und
worin
**R**⁵ ein Rest ausgewählt aus der Gruppe bestehend aus C₁₋₄-Alkyl, einem fünf- bis sechsgliedrigen Heteroaryl mit 1, 2 oder 3 Heteroatomen aus der Gruppe S, O und N und Phenyl, welcher gegebenenfalls mit einem oder mehreren Resten ausgewählt aus der Gruppe bestehend aus OH, F, Br, OR^{2.1}, Oxo, Methyl, Ethyl, Methanol, Ethanol, Phenyl, COOR^{2.1}, CH₂-NR^{2.2}R^{2.3} und NR^{2.2}R^{2.3} substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

3. Arzneimittelkombination nach Anspruch 2, **dadurch gekennzeichnet, dass** sie neben einem PDE4-Inhibitor der Formel **1,** wobei
**R²** ein Rest ist nach Formel **3** 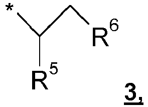 worin
**R⁶** OH oder NH₂ ist und
worin
**R⁵** Methyl, Ethyl, Propyl, Isopropyl ist
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

4. Arzneimittelkombination nach Anspruch 1, **dadurch gekennzeichnet, dass** sie neben einem PDE4-Inhibitor der Formel **1,** wobei
**R²** ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, wobei
R^{2.1} ausgewählt ist aus der Gruppe bestehend aus Methyl, Ethyl, Propyl, Isopropyl, Butyl, Isobutyl,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

5. Arzneimittelkombination nach Anspruch 1, **dadurch gekennzeichnet, dass** sie neben einem PDE4-Inhibitor der Formel **1,** wobei
**R²** ein Cyclopropyl ist, das gegebenenfalls mit einem weiteren Rest ausgewählt aus der Gruppe bestehend aus -NH₂, CH2- NH₂, -NH(CH₃), -N(CH₃)₂, Methyl, Ethyl, Propyl, Isopropyl, -NH-CO-(tert-Butyl), -NH-CO-O-(tert-Butyl),
-N(CH₃)-CO-(tert-Butyl), -N(CH₃)-CO-O-(tert-Butyl), -CF₃, -CHF₂, CH₂F, F, Cl und Br substituiert sein,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

6. Arzneimittelkombination nach Anspruch 1, **dadurch gekennzeichnet, dass** sie neben einem PDE4-Inhibitor der Formel **1,** wobei
**R²** ein Cyclopropyl
**R²** ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei R^{2.1} H, Methyl oder Ethyl, sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

7. Arzneimittelkombination nach Anspruch 1, **dadurch gekennzeichnet, dass** sie neben einem PDE4-Inhibitor der Formel **1,**
wobei
**R²** ein Rest ausgewählt aus einer Gruppe bestehend aus einem monocyclischen, gesättigten sechsgliedrigem Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃ und N(CH₃)₂, Methyl, Ethyl, Propyl, Isopropyl, Cyclopropyl, Methoxy und Ethoxy substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

8. Arzneimittelkombination nach Anspruch 7, **dadurch gekennzeichnet, dass** sie neben einer Verbindung der Formel **1**, wobei
**R²** ein Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

9. Arzneimittelkombination nach einem der Ansprüche 1 bis 8, **dadurch gekennzeichnet, dass** sie neben einem PDE4-Inhibitor der Formel **1,** wobei
**R³** ein Naphthalin oder Phenyl ist,
welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F,-OCH₃, OCH₂CH₃; SO₂-CH₃, SO₂-CH₂CH₃, COOCH₃ und CO-O-CH₂CH₃ substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

10. Arzneimittelkombination nach einem der Ansprüche 1 bis 8, **dadurch gekennzeichnet, dass** sie neben einem PDE4-Inhibitor der Formel **1,** wobei
**R³** ein Rest ausgewählt aus der Gruppe bestehend aus Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₅₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl, -COO-Propyl, -COO-Isopropyl und O-Methyl, O-Ethyl, O-Propyl und O-Isopropyl substituiert sein kann,
und worin
**R⁴** H, CN, OH, CF₃, CHF₂, CH₂F, F, Methyl, Ethyl, O-Methyl oder O-Ethyl,
wobei
**Het** ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

11. Arzneimittelkombination nach Anspruch 10, **dadurch gekennzeichnet, dass** sie neben einem PDE4-Inhibitor der Formel **1,** wobei
**R³** ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl,-COO-Ethyl und O-Methyl, O-Ethyl substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

12. Arzneimittelkombination nach einem der Ansprüche 1 bis 8, **dadurch gekennzeichnet, dass** sie neben einem PDE4-Inhibitor der Formel **1,** wobei
**R³** ein Rest ausgewählt aus einem monocyclischen, gesättigten oder teilweise gesättigten, drei- bis siebengliedrigem Heterocyclus oder einem monocyclischen fünfbis sechsgliedrigem Heteroaryl ist, der ausgewählt ist aus der Gruppe bestehend aus Imidazol, Dihydroimidazol, Oxadiazol, Oxadiazolidin, Pyrazol, Pyridin und Dihydropyrazol,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, --O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

13. Arzneimittelkombination nach einem der Ansprüche 1 bis 8, **dadurch gekennzeichnet, dass** sie neben einem oder mehreren, bevorzugt einem PDE4-Inhibitor der Formel **1,** wobei
**R**³ und **R⁴** gemeinsam einen mono- oder bicyclischen, ungesättigten oder teilweise gesättigten, drei- bis elfgliedrigen Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

14. Arzneimittelkombination nach einem der Ansprüche 1 bis 8, **dadurch gekennzeichnet, dass** sie neben einem PDE4-Inhibitor der Formel **1,** wobei
**R³** und **R⁴** gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigten, fünf- oder sechsgliedrigem Heterocyclus und einem fünf- oder sechsgliedrigem Heteroaryl substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

15. Arzneimittelkombination nach einem der Ansprüche 1 bis 8, **dadurch gekennzeichnet, dass** sie neben einem PDE4-Inhibitor der Formel **1,**
wobei
**R³** -O-R^{3.1} ist,
**R**^{3.1} ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, -CF₃, CHF₂, CH₂F, CO-(Methyl), CO-(Ethyl), CO-(Propyl), CO-(Isopropyl), CO-(Butyl), CO-(Isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂,-O-Methylen-N(Ethyl)₂, -O-Ethylen-N(Ethyl)₂, CO-NH₂, CO-NHCH₃, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇,-Cycloalkyl, -CO-Cyclopropyl, -CO-N(CH₃)-Cyclopropyl, -CO-N(CH₃)-C₄₋₇-Cycloalkyl, C₄₋₇-Cycloalkyl, Cyclopropyl, C₄₋₇-Cycloalkyl-Methylen-, Cyclopropyl-Methylen-, C₄₋₇-Cycloalkyl-Ethylen-, Cyclopropyl-Ethylen-, Hetaryl-Methylen-, Hetaryl-Ethylen- und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O- Methyl, Ethyl, O-Ethyl, OH, Oxo und CF₃ substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

16. Arzneimittelkombination nach einem der Ansprüche 1 bis 8, **dadurch gekennzeichnet, dass** sie neben einem oder mehreren, bevorzugt einem PDE4-Inhibitor der Formel **1,**
worin
**R⁴** H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl,
-CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

17. Arzneimittelkombination nach einem der Ansprüche 1 bis 8, **dadurch gekennzeichnet, dass** sie neben einem oder mehreren, bevorzugt einem PDE4-Inhibitor der Formel **1,**
worin
**R³** ein Rest ausgewählt ist aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl, O-Methyl, O-Ethyl, O-Propyl, O-Isopropyl, OH, F, Cl, Br, CF₃, Phenyl, Hetaryl und C₃₋₆-Cycloalkyl substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

18. Arzneimittelkombination nach Anspruch 1 1, **dadurch gekennzeichnet, dass** sie neben einem oder mehreren, bevorzugt einem PDE4-Inhibitor der Formel **1** ausgewählt aus der Gruppe bestehend aus:
1.1 (R)-2-{2-[4-(4-Chlorphenyl)-piperidin-1-yl]-5-oxo-6,7-dihydro-5*H*-5λ⁴-thieno[3,2-*d*]pyrimidin -4-ylamino}-3-methylbutan-1-ol
1.2 (1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-methanol
1.3 (R)-2-{2-[4-(4-Chlorphenyl)-piperidin-1-yl]-5-oxo-6,7-dihydro-5*H*-5λ⁴-thieno[3,2-*d*]pyrimidin-4-ylamino}-pentan-1-ol
1.4 (R)-1-{2-[4-(4-Chlorphenyl)-piperidin-1-yl]-5-oxo-6,7-dihydro-5*H*-5λ⁴-thieno[3,2-*d*]pyrimidin-4-ylamino}-1-(4-fluorphenyl)-2-methylpropan-2-ol
1.5 (S)-5-{2-[4-(4-Chlorphenyl)-piperidin-1-yl]-5-oxo-6,7-dihydro-5*H*-5λ⁴-thieno[3,2-*d*]pyrimidin-4-ylamino}-1-methylpiperidin-2-on
1.6 {2-[4-(4-Chlorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.7 1-(4-(1-Hydroxymethylcyclopropylamino)-5-oxo-6,7-dihydro-5*H*-5λ⁴-thieno[3,2-*d*]pyrimidin-2-yl)-3'-methyl-1'*H*-spiro[piperidin-4,4'-chinazolin]-2'(3'*H*)-on
1.8 {1-[2-(4-Benzo[*d*]isoxazol-3-yl-piperidin-1-yl)-5-oxo-6,7-dihydro-5*H*-5λ⁴-thieno[3,2-*d*]pyrimidin-4-ylamino]-cyclopropyl}-methanol
1.9 (1-{2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-ylamino}-cyclopropyl)-methanol
1.10 1-[4-((S)-1-Methyl-6-oxopiperidin-3-ylamino)-5-oxo-6,7-dihydro-5*H*-5λ⁴-thieno[3,2-*d*]pyrimidin-2-yl]-4-phenylpiperidin-4-carbonitril
1.11 3'-methyl-1-(4-(tetrahydro-2*H*-pyran-4-ylamino)-5-oxo-6,7-dihydro-5*H*-5λ⁴-thieno[3,2-*d*]pyrimidin-2-yl)-1'*H*-spiro[piperidin-4,4'-chinazolin]-2'(3'*H*)-on
1.12 (3-Fluorphenyl)-[5-oxo-2-(3,4,5,6-tetrahydro-2*H*-[4,4']bipyridinyl-1-yl)-6,7-dihydro-5*H-*5λ⁴-thieno[3,2-*d*]pyrimidin-4-yl]-amin
1.13 {2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-yl}-(3-fluorphenyl)-amin
1.14 (1-{2-[4-(2,4-Difluorphenyl)-piperidin-1-yl]-5-oxo-6,7-dihydro-5*H*-5λ⁴-thieno[3,2-*d*]pyrimidin-4-ylamino}-cyclopropyl)-methanol
1.15 {2-[4-(2,4-Difluorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.16 (S)-5-[2-(4-Benzoxazol-2-yl-piperidin-1-yl)-5-oxo-6,7-dihydro-5*H*-5λ⁴-thieno[3,2-*d*]pyrimidin-4-ylamino]-1-methylpiperidin-2-on
1.17 (1-{2-[4-(6-Fluorbenzo[*d*]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.18 (1-{2-[4-(5-Fluorbenzo[d]isoxazol-3-yl)-piperidin-1-yl]-5-oxo-6,7-dihydro-5H-5λ4-thieno[3,2-d]pyrimidin-4-ylamino}-cyclopropyl)-methanol
1.19 {2-[4-(5-Furan-2-yl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.20 (3-Fluorphenyl)-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-yl}-amin
1.21 (R)-3-Methyl-2-{5-oxo-2-[4-(3-pyridin-4-yl-[1,2,4]oxadiazol-5-yl)-piperidin-1-yl]-6,7-dihydro-5H-5λ4-thieno[3,2-d]pyrimidin-4-ylamino}-butan-1-ol
1.22 (S)-5-{2-[4-(4-Fluorphenoxy)-piperidin-1-yl]-5-oxo-6,7-dihydro-5H-5λ4-thieno[3,2-d]pyrimidin-4-ylamino}-1-methylpiperidin-2-on
1.23 (2-{4-[4-(4,5-Dihydrooxazol-2-yl)-phenoxy]-piperidin-1-yl}-5-oxo-6,7-dihydro-5H-5λ4-thieno[3,2-d]pyrimidin-4-yl)-(tetrahydropyran-4-yl)-amin
1.24 4-{1-[5-Oxo-4-(tetrahydropyran-4-ylamino)-6,7-dihydro-5H-5λ4-thieno[3,2-d]pyrimidin-2-yl]-piperidin-4-yloxy}-benzoesäure
1.25 2-(1-{2-[4-(4-Chlorphenyl)-piperidin-1-yl]-5-oxo-6,7-dihydro-5H-5λ4-thieno[3,2-d]pyrimidin-4-ylamino}-cyclopropyl)-propan-2-ol
1.26 {2-[4-(5-tert-Butyl-1-methyl-1H-indol-3-yl)-piperidin-1-yl]-5-oxo-6,7-dihydro-5H-5λ4-thieno[3,2-d]pyrimidin-4-yl}-(tetrahydropyran-4-yl)-amin
1.27 2-[4-(5-Furan-2-yl-1-methyl-1H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.28 (S)-5-(2-{4-[4-(4,5-Dihydrooxazol-2-yl)-phenoxy]-piperidin-1-yl}-5-oxo-6,7-dihydro-5H-5λ4-thieno[3,2-d]pyrimidin-4-ylamino)-1-methylpiperidin-2-on
1.29 {2-[4-(5-Furan-2-yl-2-methyl-2H-pyrazol-3-yl)-piperidin-1-yl]-5-oxo-6,7-dihydro-5H-5λ4-thieno[3,2-d]pyrimidin-4-yl}-(tetrahydropyran-4-yl)-amin
1.30 {2-[4-(1-Methyl-1H-imidazo[4,5-c]pyridin-2-yl)-piperidin-1-yl]-5-oxo-6,7-dihydro-5H-5λ4-thieno[3,2-d]pyrimidin-4-yl}-(tetrahydropyran-4-yl)-amin
1.31 2-Methoxy-N-{1-[5-oxo-4-(tetrahydropyran-4-ylamino)-6,7-dihydro-5H-5λ4-thieno[3,2-d]pyrimidin-2-yl]-4-phenylpiperidin-4-ylmethyl}-acetamid
1.32 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yl}-benzamid
1.33 N-Cyclopropyl-N-methyl-4-{1-[5-oxo-4-(tetrahydropyran-4-ylamino)-6,7-dihydro-5H-5λ4-thieno[3,2-d]pyrimidin-2-yl]-piperidin-4-yloxy}-benzamid
1.34 {5-Oxo-2-[4-(pyridin-4-yloxy)-piperidin-1-yl]-6,7-dihydro-5H-5λ4-thieno[3,2-d]pyrimidin-4-yl}-(tetrahydropyran-4-yl)-amin
1.35 {2-[4-(4-Chlorphenoxy)-piperidin-1-yl]-5-oxo-6,7-dihydro-5H-5λ4-thieno[3,2-d]pyrimidin-4-yl}-(tetrahydropyran-4-yl)-amin
1.36 (S)-1-Methyl-5-{2-[4-(5-methyl-4-phenyloxazol-2-yl)-piperidin-1-yl]-5-oxo-6,7-dihydro-5H-5λ4-thieno[3,2-d]pyrimidin-4-ylamino}-piperidin-2-on
1.37 (1-{2-[4-(5-Methyl-4-phenyloxazol-2-yl)-piperidin-1-yl]-5-oxo-6,7-dihydro-5H-5λ4-thieno[3,2-d]pyrimidin-4-ylamino}-cyclopropyl)-methanol
1.38 (S)-5-{2-[4-(4,5-Diphenyloxazol-2-yl)-piperidin-1-yl]-5-oxo-6,7-dihydro-5H-5λ4-thieno[3,2-d]pyrimidin-4-ylamino}-1-methylpiperidin-2-on
1.39 {4-(4-Chlorphenyl)-1-[5-oxo-4-(tetrahydropyran-4-ylamino)-6,7-dihydro-5H-5λ4-thieno[3,2-d]pyrimidin-2-yl]-piperidin-4-yl}-methanol
1.40 [1-(2-{4-[5-(4-Chlorphenyl)-4-methyloxazol-2-yl]-piperidin-1-yl}-5-oxo-6,7-dihydro-5H-5λ4-thieno[3,2-d]pyrimidin-4-ylamino)-cyclopropyl]-methanol
1.41 4-(4-Chlorphenyl)-1-[5-oxo-4-(tetrahydropyran-4-ylamino)-6,7-dihydro-5H-5λ4-thieno[3,2-d]pyrimidin-2-yl]-piperidin-4-ol
1.42 {2-[4-(4-Chlorphenyl)-4-methoxypiperidin-1-yl]-5-oxo-6,7-dihydro-5H-5λ4-thieno[3,2-d]pyrimidin-4-yl}-(tetrahydropyran-4-yl)-amin
1.43 4-{1-[4-(1-Hydroxymethylcyclopropylamino)-5-oxo-6,7-dihydro-5H-5λ4-thieno[3,2-d]pyrimidin-2-yl]-piperidin-4-yloxy}-benzonitril
1.44 5-Oxo-2-[4-(4,5,6,7-tetrahydrobenzoxazol-2-yl)-piperidin-1-yl]-6,7-dihydro-5H-5λ4-thieno[3,2-d]pyrimidin-4-yl}-(tetrahydropyran-4-yl)-amin
1.45 (S)-5-{2-[4-(4-Chlorphenyl)-piperidin-1-yl]-5,5-dioxo-6,7-dihydro-5H-5λ6-thieno[3,2-d]pyrimidin-4-ylamino}-1-methylpiperidin-2-on
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

19. Arzneimittelkombination nach einem der Ansprüche 1 bis 19, **dadurch gekennzeichnet, dass** sie neben einem PDE4-Inhibitor der allgemeinen Formel **1** ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125) umfasst.

20. Arzneimittelkombination nach einem der Ansprüche 1 bis 19, **dadurch gekennzeichnet, dass** als NSAID **(2)** entweder Acetylsalicylsäure (2.3) in einer Einzeldosis von 50 bis 2000 mg, Meloxicam (2.77) in einer Einzeldosis von 7,5 mg bis 30 mg und Naproxen in einer Einzeldosis von 250 bis 1000 mg eingesetzt wird.

21. Arzneimittelkombination nach einem der Ansprüche 1 bis 20 ,
zur Verwendung in der Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

22. Arzneimittelkombination nach Anspruch 21 zur Verwendung in der Behandlung einer Erkrankung ausgewählt aus COPD, chronischer Sinusitis, Asthma, Morbus Crohn, idiophatischer Lungenfibrose und Colitis ulcerosa.

## Claims

1. Drug combination, **characterised in that** in addition to a PDE4 inhibitor of general formula **1**, 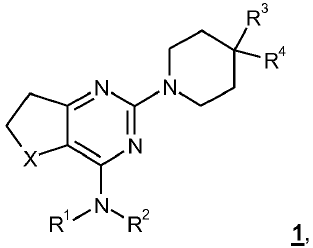 wherein
**X** denotes SO,
**R¹** denotes H,
**R²** is C₁₋₆-alkyl, which may optionally be substituted by one or more groups selected from F, Cl, CF₃, CHF₂ or CH₂F or which may optionally be substituted by one or more groups selected from among OR^{2.1}, COOR^{2.1}, CONR^{2.2}R^{2.3}, SR^{2.1}, SO-R^{2.1}, SO₂-R^{2.1} phenyl, het, hetaryl, a monocyclic C_{3.7}-cycloalkyl, CH₂-NR^{2.2}R^{2.3} and NR^{2.2}R^{2.3},
which in turn may optionally be substituted by one or more groups selected from among OH, F, Cl, Br, CF₃, CHF₂, CH₂F, OR^{2.1}, oxo, methyl, ethyl, propyl, isopropyl, methanol, ethanol, phenyl, COOR^{2.1}, CH₂-NR^{2.2}R^{2.3} and NR^{2.2}R^{2.3},
wherein
**het** is a three- to seven-membered, monocyclic, saturated or partly saturated heterocyclic group which contains 1, 2 or 3 heteroatoms selected independently of one another from among N, S or O,
and wherein
**hetaryl** is a five- to six-membered, monocyclic, aromatic heteroaryl, which contains 1, 2 or 3 heteroatoms selected independently of one another from among N, S or O,
and wherein
**cycloalkyl** may be saturated or partly saturated,
wherein **R^{2.1}** is H or a group selected from among methyl, ethyl, propyl, isopropyl, methanol, ethanol, monocyclic C₃₋₇-cycloalkyl, phenyl-C₁₋₂-alkylene, -hetaryl-C₁₋₂-alkylene, -het-C₁₋₂-alkylene, C₃₋₇-cycloalkyl-C₁₋₂-alkylene, phenyl, hetaryl and a het, which may optionally be substituted by one or more groups selected from among OH, F, Cl, methyl, ethyl, propyl, isopropyl, O-methyl, O-ethyl, O-propyl, O-isopropyl and phenyl,
wherein **R^{2.2}** and **R**^{2.3} independently of one another denote H or denote a group selected from among methyl, ethyl, propyl, isopropyl, monocyclic C₃₋₇-cycloalkyl, phenyl-C₁₋₃-alkylene, hetaryl-C₁₋₃-alkylene, phenyl, -het, -hetaryl, CO-NH₂, CO-NHCH₃, CON(CH₃)₂, SO₂-(C₁₋₂-alkyl), CO-R^{2.1} and COOR^{2.1},
which may optionally be substituted by one or more groups selected from among OH, F, Cl, methyl, ethyl, propyl, isopropyl, phenyl and COOR^{2.1},
or
**R²** denotes a monocyclic C₃₋₇ cycloalkyl, which may optionally be substituted by a group selected from among C₁₋₂-alkanol, C₁₋₃-fluoroalkyl, C₁₋₃alkylene-OR^{2.1}, OR^{2.1}, COOR^{2.1}, SO₂-NR^{2.2}R^{2.3}, -het, -NH-CO-O-(phenyl), methyl, ethyl, propyl, isopropyl, phenyl, phenyl-C₁₋₂-alkylene, -hetaryl-C₁₋₂-alkylene, monocyclic C₃₋₁₇-cycloalkyl and NR^{2.2}R^{2.3},
which may optionally be substituted by one or more groups selected from among OH, OR^{2.1}, oxo, F, Cl, CF₃, CHF₂, CH₂F, methyl, ethyl, propyl, isopropyl, phenyl and NR^{2.2}R^{2.3},
or
**R²** denotes a phenyl, which may optionally be substituted by OH, SH, F, Cl or Br or by one or more groups selected from among OR^{2.1}, COOR^{2.1}, NR^{2.2}R^{2.3}, CH₂-NR^{2.2}R^{2.3}, monocyclic C₃₋₇-cycloalkyl, -het, methyl, ethyl, propyl, isopropyl, CF₃, CHF₂, CH₂F, phenyl-C₁₋₂-alkylene, het-C₁₋₂-alkylene, hetaryl-C₁₋₂-alkylene, phenyl, SO₂-CH₃, SO₂-CH₂CH₃ and SO₂-NR^{2.2}R^{2.3},
which in turn may optionally be substituted by one or more groups selected from among OH, OR^{2.1}, oxo, F, Cl, CF₃, CHF₂, CH₂F, methyl, ethyl, propyl, isopropyl, phenyl and NR^{2.2}R^{2.3},
or
**R²** denotes a group selected from among het and hetaryl,
which may optionally be substituted by one or more groups selected from among F, Cl, OH, oxo, CF₃, CHF₂ and CH₂F or by one or more groups selected from among OR^{2.1}, C₁₋₃alkylene-OR^{2.1}, SR^{2.1}, SO-R^{2.1}, SO₂-R^{2.1}, COOR^{2.1}, COR^{2.1}, methanol, ethanol, monocyclic C₃₋₇-cycloalkyl, phenyl, methyl, ethyl, propyl, isopropyl, phenyl-C₁₋₂-alkylene, hetaryl-C₁₋₂-alkylene, -het, -hetaryl and NR^{2.2}R^{2.3},
which in turn may optionally be substituted by one or more groups selected from among OH, OR^{2.1}, oxo, F, Cl, CF₃, CHF₂, CH₂F, methyl, ethyl, propyl, isopropyl, butyl, isobutyl, phenyl and NR^{2.2}R^{2.3},
and wherein
**R³** is a naphthalene or phenyl,
which may optionally be substituted in the ortho, para or meta position by one or two groups selected independently of one another from among fluorine, chlorine, bromine, hydroxy, CN, methyl, ethyl, propyl, isopropyl, cyclopropyl, CF₃, CHF₂, CH₂F, -OCH₃, OCH₂CH₃, SO₂-CH₃, SO-CH₃, COOCH₃, COOCH₂CH₃, -CO-NH-(methylene)-hetaryl, -CO-NH-(ethylene)-hetaryl, -CO-NH-hetaryl, -CO-N(CH₃)-het, - CO-N(CH₃)-(methylene)-het, -CO-N(CH₃)-(ethylene)-het, -CO-N(CH₃)-(methylene)-hetaryl, -CO-N(CH₃)-(ethylene)-hetaryl, -CO-N(cyclopropyl)-het, CO-NH₂, CONH(CH₃), CON(CH₃)₂, -CO-NH-(methylene)-het, -CO-NH-(ethylene)-het, -NH-CO-methyl, NCH₃-CO-methyl, -NH-CO-ethyl, NCH₃-CO-ethyl, -NH-CO-propyl, NCH₃-CO-propyl, -NH-CO-isopropyl , NCH₃-CO-isopropyl, phenyl, phenyl-methylene, phenylethylene, het-methylene, het-ethylene, -het, -CO-het, -CO-N(CH₃)-het, CO-N(CH₃)-cyclopropyl, C₃₋₇-cycloalkyl, C₃₋₇-cycloalkyl-methylene, C₃₋₇-cycloalkyl-ethylene, hetaryl-methylene, hetaryl-ethylene, -hetaryl, CH₂-NH₂, CH₂-NH(CH₃), CH₂-N(CH₃)₂, -NH₂, -NH(CH₃) and -N(CH₃)₂,
wherein this group may optionally be substituted by one or more groups selected from among OH, F, Cl, -CF₃, CHF₂, CH₂F, oxo, methyl and phenyl,
or wherein
**R³** denotes a group selected from among het and hetaryl, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO-(CH₃), SO-(CH₂-CH₃), SO₂-(CH₃), SO₂-(CH₂-CH₃), phenyl, CH₂-NH₂, CH₂-NH(CH₃), CH₂-N(CH₃)₂, -NH₂, -NH(CH₃), -N(CH₃)₂, het and hetaryl,
which in turn may optionally be substituted by one or more groups selected from among OH, F, Cl, CF₃, CHF₂, CH₂F, methyl, ethyl, propyl, isopropyl, phenyl, -COO-methyl, -COO-ethyl and O-methyl, O-ethyl,
or wherein
**R³** denotes -O-R^{3.1},
wherein
**R**^{3.1} is a group selected from among -C₁₋₃-alkyl, -phenyl, -C₁₋₃-alkylene-phenyl, hetaryl and het,
which is optionally substituted in the ortho, para or meta position by one, two or three groups selected independently of one another from among fluorine, chlorine, bromine, hydroxy, CN, methyl, ethyl, propyl, isopropyl, butyl, isobutyl, CF₃, CHF₂, CH₂F, CO-(methyl), CO-(ethyl), CO-(propyl), CO-(isopropyl), -CO-(CF₃), -CO-NH-(methylene)-hetaryl, -CO-NH-(ethylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl, -CO-N(CH₃)-het, -CO-N(cyclopropyl)-het, -CO-N(C₅₋₇-cycloalkyl)-het, -methylene-O-methyl, -ethylene-O-methyl, -propylene-O-methyl, -methylene-O-ethyl, -ethylene-O-ethyl, -propylene-O-ethyl, -methylene-NH₂, -methylene-NHCH₃, -methylene-N(CH₃)₂, -ethylene-NH₂, -ethylene-NHCH₃, -ethylene-N(CH₃)₂, NH₂, N(CH₃)₂, NHCH₃, -O-methyl, O-ethyl, O-propyl, O-isopropyl, O-butyl, O-isobutyl, -SO-CH₃, SO-ethyl, -SO-propyl, -SO-isopropyl, SO₂-methyl, -SO₂-ethyl, SO₂-propyl, SO₂-isopropyl, COOH, COO-(methyl), COO-(ethyl), COO-(propyl), COO-(isopropyl), -O-methylene-N(methyl)₂, -O-ethylene-N(methyl)₂, -O-methylene-N(ethyl)₂, -O-ethylene-N(ethyl)₂, CO-NH₂, CO-NH(CH₃), CO-N(CH₃)₂, -NH-CO-methyl, -NCH₃-CO-methyl, -NH-CO-ethyl, NCH₃-CO-ethyl, phenyl, phenyl-methylene-, phenyl-ethylene-, het-methylene-, het-ethylene-, -CO-het, het, -CO-C₅₋₇-cycloalkyl, -CO-cyclopropyl, -CO-N(CH₃)-C₅₋₇-cycloalkyl, -CO-N(CH₃)-cyclopropyl, C₅₋₇-cycloalkyl, cyclopropyl, C₅₋₇-cycloalkyl-methylene, C₅₋₇-cycloalkyl-ethylene, cyclopropyl-methylene, cyclopropyl-ethylene, hetaryl-methylene, hetaryl-ethylene and hetaryl,
which in turn may optionally be substituted by 1, 2, 3 or 4 groups selected independently of one another from among F, Cl, Br, methyl, O-methyl, ethyl, O-ethyl, OH, oxo and CF₃,
and wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl), -(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl, -ethylene-O-ethyl, -(methylene)-N(CH₃)-CO-(methyl), -(methylene)-N(CH₃)-CO-(ethyl) -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-CO-(ethylene)-N(methyl)₂, -methylene-NH-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(methyl), -(ethylene)-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(methylene)-O-(ethyl), -O-(methylene)-phenyl, -O-(ethylene)-phenyl,
-CO-phenyl,
wherein the phenyl in the above groups may optionally be substituted by one or more other groups selected from among F, Cl, Br, methyl, ethyl, propyl, -O-methyl, -O-ethyl, -O-propyl, -OH and CF₃,
or wherein
**R³** and **R⁴** together form a mono- or bicyclic, unsaturated, saturated or partly saturated heterocyclic group which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), N(CH₃)₂, phenyl, C₅₋₇-cycloalkyl, het and hetaryl,
it contains at least one NSAID (= non-steroidal anti-inflammatory drug) (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

2. Drug combination according to claim 1, **characterised in that** in addition to a PDE4 inhibitor of formula **1**, wherein
**R²** is a group according to formula **3** 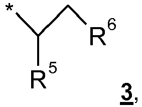 wherein
**R⁶** is OH or NH₂ and
wherein
**R⁵** denotes a group selected from among C₁₋₄-alkyl, a five- to six-membered heteroaryl with 1, 2 or 3 heteroatoms selected from among S, O and N and phenyl, which may optionally be substituted by one or more groups selected from among OH, F, Br, OR^{2.1}, oxo, methyl, ethyl, methanol, ethanol, phenyl, COOR^{2.1}, CH₂-NR^{2.2}R^{2.3} and NR^{2.2}R^{2.3},
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

3. Drug combination according to claim 2, **characterised in that** in addition to a PDE4 inhibitor of formula **1**, wherein
**R²** is a group according to formula **3** 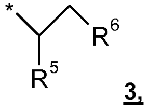 wherein
**R⁶** is OH or NH₂ and
wherein
**R⁵** is methyl, ethyl, propyl, isopropyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

4. Drug combination according to claim 1, **characterised in that** in addition to a PDE4 inhibitor of formula **1,** wherein
**R²** is a monocyclic three-, four-, five-, six- or seven-membered cycloalkyl ring which may optionally be substituted in the spiro position by a group selected from among -CH₂-OR^{2.1}, branched or unbranched C₂₋₆-alkylene-OR^{2.1}, methyl, ethyl, propyl, isopropyl, butyl, isobutyl, cyclopropyl, -CF₃, CHF₂, CH₂F and C₂₋₄-fluoroalkyl, wherein
R^{2.1} is selected from among methyl, ethyl, propyl, isopropyl, butyl, isobutyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

5. Drug combination according to claim 1, **characterised in that** in addition to a PDE4 inhibitor of formula **1,** wherein
**R²** is a cyclopropyl which may optionally be substituted by another group selected from among -NH₂, CH₂- NH₂, -NH(CH₃), -N(CH₃)₂, methyl, ethyl, propyl, isopropyl, -NH-CO-(tert-butyl), -NH-CO-O-(tert-butyl), -N(CH₃)-CO-(tert-butyl), -N(CH₃)-CO-O-(tert-butyl), -CF₃, -CHF₂, CH₂F, F, Cl and Br,
it contains at least one NSAID (2) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

6. Drug combination according to claim 1, **characterised in that** in addition to a PDE4 inhibitor of formula **1,** wherein
**R²** denotes a cyclopropyl
**R²** is a phenyl which may optionally be substituted in one or both meta positions by one or more groups selected from among methyl, ethyl, propyl, isopropyl, cyclopropyl, F, Cl, Br, OH, OR^{2.1}, COOR^{2.1}, CF₃, CHF₂, CH₂F, NH₂, NH(CH₃) and N(CH₃)₂, wherein R^{2.1} may be H, methyl or ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

7. Drug combination according to claim 1, **characterised in that** in addition to a PDE4 inhibitor of formula **1**,
wherein
**R²** is a group selected from among a monocyclic, saturated six-membered heterocyclic group with one heteroatom selected from among N, O and S, which may optionally be substituted by one or more groups selected from among F, Cl, Br, CF₃, CHF₂, CH₂F, OH, oxo, NH₂, NHCH₃ and N(CH₃)₂, methyl, ethyl, propyl, isopropyl, cyclopropyl, methoxy and ethoxy,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

8. Drug combination according to claim 7, **characterised in that** in addition to a compound of formula **1**, wherein
**R²** denotes a group selected from among piperidine or tetrahydropyran, which may optionally be substituted by one or more groups selected from among F, Cl, Br, OH, CF₃, CHF₂, CH₂F, NH₂, NHCH₃, N(CH₃)₂, oxo, methyl and methoxy,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

9. Drug combination according to one of claims 1 to 8, **characterised in that** in addition to a PDE4 inhibitor of formula **1,** wherein
**R³** is a naphthalene or phenyl,
which may optionally be substituted in any desired position by one, two or three groups selected independently of one another from among fluorine, chlorine, bromine, hydroxy, CN, methyl, ethyl, propyl, isopropyl, cyclopropyl, CF₃, CHF₂, CH₂F, -OCH₃, OCH₂CH₃, SO₂-CH₃, SO₂-CH₂CH₃, COOCH₃ and CO-O-CH₂CH₃,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

10. Drug combination according to one of claims 1 to 8, **characterised in that** in addition to a PDE4 inhibitor of formula **1,** wherein
**R³** denotes a group selected from among het and hetaryl, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, C₅₋₇-cycloalkyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, het and hetaryl, which in turn may be substituted by one or more groups selected from among OH, F, Cl, Br, CF₃, CHF₂, CH₂F, methyl, ethyl, propyl, isopropyl, phenyl, -COO-methyl, -COO-ethyl, -COO-propyl, -COO-isopropyl and O-methyl, O-ethyl, O-propyl and O-isopropyl,
and wherein
**R⁴** denotes H, CN, OH, CF₃, CHF₂, CH₂F, F, methyl, ethyl, O-methyl or O-ethyl,
wherein
**het** is a three- to seven-membered, monocyclic, saturated or partly saturated heterocyclic group or a seven- to eleven-membered, bicyclic, anellated, saturated or partly saturated heterocyclic group which contains 1, 2 or 3 heteroatoms selected independently of one another from among N, S or O,
and wherein
**hetaryl** is a five- to six-membered, monocyclic, aromatic heteroaryl or a seven- to eleven-membered, bicyclic, annelated, aromatic heteroaryl which contains in each case 1, 2 or 3 heteroatoms selected independently of one another from among N, S or O,
and wherein
**cycloalkyl** may be saturated or partly saturated,
it contains at least one NSAID **(2)** selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

11. Drug combination according to claim 10, **characterised in that** in addition to a PDE4 inhibitor of formula **1,** wherein
**R³** is a group selected from a bicyclic, seven- to eleven-membered, saturated or partly saturated heterocyclic group or a bicyclic, seven- to eleven-membered heteroaryl, which is selected from among indole, dihydroindole, quinazoline, dihydroquinazoline, tetrahydroquinazoline, benzoisoxazole, dihydrobenzoisoxazole, benzoxazine, dihydrobenzoxazine, benzothiazole, dihydrobenzothiazole, triazolopyridine, dihydrotriazolopyridine, benzofuran, dihydrobenzofuran, isobenzofuran and dihydroisobenzofuran,
which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl,
which in turn may be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

12. Drug combination according to one of claims 1 to 8, **characterised in that** in addition to a PDE4 inhibitor of formula **1**, wherein
**R³** is a group selected from a monocyclic, saturated or partly saturated, three- to seven-membered heterocyclic group or a monocyclic five- to six-membered heteroaryl,
which is selected from among imidazole, dihydroimidazole, oxadiazole, oxadiazolidine, pyrazole, pyridine and dihydropyrazole,
which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl, which in turn may be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

13. Drug combination according to one of claims 1 to 8, **characterised in that** in addition to a PDE4 inhibitor of formula **1**, wherein
**R³** and **R⁴** together form a mono- or bicyclic, unsaturated or partly saturated, three- to eleven-membered heterocyclic group which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), SO-(CH₃), SO-(CH₂-CH₃), phenyl, -CH₂-NH₂, -CH₂NHCH₃, -CH₂-N(CH₃)₂, NH₂, NHCH₃, N(CH₃)₂, a saturated or partly saturated, five- to six-membered heterocyclic group and a five- to six-membered heteroaryl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

14. Drug combination according to one of claims 1 to 8, **characterised in that** in addition to a PDE4 inhibitor of formula **1**, wherein
**R³** and **R⁴** together form a bicyclic heterocyclic group selected from among tetrahydroquinazoline, tetrahydrobenzoxazine and dihydroindole, dihydroisobenzofuran, which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), phenyl, -CH₂-NH₂,-CH₂NHCH₃, -CH₂-N(CH₃)₂, NH₂, NHCH₃, N(CH₃)₂, a saturated or partly saturated, five or six-membered heterocyclic group and a five or six-membered heteroaryl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

15. Drug combination according to one of claims 1 to 8, **characterised in that** in addition to a PDE4-inhibitor of formula **1**,
wherein
**R³** is-O-R^{3.1},
**R^{3.1}** is a group selected from among methyl, ethyl, propyl, isopropyl, butyl, isobutyl, pentyl, isopentyl, -phenyl, -methylene-phenyl, -ethylene-phenyl, -propylene-phenyl, -isopropylene-phenyl, hetaryl and het,
which is optionally substituted in the ortho, para or meta position by one, two or three groups selected independently of one another from among fluorine, chlorine, bromine, hydroxy, CN, methyl, ethyl, propyl, isopropyl, butyl, isobutyl, -CF₃, CHF₂, CH₂F, CO-(methyl), CO-(ethyl), CO-(propyl), CO-(isopropyl), CO-(butyl), CO-(isobutyl), -CO-(CF₃), -CO-(CH₂F), -CO-(CHF₂), -CO-NH-(methylene)-hetaryl, -CO-NH-(ethylene)-hetaryl, -CO-NH-(propylene)-hetaryl, -CO-NH-(isopropylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl, -CO-N(CH₃)-het, -CO-N(C₃₋₇cycloalkyl)-het, -methylene-O-methyl, -ethylene-O-methyl, -methylene-O-ethyl, -ethylene-O-ethyl, -methylene-NH₂, -ethylene-NH₂, -methylene-NHCH₃, -ethylene-NHCH₃, -methylene-N(CH₃)₂, -ethylene-N(CH₃)₂, -NH₂, -NHCH₃, -N(CH₃)₂, -O-methyl, -O-ethyl, -O-propyl, -O-isopropyl, -SO-CH₃, -SO-(CH₂CH₃), -SO₂-CH₃, -SO₂-(CH₂CH₃), COOH, COO-(methyl), COO-(ethyl), COO-(propyl), COO-(isopropyl), -O-methylene-N(methyl)₂, -O-ethylene-N(methyl)₂,-O-methylene-N(ethyl)₂, -O-ethylene-N(ethyl)₂, CO-NH₂, CO-NHCH₃, CO-N(CH₃)₂, NH-CO-methyl, NCH₃-CO-methyl, NH-CO-ethyl, N(CH₃)-CO-ethyl,
phenyl, phenyl-methylene, phenyl-ethylene, het-methylene, het-ethylene, -CO-het, het, -CO-C₄₋₇-cycloalkyl, -CO-cyclopropyl, -CO-N(CH₃)-cyclopropyl, -CO-N(CH₃)-C₄₋₇-cycloalkyl, C₄₋₇-cycloalkyl, cyclopropyl, C₄₋₇-cycloalkyl-methylene-, cyclopropyl-methylene-, C₄₋₇-cycloalkyl-ethylene-, cyclopropyl-ethylene-, hetaryl-methylene-, hetaryl-ethylene- and hetaryl,
which in turn may optionally be substituted by 1, 2, 3 or 4 groups selected independently of one another from among F, Cl, Br, methyl, O-methyl, ethyl, O-ethyl, OH, oxo and CF₃,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

16. Drug combination according to one of claims 1 to 8, **characterised in that** in addition to one or more, preferably one PDE4 inhibitor of formula **1**,
wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl), -(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl -ethylene-O-ethyl, -(methylene)-N(CH₃)-CO-(methyl), -(methylene)-N(CH₃)-CO-(ethyl) -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-CO-(ethylene)-N(methyl)₂, -methylene-NH-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(methyl), -(ethylene)-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(methylene)-O-(ethyl), -O-(methylene)-phenyl, -O-(ethylene)-phenyl,
-CO-phenyl,
wherein the phenyl in the above groups may optionally be substituted by one or more other groups selected from among F, Cl, Br, methyl, ethyl, propyl, -O-methyl, -O-ethyl, -O-propyl, -OH and CF₃,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

17. Drug combination according to one of claims 1 to 8, **characterised in that** in addition to one or more, preferably one PDE4 inhibitor of formula **1**,
wherein
**R³** is a group selected from among oxazole, imidazole and thiazole, wherein this group may optionally be substituted by one, two or three other groups selected independently of one another from among methyl, ethyl, propyl, isopropyl, O-methyl, O-ethyl, O-propyl, O-isopropyl, OH, F, Cl, Br, CF₃, phenyl, hetaryl and C₃₋₆-cycloalkyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

18. Drug combination according to claim 11, **characterised in that** in addition to one or more, preferably one PDE4 inhibitor of formula 1 selected from among:
1.1 (R)-2-{2-[4-(4-chlorophenyl)-piperidin-1-yl]-5-oxo-6,7-dihydro-5H-5λ⁴-thieno[3,2-d]pyrimidin-4-ylamino}-3-methylbutan-1-ol
1.2 (1-{2-[4-(4-chlorophenyl)-piperidin-1-yl]-5-oxo-6,7-dihydro-5H-5λ⁴-thieno[3,2-d]pyrimidin-4-ylamino}-cyclopropyl)-methanol
1.3 (R)-2-{2-[4-(4-chlorophenyl)-piperidin-1-yl]-5-oxo-6,7-dihydro-5H-5λ⁴-thieno[3,2-d]pyrimidin-4-ylamino}-pentan-1-ol
1.4 (R)-1-{2-[4-(4-chlorophenyl)-piperidin-1-yl]-5-oxo-6,7-dihydro-5H-5λ⁴-thieno[3,2-d]pyrimidin-4-ylamino}-1-(4-fluorophenyl)-2-methylpropan-2-ol
1.5 (S)-5-{2-[4-(4-chlorophenyl)-piperidin-1-yl]-5-oxo-6,7-dihydro-5H-5λ⁴-thieno[3,2-d]pyrimidin-4-ylamino}-1-methylpiperidin-2-one
1.6 {2-[4-(4-chlorophenyl)-piperidin-1-yl]-5-oxo-6,7-dihydro-5H-5λ⁴-thieno[3,2-d]pyrimidin-4-yl}-(tetrahydropyran-4-yl)-amine
1.7 1-(4-(1-hydroxymethylcyclopropylamino)-5-oxo-6,7-dihydro-5H-5λ⁴-thieno[3,2-d]pyrimidin-2-yl)-3'-methyl-1'H-spiro[piperidin-4,4'-quinazolin]-2'(3'H)-one
1.8 {1-[2-(4-benzo[d]isoxazol-3-yl-piperidin-1-yl)-5-oxo-6,7-dihydro-5H-5λ⁴-thieno[3,2-d]pyrimidin-4-ylamino]-cyclopropyl}-methanol
1.9 (1-{2-[4-(2-ethyl-5-fluoro-1H-indol-3-yl)-piperidin-1-yl]-5-oxo-6,7-dihydro-5H-5λ⁴-thieno[3,2-d]pyrimidin-4-ylamino}-cyclopropyl)-methanol
1.10 1-[4-((S)-1-methyl-6-oxopiperidin-3-ylamino)-5-oxo-6,7-dihydro-5H-5λ⁴-thieno[3,2-d]pyrimidin-2-yl]-4-phenylpiperidin-4-carbonitrile
1.11 3'-methyl-1-(4-(tetrahydro-2H-pyran-4-ylamino)-5-oxo-6,7-dihydro-5H-5λ⁴-thieno[3,2-d]pyrimidin-2-yl)-1'H-spiro[piperidin-4,4'-quinazolin]-2'(3'H)-one
1.12 (3-fluorophenyl)-[5-oxo-2-(3,4,5,6-tetrahydro-2H-[4,4']bipyridinyl-1-yl)-6,7-dihydro-5H-5λ⁴-thieno[3,2-d]pyrimidin-4-yl]-amine
1.13 {2-[4-(2-ethyl-5-fluoro-1H-indol-3-yl)-piperidin-1-yl]-5-oxo-6,7-dihydro-5H-5λ⁴-thieno[3,2-d]pyrimidin-4-yl}-(3-fluorophenyl)-amine
1.14 (1-{2-[4-(2,4-difluorophenyl)-piperidin-1-yl]-5-oxo-6,7-dihydro-5H-5λ⁴-thieno[3,2-d]pyrimidin-4-ylamino}-cyclopropyl)-methanol
1.15 {2-[4-(2,4-difluorophenyl)-piperidin-1-yl]-5-oxo-6,7-dihydro-5H-5λ⁴-thieno[3,2-d]pyrimidin-4-yl}-(tetrahydropyran-4-yl)-amine
1.16 (S)-5-[2-(4-benzoxazol-2-yl-piperidin-1-yl)-5-oxo-6,7-dihydro-5H-5λ⁴-thieno[3,2-d]pyrimidin-4-ylamino]-1-methylpiperidin-2-one
1.17 (1-{2-[4-(6-fluorobenzo[d]isoxazol-3-yl)-piperidin-1-yl]-5-oxo-6,7-dihydro-5H-5λ⁴-thieno[3,2-d]pyrimidin-4-ylamino}-cyclopropyl)-methanol
1.18 (1-{2-[4-(5-fluorobenzo[d]isoxazol-3-yl)-piperidin-1-yl]-5-oxo-6,7-dihydro-5H-5λ⁴-thieno[3,2-d]pyrimidin-4-ylamino}-cyclopropyl)-methanol
1.19 {2-[4-(5-furan-2-yl-2H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amine
1.20 (3-fluorophenyl)-{5-oxo-2-[4-(3-pyridin-4-yl-[1,2,4]oxadiazol-5-yl)-piperidin-1-yl]-6,7-dihydro-5H-5λ⁴-thieno[3,2-d]pyrimidin-4-yl}-amine
1.21 (R)-3-methyl-2-{5-oxo-2-[4-(3-pyridin-4-yl-[1,2,4]oxadiazol-5-yl)-piperidin-1-yl]-6,7-dihydro-5H-5λ⁴-thieno[3,2-d]pyrimidin-4-ylamino}-butan-1-ol
1.22 (S)-5-{2-[4-(4-fluorophenoxy)-piperidin-1-yl]-5-oxo-6,7-dihydro-5H-5λ⁴-thieno[3,2-d]pyrimidin-4-ylamino}-1-methylpiperidin-2-one
1.23 (2-{4-[4-(4,5-dihydrooxazol-2-yl)-phenoxy]-piperidin-1-yl}-5-oxo-6,7-dihydro-5H-5λ⁴-thieno[3,2-d]pyrimidin-4-yl)-(tetrahydropyran-4-yl)-amine
1.24 4-{1-[5-oxo-4-(tetrahydropyran-4-ylamino)-6,7-dihydro-5H-5λ⁴-thieno[3,2-d]pyrimidin-2-yl]-piperidin-4-yloxy}-benzoic acid
1.25 2-(1-{2-[4-(4-chlorophenyl)-piperidin-1-yl]-5-oxo-6,7-dihydro-5H-5λ⁴-thieno[3,2-d]pyrimidin-4-ylamino}-cyclopropyl)-propan-2-ol
1.26 {2-[4-(5-tert-butyl-1-methyl-1H-indol-3-yl)-piperidin-1-yl]-5-oxo-6,7-dihydro-5H-5λ⁴-thieno[3,2-d]pyrimidin-4-yl}-(tetrahydropyran-4-yl)-amine
1.27 2-[4-(5-furan-2-yl-1-methyl-1H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amine
1.28 (S)-5-(2-{4-[4-(4,5-dihydrooxazol-2-yl)-phenoxy]-piperidin-1-yl}-5-oxo-6,7-dihydro-5H-5λ⁴-thieno[3,2-d]pyrimidin-4-ylamino)-1-methylpiperidin-2-one
1.29 {2-[4-(5-furan-2-yl-2-methyl-2H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amine
1.30 {2-[4-(1-methyl-1H-imidazo[4,5-c]pyridin-2-yl)-piperidin-1-yl]-5-oxo-6,7-dihydro-5H-5λ⁴-thieno[3,2-d]pyrimidin-4-yl}-(tetrahydropyran-4-yl)-amine
1.31 2-methoxy-N-{1-[5-oxo-4-(tetrahydropyran-4-ylamino)-6,7-dihydro-5H-5λ⁴-thieno[3,2-d]pyrimidin-2-yl]-4-phenylpiperidin-4-ylmethyl}-acetamide
1.32 N-cyclopropyl-N-methyl-4-{1-[5-oxo-4-(tetrahydropyran-4-ylamino)-6,7-dihydro-5H-5λ⁴-thieno[3,2-d]pyrimidin-2-yl]-piperidin-4-yl}-benzamide
1.33 N-cyclopropyl-N-methyl-4-{1-[5-oxo-4-(tetrahydropyran-4-ylamino)-6,7-dihydro-5H-5λ⁴-thieno[3,2-d]pyrimidin-2-yl]-piperidin-4-yloxy}-benzamide
1.34 {5-oxo-2-[4-(pyridin-4-yloxy)-piperidin-1-yl]-6,7-dihydro-5H-5λ⁴-thieno[3,2-d]pyrimidin-4-yl}-(tetrahydropyran-4-yl)-amine
1.35 {2-[4-(4-chlorophenoxy)-piperidin-1-yl]-5-oxo-6,7-dihydro-5H-5λ⁴-thieno[3,2-d]pyrimidin-4-yl}-(tetrahydropyran-4-yl)-amine
1.36 (S)-1-methyl-5-{2-[4-(5-methyl-4-phenyloxazol-2-yl)-piperidin-1-yl]-5-oxo-6,7-dihydro-5H-5λ⁴-thieno[3,2-d]pyrimidin-4-ylamino}-piperidin-2-one
1.37 (1-{2-[4-(5-methyl-4-phenyloxazol-2-yl)-piperidin-1-yl]-5-oxo-6,7-dihydro-5H-5λ⁴-thieno[3,2-d]pyrimidin-4-ylamino}-cyclopropyl)-methanol
1.38 (S)-5-{2-[4-(4,5-diphenyloxazol-2-yl)-piperidin-1-yl]-5-oxo-6,7-dihydro-5H-5λ⁴-thieno[3,2-d]pyrimidin-4-ylamino}-1-methylpiperidin-2-one
1.39 {4-(4-chlorophenyl)-1-[5-oxo-4-(tetrahydropyran-4-ylamino)-6,7-dihydro-5H-5λ⁴-thieno[3,2-d]pyrimidin-2-yl]-piperidin-4-yl}-methanol
1.40 [1-(2-{4-[5-(4-chlorophenyl)-4-methyloxazol-2-yl]-piperidin-1-yl}-5-oxo-6,7-dihydro-5H-5λ⁴-thieno[3,2-d]pyrimidin-4-ylamino)-cyclopropyl]-methanol
1.41 4-(4-chlorophenyl)-1-[5-oxo-4-(tetrahydropyran-4-ylamino)-6,7-dihydro-5H-5λ⁴-thieno[3,2-d]pyrimidin-2-yl]-piperidin-4-ol
1.42 {2-[4-(4-chlorophenyl)-4-methoxypiperidin-1-yl]-5-oxo-6,7-dihydro-5H-5λ⁴-thieno[3,2-d]pyrimidin-4-yl}-(tetrahydropyran-4-yl)-amine
1.43 4-{1-[4-(1-hydroxymethylcyclopropylamino)-5-oxo-6,7-dihydro-5H-5λ⁴-thieno[3,2-d]pyrimidin-2-yl]-piperidin-4-yloxy}-benzonitrile
1.44 5-oxo-2-[4-(4,5,6,7-tetrahydrobenzoxazol-2-yl)-piperidin-1-yl]-6,7-dihydro-5H-5λ⁴-thieno[3,2-d]pyrimidin-4-yl}-(tetrahydropyran-4-yl)-amine
1.45 (S)-5-{2-[4-(4-chlorophenyl)-piperidin-1-yl]-5,5-dioxo-6,7-dihydro-5H-5λ⁶-thieno[3,2-d]pyrimidin-4-ylamino}-1-methylpiperidin-2-one
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

19. Drug combination according to one of claims 1 to 19, **characterised in that** in addition to a PDE4 inhibitor of general formula **1**, it contains at least one NSAID (**2**) selected from among celecoxib (2.25), etoricoxib (2.41), lumiracoxib (2.74), parecoxib (2.88), rofecoxib (2.101) and valdecoxib (2.125).

20. Drug combination according to one of claims 1 to 19, **characterised in that** the NSAID (**2**) used is either acetylsalicylic acid (2.3) in a single dose of 50 to 2000 mg, meloxicam (2.77) in a single dose of 7.5 mg to 30 mg and naproxen in a single dose of 250 to 1000 mg.

21. Drug combination according to one of claims 1 to 20 for use in the treatment of a disease selected from among respiratory complaints, pulmonary diseases, gastrointestinal ailments and diseases and also inflammatory diseases of the joints, skin or eyes, cancers and diseases of the peripheral or central nervous system.

22. Drug combination according to claim 21 for use in the treatment of a disease selected from COPD, chronic sinusitis, asthma, Crohn's disease, idiopathic pulmonary fibrosis and ulcerative colitis.

## Revendications

1. Combinaison de médicaments, **caractérisée en ce qu'**elle comprend, outre un inhibiteur de PDE4 de formule **1**, 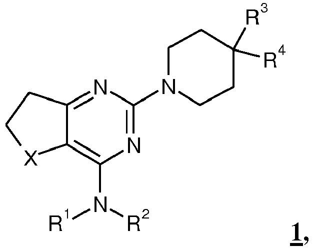 dans laquelle
**X** est SO,
**R¹** est H
**R²** désigne un groupe alkyle en C₁₋₆ qui peut être substitué éventuellement par un ou plusieurs radicaux choisis parmi F, Cl, CF₃, CHF₂ ou CH₂F ou qui peut être substitué éventuellement par un ou plusieurs radicaux choisis dans le groupe consistant en OR^{2.1}, COOR^{2.1}, CONR^{2.2}R^{2.3}, SR^{2.1}, SO-R^{2.1}, SO₂-R^{2.1}, phényle, het, hétaryle, un groupe cycloalkyle en C₃₋₇ monocyclique, CH₂-NR^{2.2}R^{2.3} et NR^{2.2}R^{2.3},
qui peut lui-même être substitué par un ou plusieurs radicaux choisis dans le groupe consistant en OH, F, Cl, Br, CF₃, CHF₂, CH₂F, OR^{2.1}, un groupe oxo, méthyle, éthyle, propyle, isopropyle, méthanol, éthanol, phényle, COOR^{2.1}, CH₂-NR^{2.2}R^{2.3} et NR^{2.2}R^{2.3},
dans laquelle
**het** désigne un hétérocycle monocyclique de trois à sept chaînons, saturé ou partiellement saturé qui contient 1, 2 ou 3 hétéroatomes choisis indépendamment les uns des autres dans le groupe consistant en N, S ou O,
et dans laquelle
**hétaryle** désigne un groupe hétéroaryle aromatique de cinq à sept chaînons, monocyclique, aromatique qui contient 1, 2 ou 3 hétéroatomes choisis indépendamment les uns des autres dans le groupe consistant en N, S ou O,
et dans laquelle
**le groupe cycloalkyle** peut être saturé ou partiellement saturé,
dans laquelle **R^{2.1}** est H ou un radical choisi dans le groupe consistant en les groupes méthyle, éthyle, propyle, isopropyle, méthanol, éthanol, groupe cycloalkyle en C₃₋₇ monocyclique, phényl-alkylène en C₁₋₂, -hétaryl-alkylène en C₁₋₂,
-het-alkylène en C₁₋₂, cycloalkyle en C₃₋₇-alkylène en C₁₋₂, phényle, hétaryle et un groupe het,
qui peut être substitué éventuellement par un ou plusieurs radicaux choisis dans le groupe consistant en OH, F, Cl, méthyle, éthyle, propyle, isopropyle, O-méthyle, O-éthyle, O-propyle, O-isopropyle et phényle,
dans laquelle **R^{2.2}** et **R^{2.3}** sont indépendamment l'un de l'autre H ou un radical choisi dans le groupe consistant en les groupes méthyle, éthyle, propyle, isopropyle, cycloalkyle en C₃₋₇ monocyclique, phényl-alkylène en C₁₋₃, hétaryl-alkylène en C₁₋₃, phényle, -het, -hétaryle, CO-NH₂, CO-NHCH₃, CON(CH₃)₂, SO₂-(C₁₋₂-Alkyl), CO-R^{2.1} et COOR^{2.1},
qui peut être substitué éventuellement par un ou plusieurs radicaux choisis dans le groupe consistant en OH, F, Cl, méthyle, éthyle, propyle, isopropyle, phényle et COOR^{2.1}
ou
**R²** désigne un groupe cycloalkyle en C₃₋₇ monocyclique qui peut être substitué éventuellement par un radical choisi dans le groupe consistant en les groupes alcanol en C₁₋₂,
fluoroalkyle en C₁₋₃, alkylène en C₁₋₃-OR^{2.1}, OR^{2.1}, COOR^{2.1}, SO₂-NR^{2.2}R^{2.3}, -Het, -NH-CO-O-(phényl), méthyle, éthyle, propyle, isopropyle, phényle, phényl-alkylène en C₁₋₂, -hétaryl-alkylène en C₁₋₂, un groupe cycloalkyle en C₃₋₇ monocyclique et NR^{2.2}R^{2.3},
qui peut être substitué éventuellement par un ou plusieurs radicaux choisis dans le groupe consistant en OH, OR^{2.1}, Oxo, F, Cl, CF₃, CHF₂, CH₂F, un groupe méthyle, éthyle, propyle, isopropyle, phényle et NR^{2.2}R^{2.3},
ou
**R²** désigne un groupe phényle qui peut être substitué éventuellement par OH, SH, F, Cl ou Br ou un ou plusieurs radicaux choisis dans le groupe consistant en OR^{2.1}, COOR^{2.1}, NR^{2.2}R^{2.3}, CH₂-NR^{2.2}R^{2.3}, un groupe cycloalkyle en C₃₋₇ monocyclique, -het, méthyle, éthyle, propyle, isopropyle, CF₃, CHF₂, CH₂F, phényl-alkylène en C₁₋₂, het-alkylène en C₁₋₂, hétaryl-alkylène en C₁₋₂, phényle, SO₂-CH₃, SO₂-CH₂CH₃ et SO₂-NR^{2.2}R^{2.3},
qui peut lui-même être substitué éventuellement par un ou plusieurs radicaux choisis dans le groupe consistant en OH, OR^{2.1}, oxo, F, Cl, CF₃, CHF₂, CH₂F, méthyle, éthyle, propyle, isopropyle, phényle et NR^{2.2}R^{2.3},
ou
**R²** désigne un radical choisi dans un groupe consistant en het et hétaryle, qui peut être substitué éventuellement par un ou plusieurs radicaux choisis dans le groupe consistant en F, Cl, OH, Oxo, CF₃, CHF₂ et CH₂F ou par un ou plusieurs radicaux choisis dans le groupe consistant en OR^{2.1}, alkylène en C₁₋₃-OR^{2.1}, SR^{2.1},SO-R^{2.1}, SO₂-R^{2.1}, COOR^{2.1}, COR^{2.1}, méthanol, éthanol, cycloalkyle en C₃₋₇ monocyclique, phényle, méthyle, éthyle, propyle, isopropyle, phényl-alkylène en C₁₋₂, hétaryl-alkylène en C₁₋₂, -het, -hétaryle et NR^{2.2}R^{2.3},
qui peut lui-même être substitué par un ou plusieurs radicaux choisis dans le groupe consistant en OH, OR^{2.1}, oxo, F, Cl, CF₃, CHF₂, CH₂F, en groupes méthyle, éthyle, propyle, isopropyle, butyle, isobutyle, phényle et NR^{2.2}R^{2.3},
et dans laquelle
**R³** désigne un groupe naphtaline ou phényle,
qui peut être substitué éventuellement en position ortho, para ou méta par un ou deux radicaux choisis indépendamment les uns des autres dans le groupe consistant en les atomes de fluor, de chlore, de brome, un groupe hydroxy, CN, méthyle, éthyle, propyle, isopropyle, cyclopropyle, CF₃, CHF₂, CH₂F, -OCH₃, OCH₂CH₃ ; SO₂-CH₃, SO-CH₃, COOCH₃, COOCH₂CH₃, -CO-NH-(méthylène)-hétaryle, -CO-NH-(éthylène)-hétaryle, -CO-NH-hétaryle, -CO-N(CH₃)-het, -CO-N(CH₃)-(méthylène)-het, -CO-N(CH₃)-(éthylène)-het, -CO-N(CH₃)-(méthylène)-hétaryle, -CO-N(CH₃)-(éthylène)-hétaryle, -CO-N(cyclopropyl)-het, CO-NH₂, CONH(CH₃), CON(CH₃)₂, -CO-NH-(méthylène)-het, -CO-NH-(éthylène)-het,
-NH-CO-méthyle, NCH₃-CO-méthyle, -NH-CO-éthyle, NCH₃-CO-éthyle, -NH-CO-propyle, NCH₃-CO-propyle, -NH-CO-isopropyle, NCH₃-CO-isopropyle, phényle, phényl-méthylène, phényl-éthylène, het-méthylène, het-éthylène, -het, -CO-het, -CO-N(CH₃)-het, CO-N(CH₃)-cyclopropyle, C₃₋₇-cycloalkyle, C₃₋₇-cycloalkyl-méthylène, cycloalkyle en C₃₋₇-éthylène, hétaryl-méthylène, hétaryl-éthylène, -hétaryle, CH₂-NH₂, CH₂-NH(CH₃), CH₂-N(CH₃)₂, -NH₂, -NH(CH₃) et -N(CH₃)₂,
dans laquelle ce radical peut être substitué éventuellement par un ou plusieurs radicaux choisis dans le groupe consistant en OH, F, Cl, -CF₃, CHF₂, CH₂F, oxo, méthyle et phényle
ou dans laquelle
**R³** désigne un radical choisi dans le groupe consistant en un groupe het et hétaryle qui peut être substitué éventuellement par un ou plusieurs radicaux choisis dans le groupe consistant en F, Cl, Br, CF₃, CHF₂, CH₂F, CN, OH, oxo, méthyle, éthyle, propyle, isopropyle, butyle, isobutyle, cyclopropyle, -O-méthyle, -O-éthyle, -O-propyle, -O-isopropyle, -COO-méthyle, -COO-éthyle, -COO-propyle, -COO-isopropyle, SO-(CH₃), SO-(CH₂-CH₃), SO₂-(CH₃), SO₂-(CH₂-CH₃), phényle, CH₂-NH₂, CH₂-NH(CH₃), CH₂-N(CH₃)₂, -NH₂, -NH(CH₃), -N(CH₃)₂, het et hétaryle,
qui peut lui-même être substitué éventuellement par un ou plusieurs radicaux choisis dans le groupe consistant en OH, F, Cl, CF₃, CHF₂, CH₂F, méthyle, éthyle, propyle, isopropyle, phényle, -COO-méthyle, -COO-éthyle et O-méthyle, O-éthyle,
ou dans laquelle
**R**³ dans laquelle est -O₋R^{3.1},
**R^{3.1}** désigne un radical choisi dans le groupe consistant en groupes alkyle en -C₁₋₃, -phényle, -alkylène en C₁₋₃-phényle, hétaryle et het,
qui peut être substitué éventuellement en position ortho, para ou méta par deux ou trois radicaux choisis indépendamment les uns des autres dans le groupe consistant en les atomes de fluor, de chlore, de brome, en les groupes hydroxy, CN, méthyle, éthyle, propyle, isopropyle, butyle, isobutyle, CF₃, CHF₂, CH₂F, CO-(méthyle), CO-(éthyl), CO-(propyle), CO-(isopropyle), -CO-(CF₃), -CO-NH-(méthylène)-hétaryle, -CO-NH-(éthylène)-hétaryle,
-CO-N(CH₃)-(méthylène)-hétaryle, -CO-N(CH₃)-(éthylène)-hétaryle, -CO-N(CH₃)-(propylène)-hétaryle, -CO-N(CH₃)-(isopropylène)-hétaryle -CO-N(CH₃)-het, -CO-N(cyclopropyl)-het, -CO-N(cycloalkyle en C₅₋₇₎-het, -méthylène-O-méthyle, -éthylène-O-méthyle, -propylène-O-méthyle, -méthylène-O-éthyle, -éthylène-O-éthyle, -propylène-O-éthyl, -méthylène-NH₂, -méthylène-NHCH₃, -méthylène-N(CH₃)₂, -éthylène-NH₂, -éthylène-NHCH₃, -éthylène-N(CH₃)₂, NH₂, N(CH₃)₂, NHCH₃, -O-méthyle, O-éthyle, O-propyle, O-isopropyle, O-butyle, O-isobutyle, -SO-CH₃, SO-éthyle,-SO-propyle,
-SO-isopropyle, SO₂-méthyle, -SO₂-éthyle, SO₂-propyle, SO₂-isopropyle, COOH, COO-(méthyl), COO-(éthyle), COO-(propyle), COO-(isopropyle), -O-méthylène-N(méthyl)₂, -O-éthylène-N(méthyl)₂, -O-méthylène-N(éthyl)₂, -O-éthylène-N(éthyl)₂, CO-NH₂, CO-NH(CH₃), CO-N(CH₃)₂, -NH-CO-méthyle, -NCH₃-CO-méthyle, -NH-CO-éthyle, NCH₃-CO-éthyle, phényle, phényl-méthylène-, phényl-éthylène-, het-méthylène-, het-éthylène-, -CO-het, het, -CO-cycloalkyle en C₅-₇, -CO-cyclopropyle, -CO-N(CH₃)-cycloalkyle en C₅-₇, -CO-N(CH₃)-cyclopropyle,
cycloalkyle en C₅-₇, cyclopropyle, cycloalkyle en C₅₋₇-méthylène, cycloalkyle en C₅₋₇-éthylène, cyclopropyl-méthylène, cyclopropyl-éthylène, hétaryl-méthylène, hétaryl-éthylène et hétaryle,
qui peut lui-même être substitué éventuellement par 1, 2, 3 ou 4 radicaux choisis indépendamment les uns des autres dans le groupe consistant en F, CI, Br, Méthyl, O-méthyle, éthyle, O-éthyle, OH, oxo et CF₃,
et dans laquelle
**R⁴** désigne H, CN, OH, CF₃, CHF₂, CH₂F, F, méthyle, éthyle, O-méthyle ou O-éthyle,-méthylène-OH, -éthylène-OH, -propylène-OH, isopropylène-OH, -COO(méthyle), -COO(éthyle), -COO(propyle), -COO(isopropyle), -CO-het, -(méthylène)-NH-SO₂-(méthyle), -(méthylène)-NH-SO₂-(éthyle), -(éthylène)-NH-SO₂-(méthyl), -(éthylène)-NH-SO₂-(éthyle), -(méthylène)-N(CH3₎-SO2₋(méthyl), -(méthylène)-N(CH₃)-SO₂-(éthyle), -(éthylène)-N(CH₃)-SO₂-(méthyle), -(éthylène)-N(CH₃)-SO₂-(éthyle), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle -éthylène-O-éthyle, -(méthylène)-N(CH₃)-CO-(méthyl), -(méthylène)-N(CH₃)-CO-(éthyl) -(éthylène)-N(CH₃)-CO-(méthyl), -(éthylène)-N(CH₃)-CO-(éthyl), -NH-CO-(méthylène)-O-(méthyl), -NH-CO-(méthylène)-O-(éthyl), -NH-CO-(éthylène)-O-(méthyl), -NH-CO-(éthylène)-O-(éthyl), -méthylène-NH-CO-(méthyl), -méthylène-NH-CO-(éthyl), -éthylène-NH-CO-(méthyl), -éthylène-NH-CO-(éthyl), -méthylène-NH-CO-(méthylène)-N(méthyl)₂, -méthylène-NH-CO-(éthylène)-N(méthyl)₂, -éthylène-NH-CO-(méthylène)-N(méthyl)₂, -éthylène-NH-CO-(éthylène)-N(méthyl)₂, -méthylène-NH-CO-(méthylène)-O-(méthyl), -méthylène-NH-CO-(éthylène)-O-(méthyl), -éthylène-NH-CO-(méthylène)-O-(méthyl), -méthylène-NH-CO-(méthylène)-O-(éthyl), -méthylène-NH-CO-(éthylène)-O-(éthyl), -Éthylène-NH-CO-(méthylène)-O-(éthyl), -(méthylène)-N(CH3₎-CO-(méthylène)-O-(méthyl), -(méthylène)-N(CH3₎-CO-(éthylène)-O-(méthyl), -(éthylène)-N(CH₃)-CO-(méthylène)-O-(méthyl), -(méthylène)-N(CH3₎-CO-(méthylène)-O-(éthyl), -(méthylène)-N(CH3)-CO-(Éthylène)-O-(Éthyl), -(éthylène)-N(CH₃)-CO-(méthylène)-O-(éthyl), -O-(méthylène)-phényl, -O-(éthylène)-phényle,
-CO-phényle,
dans laquelle le groupe phényle dans les radicaux ci-dessus peut être substitué éventuellement par un ou plusieurs autres radicaux choisis dans le groupe consistant en F, Cl, Br, méthyle, éthyle, propyle, -O-méthyle, -O-éthyle, -O-propyle, -OH et CF₃
ou dans laquelle
**R**³ et **R⁴** forment conjointement un hétérocycle mono- ou bicyclique insaturé ou saturé ou partiellement saturé qui contient 1, 2 ou 3 hétéroatomes choisis dans le groupe consistant en N, O et S et qui peut être substitué éventuellement par un ou plusieurs radicaux choisis dans le groupe consistant en F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, phényle, cycloalkyle en C₅-₇, het et hétaryle,
au moins un AINS (= anti-inflammatoire non stéroïdien) (2) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

2. Combinaison de médicaments selon la revendication 1, **caractérisée en ce qu'**elle comprend, outre un inhibiteur de PDE4 de formule **1,** dans laquelle
**R²** est un radical de formule **3** 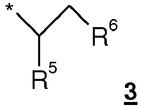 dans laquelle
**R⁶** est OH ou NH₂ et
dans laquelle
**R⁵** désigne un radical choisi dans le groupe consistant en un groupe alkyle en C₁₋₄, un groupe hétéroaryle de cinq à six chaînons comportant 1, 2 ou 3 hétéroatomes choisis dans le groupe consistant en S, O et N et un groupe phényle, qui peut être substitué éventuellement par un ou plusieurs radicaux choisis dans le groupe consistant en OH, F, Br, OR^{2.1}, oxo, méthyle, éthyle, méthanol, éthanol, phényle, COOR^{2.1}_{,} CH₂-NR^{2.2}R^{2.3} et NR^{2.2}R^{2.3},
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

3. Combinaison de médicaments selon la revendication 2, **caractérisée** en ce en ce qu'elle comprend, outre un inhibiteur de PDE4 de formule **1**, dans laquelle
**R²** est un radical de formule **3** 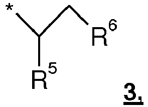 dans laquelle
**R⁶** est OH ou NH₂ et
dans laquelle
**R⁵** est un groupe méthyle, éthyle, propyle, isopropyle
au moins un AINS **(2)** choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

4. Combinaison de médicaments selon la revendication 1, **caractérisée** en ce en ce qu'elle comprend, outre un inhibiteur de PDE4 de formule **1**, dans laquelle
**R²** est un cycle cycloalkyle monocyclique de trois, quatre, cinq, six ou sept chaînons qui peut être substitué éventuellement en position spiro par un radical choisi dans le groupe consistant en -CH₂-OR^{2.1}, alkylène en C₂₋₆-OR^{2.1} ramifié ou non ramifié, méthyle, éthyle, propyle, isopropyle, butyle, isobutyle, cyclopropyle, -CF₃, CHF₂, CH₂F et fluoroalkyle en C₂₋₄, dans laquelle
R^{2.1} est choisi dans le groupe consistant en les groupes méthyle, éthyle, propyle, isopropyle, butyle, isobutyle,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

5. Combinaison de médicaments selon la revendication 1, **caractérisée en ce qu'**elle comprend, outre un inhibiteur de PDE4 de formule **1**, dans laquelle
**R²** est un groupe cyclopropyle qui peut être substitué éventuellement par un autre radical choisi dans le groupe consistant en -NH₂, CH2- NH₂, -NH(CH₃), -N(CH₃)₂, en groupes méthyle, éthyle, propyle, isopropyle, -NH-CO-(tert-butyl), -NH-CO-O-(tert-butyl),
-N(CH₃)-CO-(tert-butyl), -N(CH₃)-CO-O-(tert-butyl), -CF₃, -CHF₂, CH₂F, F, Cl et Br
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

6. Combinaison de médicaments selon la revendication 1, **caractérisée en ce qu'**elle comprend, outre un inhibiteur de PDE4 de formule **1**, dans laquelle
**R²** est un groupe cyclopropyle
**R²** est un groupe phényle qui peut être substitué éventuellement dans une ou les deux positions méta par un ou plusieurs radicaux choisis dans le groupe consistant en les groupes méthyle, éthyle, propyle, isopropyle, cyclopropyle, F, Cl, Br, OH, OR^{2.1}, COOR^{2.1}, CF₃, CHF₂, CH₂F, NH₂, NH(CH₃) et N(CH₃)₂, dans laquelle R^{2.1} peut être H, méthyle ou éthyle,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

7. Combinaison de médicaments selon la revendication 1, **caractérisée en ce qu'**elle comprend, outre un inhibiteur de PDE4 de formule **1**
dans laquelle
R² est un radical choisi dans un groupe consistant en un hétérocycle monocyclique saturé à six chaînons comportant un hétéroatome choisi dans le groupe consistant en N, O et S qui peut être substitué éventuellement par un ou plusieurs radicaux choisis dans le groupe consistant en F, Cl, Br, CF₃, CHF₂, CH₂F, OH, Oxo, NH₂, NHCH₃ et N(CH₃)₂, en groupes méthyle, éthyle, propyle, isopropyle, cyclopropyle, méthoxy et éthoxy,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

8. Combinaison de médicaments selon la revendication 7, **caractérisée en ce qu'**elle comprend, outre un inhibiteur de PDE4 de formule **1**, dans laquelle
**R²** est un radical choisi dans un groupe consistant en une pipéridine ou un tétrahydropyrane qui peut être substitué éventuellement par un ou plusieurs radicaux choisis dans le groupe consistant en F, Cl, Br, OH, CF₃, CHF₂, CH₂F, NH₂, NHCH₃, N(CH₃)₂, oxo, méthyle et méthoxy,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

9. Combinaison de médicaments selon l'une des revendications 1 à 8, **caractérisée en ce qu'**elle comprend, outre un inhibiteur de PDE4 de formule **1,** dans laquelle
**R³** est un groupe naphtaline ou phényle,
qui peut être substitué en une position quelconque par un, deux ou trois radicaux choisis indépendamment les uns des autres dans le groupe consistant en les atomes de fluor, de chlore, de brome, un groupe hydroxy, CN, méthyle, éthyle, propyle, isopropyle, cyclopropyle, CF₃, CHF₂, CH₂F, -OCH₃, OCH₂CH₃ ; SO₂-CH₃, SO₂-CH₂CH₃, COOCH₃ et CO-O-CH₂CH₃,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

10. Combinaison de médicaments selon l'une des revendications 1 à 8, **caractérisée en ce qu'**elle comprend, outre un inhibiteur de PDE4 de formule **1,** dans laquelle
**R³** est un radical choisi dans le groupe consistant en het et hétaryle, qui peut être substitué éventuellement par un ou plusieurs radicaux choisis dans le groupe consistant en F, Cl, Br, CF₃, CHF₂, CH₂F, CN, OH, oxo, méthyle, éthyle, propyle, isopropyle, butyle, isobutyle, cyclopropyle, cycloalkyle en C₅-₇, -O-méthyle, -O-éthyle, -O-propyle, -O-isopropyle, -COO-méthyle, -COO-éthyle, -COO-propyle, -COO-isopropyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, het et hétaryle qui peut lui-même est substitué par un ou plusieurs radicaux choisis dans le groupe consistant en OH, F, Cl, Br, CF₃, CHF₂, CH₂F, méthyle, éthyle, propyle, isopropyle, phényle, -COO-méthyle, -COO-éthyle, -COO-propyle, -COO-isopropyle et O-méthyle, O-éthyle, O-propyle et O-isopropyle,
et dans laquelle
**R⁴** est H, CN, OH, CF₃, CHF₂, CH₂F, F, un groupe méthyle, éthyle, O-méthyle ou O-éthyle,
dans laquelle
**het** désigne un hétérocycle monocyclique de trois à sept chaînons, saturé ou partiellement saturé ou un hétérocycle de sept à onze chaînons, bicyclique, annelé, saturé ou partiellement saturé qui contient 1, 2 ou 3 hétéroatomes choisis indépendamment les uns des autres dans le groupe consistant en N, S ou O
et dans laquelle
**hétaryle** désigne un groupe hétéroaryle aromatique de cinq à sept chaînons, monocyclique, ou un groupe hétéraryle de sept à onze chaînons, bicyclique, annelé, aromatique qui contient respectivement 1, 2 ou 3 hétéroatomes choisis indépendamment les uns des autres dans le groupe consistant en N, S ou O
et dans laquelle
**le groupe cycloalkyle** peut être saturé ou partiellement saturé
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

11. Combinaison de médicaments selon la revendication 10, **caractérisée en ce qu'**elle comprend, outre un inhibiteur de PDE4 de formule 1, dans laquelle
**R³** est un radical choisi parmi un hétérocycle bicyclique, de sept à onze chaînons, saturé ou partiellement saturé ou un groupe hétéroaryle bicyclique, de sept à onze chaînons choisi dans le groupe consistant en indole, dihydro-indole, quinazoline, dihydroquinazoline, tétrahydroquinazoline, benzo-isoxazole, dihydrobenzo-isoxazole, benzo-oxazine, dihydrobenzo-oxazine, benzothiazole, dihydrobenzothiazole, triazolopyridine, dihydrotriazolopyridine, benzofurane, dihydrobenzofurane, isobenzofurane et dihydro-isobenzofurane,
qui peut être substitué éventuellement par un ou plusieurs radicaux choisis dans le groupe consistant en F, Cl, Br, CF₃, CHF₂, CH₂F, CN, OH, en groupes oxo, méthyle, éthyle, propyle, isopropyle, butyle, isobutyle, cyclopropyle, -O-méthyle, -O-éthyle, -O-propyle, -O-isopropyle, -COO-méthyle, -COO-éthyle, -COO-propyle, -COO-isopropyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, furanyle et pyridinyle,
qui peut lui-même être substitué éventuellement par un ou plusieurs radicaux choisis dans le groupe OH, F, Cl, Br, CF₃, CHF₂, CH₂F, méthyle, éthyle, propyle, isopropyle, phényle, -COO-méthyle, -COO-éthyle et O-méthyle, O-éthyle,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

12. Combinaison de médicaments selon l'une des revendications 1 à 8, **caractérisée en ce qu'**elle comprend, outre un inhibiteur de PDE4 de formule **1,** dans laquelle
**R³** est un radical choisi parmi un hétérocycle monocyclique saturé ou partiellement saturé, de trois à sept chaînons ou un groupe hétéroaryle monocyclique de cinq à six chaînons
qui est choisi dans le groupe consistant en imidazole, dihydro-imidazole, oxadiazole, oxadiazolidine, pyrazole, pyridine et dihydropyrazole,
qui peut être substitué éventuellement par un ou plusieurs radicaux choisis dans le groupe consistant en F, Cl, Br, CF₃, CHF₂, CH₂F, CN, OH, oxo, méthyle, éthyle, propyle, isopropyle, butyle, isobutyle, cyclopropyle, --O-méthyle, -O-éthyle, -O-propyle, -O-isopropyle, -COO-méthyle, -COO-éthyle, -COO-propyle, -COO-isopropyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, furanyle et pyridinyle qui peut lui-même être substitué par un ou plusieurs radicaux choisis dans le groupe consistant en OH, F, Cl, Br, CF₃, CHF₂, CH₂F, méthyle, éthyle, propyle, isopropyle, phényle,-COO-méthyle, -COO-éthyle et O-méthyle, O-éthyle,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

13. Combinaison de médicaments selon l'une des revendications 1 à 8, **caractérisée en ce qu'**elle comprend, outre un ou plusieurs, de préférence un inhibiteur(s) de PDE4 de formule **1**, dans laquelle
**R³** et **R⁴** forment conjointement un hétérocycle mono- ou bicyclique, insaturé ou partiellement saturé, de trois à onze chaînons qui contient 1, 2 ou 3 hétéroatomes choisis dans le groupe consistant en N, O et S et qui peut être substitué éventuellement par un ou plusieurs radicaux choisis dans le groupe consistant en F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂-CH₃), SO-(CH₃), SO-(CH₂-CH₃), phényle, -CH₂-NH₂, -CH₂NHCH₃, -CH₂-N(CH₃)₂, NH₂, NHCH₃, N(CH₃)₂, un hétérocycle saturé ou partiellement saturé de cinq à six chaînons,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

14. Combinaison de médicaments selon l'une des revendications 1 à 8, **caractérisée en ce qu'**elle comprend, outre un inhibiteur de PDE4 de formule **1,** dans laquelle
**R³** et **R⁴** forment conjointement, un hétérocycle bicyclique choisi dans le groupe consistant en la tétrahydroquinazoline, tétrahydrobenzoxazine et dihydro-indole, dihydro-isobenzofurane, qui peut être substitué éventuellement par un ou plusieurs radicaux choisis dans le groupe consistant en F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂-CH₃), phényle, -CH₂-NH₂, -CH₂NHCH₃, -CH₂-N(CH₃)₂, NH₂, NHCH₃, N(CH₃)₂, un hétérocycle saturé ou partiellement saturé de cinq ou six chaînons et un groupe hétéroaryle de cinq ou six chaînons,
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

15. Combinaison de médicaments selon l'une des revendications 1 à 8, **caractérisée en ce qu'**elle comprend, outre un inhibiteur de PDE4 de formule **1**,
dans laquelle
**R**³ est -O₋R^{3.1},
**R^{3.1}** est un radical choisi dans le groupe consistant en les groupes méthyle, éthyle, propyle, isopropyle, butyle, isobutyle, pentyle, isopentyle, -phényle, -méthylène-phényle,
-éthylène-phényle, -propylène-phényle, -isopropylène-phényle, hétaryle et het,
qui peut être substitué éventuellement en position ortho, para ou méta par un, deux ou trois radicaux choisis indépendamment les uns des autres dans le groupe consistant en les atomes de fluor, de chlore, de brome, en les groupes hydroxy, CN, méthyle, éthyle, propyle, isopropyle, butyle, isobutyle, -CF₃, CHF₂, CH₂F, CO-(méthyl), CO-(éthyl),
CO-(propyl), CO-(isopropyl), CO-(butyl), CO-(isobutyl), -CO-(CF₃), -CO-(CH₂F), -CO-(CHF₂), -CO-NH-(méthylène)-hétaryle,
-CO-NH-(éthylène)-hétaryle, -CO-NH-(propylène)-hétaryle, -CO-NH-(isopropylène)-hétaryle, -CO-N(CH₃)-(méthylène)-hétaryle, -CO-N(CH₃)-(éthylène)-hétaryle, -CO-N(CH₃)-(propylène)-hétaryle, -CO-N(CH₃)-(isopropylène)-hétaryle, -CO-N(CH₃)-Het, -CO-N(cycloalkyle en C₃-₇)-het, -méthylène-O-méthyle, -éthylène-O-méthyle, -méthylène-O-éthyle, -éthylène-O-éthyle, -méthylène-NH₂, -éthylène-NH₂, -méthylène-NHCH₃, -éthylène-NHCH₃, -méthylène-N(CH₃)₂, -éthylène-N(CH₃)_{2,} -NH₂, -NHCH₃, -N(CH₃)₂, -O-méthyle, -O-éthyle, -O-propyle, -O-isopropyle, -SO-CH₃, -SO-(CH₂CH₃), -SO₂-CH₃, -SO₂-(CH₂CH₃), COOH, COO-(méthyl), COO-(éthyl), COO-(propyl), COO-(isopropyl), -O-méthylène-N(méthyl)2, -O-éthylène-N(méthyl)₂,-O-méthylène-N(éthyl)₂, -O-éthylène-N(éthyl)₂, CO-NH₂, CO-NHCH₃, CO-N(CH₃)₂, NH-CO-méthyle, NCH₃-CO-méthyle, NH-CO-éthyle, N(CH₃)-CO-éthyle, phényle, phényl-méthylène-, phényl-éthylène-, het-méthylène-, het-éthylène-, -CO-het, het, -CO-cycloalkyle en C₄₋₇, -CO-cyclopropyle, -CO-N(CH₃)-cyclopropyle, -CO-N(CH₃)-cycloalkyle en C₄₋₇, cycloalkyle en C₄₋₇, cyclopropyle, cycloalkyle en C₄₋₇-méthylène-, cyclopropyl-méthylène-, cycloalkyle en C₄-₇-éthylène-, cyclopropyl-éthylène-, hétaryl-méthylène-, hétaryl-éthylène- et hétaryle,
qui peut lui-même être substitué éventuellement par 1, 2, 3 ou 4 radicaux choisis indépendamment les uns des autres dans le groupe consistant en F, Cl, Br, méthyle, O-méthyle, éthyle, O-éthyle, OH, oxo et CF₃,
au moins un AINS (**2)** choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

16. Combinaison de médicaments selon l'une des revendications 1 à 8, **caractérisée en ce qu'**elle comprend, outre un ou plusieurs, de préférence un inhibiteur(s) de PDE4 de formule **1,** dans laquelle
**R**⁴ est H, CN, OH, CF₃, CHF₂, CH₂F, F, méthyle, éthyle, O-méthyle ou O-éthyle,-méthylène-OH, -éthylène-OH, -propylène-OH, isopropylène-OH, -COO(méthyl), -COO(éthyl), -COO(propyl), -COO(isopropyl), -CO-het, -(méthylène)-NH-SO₂-(méthyl), -(méthylène)-NH-SO₂-(éthyl), -(éthylène)-NH-SO2-(m_{é}thyl), -(éthylène)-NH-SO2-₍éthyl), -(méthylène)-N(CH₃)-SO₂-(méthyl), -(méthylène)-N(CH₃)-SO₂-(éthyl), -(éthylène)-N(CH₃)-SO₂-(méthyl), -(éthylène)-N(CH₃)-SO₂-(éthyl), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle -éthylène-O-éthyle, -(méthylène)-N(CH₃)-CO-(méthyl), -(méthylène)-N(CH₃)-CO-(éthyl) -(éthylène)-N(CH₃)-CO-(méthyl), -(éthylène)-N(CH₃)-CO-(éthyl), -NH-CO-(méthylène)-O-(méthyl), -NH-CO-(méthylène)-O-(éthyl), -NH-CO-(éthylène)-O-(méthyl), -NH-CO-(éthylène)-O-(éthyl), -méthylène-NH-CO-(méthyl), -méthylène-NH-CO-(éthyl), -éthylène-NH-CO-(méthyl), -éthylène-NH-CO-(éthyl), -méthylène-NH-CO-(méthylène)-N(méthyl)₂, -méthylène-NH-CO-(éthylène)-N(méthyl)₂, -éthylène-NH-CO-(méthylène)-N(méthyl)₂, -éthylène-NH-CO-(éthylène)-N(méthyl)2, -méthylène-NH-CO-(méthylène)-O-(méthyl), -méthylène-NH-CO-(éthylène)-O-(méthyl), -éthylène-NH-CO-(méthylène)-O-(méthyl), -méthylène-NH-CO-(méthylène)-O-(éthyl), -méthylène-NH-CO-(éthylène)-O-(éthyl), -éthylène-NH-CO-(méthylène)-O-(éthyl), -(méthylène)-N(CH3₎-CO-(méthylène)-O-(méthyl), -(méthylène)-N(CH3₎-CO-(éthylène)-O-(méthyl), -(éthylène)-N(CH₃)-CO-(méthylène)-O-(méthyl), -(méthylène)-N(CH3₎-CO-(méthylène)-O-(éthyl), -(méthylène)-N(CH3₎-CO-(éthylène)-O-(éthyl), -(éthylène)-N(CH₃)-CO-(méthylène)-O-(éthyl), -O-(méthylène)-phényle, -O-(éthylène)-phényle,
-CO-phényle,
dans laquelle le groupe phényle dans les radicaux ci-dessus peut être substitué éventuellement par un ou plusieurs autres radicaux choisis dans le groupe consistant en F, Cl, Br, méthyle, éthyle, propyle, -O-méthyle, -O-éthyle, -O-propyle, -OH et CF₃,
au moins un AINS (2) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

17. Combinaison de médicaments selon l'une des revendications 1 à 8, **caractérisée en ce qu'**elle comprend, outre un ou plusieurs, de préférence un inhibiteur(s) de PDE4 de formule **1**,
dans laquelle
**R³** est un radical choisi dans le groupe consistant en les groupes oxazole, imidazole et thiazole, dans laquelle ce radical peut être substitué éventuellement par un, deux ou trois autres radicaux choisis indépendamment dans le groupe consistant en méthyle, éthyle, propyle, isopropyle, O-méthyle, O-éthyle, O-propyle, O-isopropyle, OH, F, Cl, Br, CF₃, phényle, hétaryle et cycloalkyle en C₃₋₆,
au moins un AINS (**2)** choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

18. Combinaison de médicaments selon la revendication 11, **caractérisée en ce qu'**elle comprend, outre un ou plusieurs, de préférence un inhibiteur(s) de PDE4 de formule **1,** choisis dans le groupe consistant en :
1.1 (R)-2-{2-[4-(4-chlorophényl)-pipéridin-1-yl]-5-oxo-6,7-dihydro-5*H*-5λ4-thiéno[3,2-*d*]pyrimidin -4-ylamino}-3-méthylbutan-1-ol
1.2 (1-{2-[4-(4-chlorophényl)-pipéridin-1-yl]-5-oxo-6,7-dihydro-5H-5λ⁴-thiéno[3,2-*d*]pyrimidin-4-ylamino}-cyclopropyl)-méthanol
1.3 (R)-2-{2-[4-(4-chlorophényl)-pipéridin-1-yl]-5-oxo-6,7-dihydro-5*H*-5λ⁴-thiéno[3,2-*d*]pyrimidin-4-ylamino}-pentan-1-ol
1.4 (R)-1-{2-[4-(4-chlorophényl)-pipéridin-1-yl]-5-oxo-6,7-dihydro-5*H*-5λ⁴-thiéno[3,2-*d*]pyrimidin-4-ylamino}-1-(4-fluorophényl)-2-méthylpropan-2-ol
1.5 (S)-5-{2-[4-(4-chlorophényl)-pipéridin-1-yl]-5-oxo-6,7-dihydro-5*H*-5λ⁴-thiéno[3,2-*d*]pyrimidin-4-ylamino}-1-méthylpipéridin-2-one
1.6 {2-[4-(4-chlorophényl)-pipéridin-1-yl]-5-oxo-6,7-dihydro-5*H*-5λ⁴-thiéno[3,2-d]pyrimidin-4-yl}-(tétrahydropyran-4-yl)-amine
1.7 1-(4-(1-hydroxyméthylcyclopropylamino)-5-oxo-6,7-dihydro-5*H*-5λ⁴-thiéno[3,2-*d*]pyrimidin-2-yl)-3'-méthyl-1'*H*-spiro[pipéridin-4,4'-quinazolin]-2'(3'*H*)-one
1.8 {1-[2-(4-benzo[*d*]isoxazol-3-yl-pipéridin-1-yl)-5-oxo-6,7-dihydro-5*H*-5λ⁴-thiéno[3,2-*d*]pyrimidin-4-ylamino]-cyclopropyl}-méthanol
1.9 (1-{2-[4-(2-éthyl-5-fluoro-1 *H*-indol-3-yl)-pipéridin-1-yl]-5-oxo-6,7-dihydro-5*H*-5λ-thiéno[3,2-*d*]pyrimidin-4-ylamino}-cyclopropyl)-méthanol
1.10 1-[4-((S)-1-méthyl-6-oxopipéridin-3-ylamino)-5-oxo-6,7-dihydro-5*H*-5λ⁴-thiéno[3,2-*d*]pyrimidin-2-yl]-4-phénylpipéridin-4-carbonitrile
1.11 3'-méthyl-1-(4-(tétrahydro-2*H*-pyran-4-ylamino)-5-oxo-6,7-dihydro-5H-5λ⁴-thiéno[3,2-d]pyrimidin-2-yl)-1'*H*-spiro[pipéridin-4,4'-quinazolin]-2'(3'*H*)-one
1.12 (3-fluorophényl)-[5-oxo-2-(3,4,5,6-tétrahydro-2H-[4,4']bipyridinyl-1-yl)-6,7-dihydro-5*H-*5λ⁴-thiéno[3,2-*d*]pyrimidin-4-yl]-amine
1.13 {2-[4-(2-éthyl-5-fluoro-1*H*-indol-3-yl)-pipéridin-1-yl]-5-oxo-6,7-dihydro-5H-5λ⁴-thiéno[3,2-d]pyrimidin-4-yl}-(3-fluorophényl)-amine
1.14 (1-{2-[4-(2,4-difluorophényl)-pipéridin-1-yl]-5-oxo-6,7-dihydro-5*H*-5λ⁴-thiéno[3,2-*d*]pyrimidin-4-ylamino}-cyclopropyl)-méthanol
1.15 {2-[4-(2,4-difluorophényl)-pipéridin-1-yl]-5-oxo-6,7-dihydro-5*H*-5λ⁴-thiéno[3,2-*d*]pyrimidin-4-yl}-(tétrahydropyran-4-yl)-amine
1.16 (S)-5-[2-(4-benzoxazol-2-yl-pipéridin-1-yl)-5-oxo-6,7-dihydro-5*H*-5λ⁴-thiéno[3,2-*d*]pyrimidin-4-ylamino]-1-méthylpipéridin-2-one
1.17 (1-{2-[4-(6-fluorobenzo[*d*]isoxazol-3-yl)-pipéridin-1-yl]-5-oxo-6,7-dihydro-5*H*-5λ⁴-thiéno[3,2-*d*]pyrimidin-4-ylaminol-cyclopropyl)-méthanol
1.18 (1-{2-[4-(5-fluorobenzo[d]isoxazol-3-yl)-pipéridin-1-yl]-5-oxo-6,7-dihydro-5H-5λ⁴-thiéno[3,2-d]pyrimidin-4-ylamino}-cyclopropyl)-méthanol
1.19 {2-[4-(5-furan-2-yl-2H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.20 (3-fluorophényl)-{5-oxo-2-[4-(3-pyridin-4-yl-[1,2,4]oxadiazol-5-yl)-pipéridin-1-yl]-6,7-dihydro-5H-5λ⁴-thiéno[3,2-d]pyrimidin-4-yl}-amine
1.21 (R)-3-méthyl-2-{5-oxo-2-[4-(3-pyridin-4-yl-[1,2,4]oxadiazol-5-yl)-pipéridin-1-yl]-6,7-dihydro-5H-5λ4-thiéno[3,2-d]pyrimidin-4-ylaminol-butan-1-ol
1.22 (S)-5-{2-[4-(4-fluorophénoxy)-pipéridin-1-yl]-5-oxo-6,7-dihydro-5H-5λ⁴-thiéno[3,2-d]pyrimidin-4-ylamino}-1-méthylpipéridin-2-one
1.23 (2-{4-[4-(4,5-dihydrooxazol-2-yl)-phénoxy]-pipéridin-1-yl}-5-oxo-6,7-dihydro-5H-5λ⁴-thiéno[3,2-d]pyrimidin-4-yl)-(tétrahydropyran-4-yl)-amine
1.24 acide 4-{1-[5-oxo-4-(tétrahydropyran-4-ylamino)-6,7-dihydro-5H-5λ⁴-thiéno[3,2-d]pyrimidin-2-yl]-pipéridin-4-yloxy}-benzoïque
1.25 2-(1 -{2-[4-(4-chlorophényl)-pipéridin-1-yl]-5-oxo-6,7-dihydro-5H-5λ⁴-thiéno[3,2-*d*]pyrimidin-4-ylamino}-cyclopropyl)-propan-2-ol
1.26 {2-[4-(5-tert-butyl-1-méthyl-1H-indol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.27 2-[4-(5-furan-2-yl-1-méthyl-1H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.28 (S)-5-(2-{4-[4-(4,5-dihydrooxazol-2-yl)-phénoxy]-pipéridin-1-yl}-5-oxo-6,7-dihydro-5H-5À4-thiéno[3,2-d]pyrimidin-4-ylamino)-1-méthylpipéridin-2-one
1.29 {2-[4-(5-furan-2-yl-2-méthyl-2H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.30 {2-[4-(1-méthyl-1H-imidazo[4,5-c]pyridin-2-yl)-pipéridin-1-yl]-5-oxo-6,7-dihydro-5H-5λ⁴-thiéno[3,2-d]pyrimidin-4-yl}-(tétrahydropyran-4-yl)-amine
1.31 2-méthoxy-N-{1-[5-oxo-4-(tétrahydropyran-4-ylamino)-6,7-dihydro-5H-5λ⁴-thiéno[3,2-d]pyrimidin-2-yl]-4-phénylpipéridin-4-ylméthyl}-acétamide
1.32 N-cyclopropyl-N-méthyl-4-{1-[5-oxo-4-(tétrahydropyran-4-ylamino)-6,7-dihydro-5H-5λ⁴-thiéno[3,2-d]pyrimidin-2-yl]-pipéridin-4-yll-benzamide
1.33 N-cyclopropyl-N-méthyl-4-{1-[5-oxo-4-(tétrahydropyran-4-ylamino)-6,7-dihydro-5H-5λ4-thiéno[3,2-d]pyrimidin-2-yl]-pipéridin-4-yloxy}-benzamide
1.34 {5-oxo-2-[4-(pyridin-4-yloxy)-pipéridin-1-yl]-6,7-dihydro-5H-5λ4-thiéno[3,2-d]pyrimidin-4-yl}-(tétrahydropyran-4-yl)-amine
1.35 {2-[4-(4-chlorophénoxy)-pipéridin-1-yl]-5-oxo-6,7-dihydro-5H-5λ4-thiéno[3,2-d]pyrimidin-4-yl}-(tétrahydropyran-4-yl)-amine
1.36 (S)-1-méthyl-5-{2-[4-(5-méthyl-4-phényloxazol-2-yl)-pipéridin-1-yl]-5-oxo-6,7-dihydro-5H-5λ4-thiéno[3,2-d]pyrimidin-4-ylaminol-pipéridin-2-one
1.37 (1-{2-[4-(5-méthyl-4-phényloxazol-2-yl)-pipéridin-1-yl]-5-oxo-6,7-dihydro-5H-5λ4-thiéno[3,2-d]pyrimidin-4-ylamino}-cyclopropyl)-méthanol
1.38 (S)-5-{2-[4-(4,5-diphényloxazol-2-yl)-pipéridin-1-yl]-5-oxo-6,7-dihydro-5H-5λ4-thiéno[3,2-d]pyrimidin-4-ylamino}-1-méthylpipéridin-2-one
1.39 {4-(4-chlorophényl)-1-[5-oxo-4-(tétrahydropyran-4-ylamino )-6,7-dihydro-5H-5λ4-thiéno[3,2-d]pyrimidin-2-yl]-pipéridin-4-yl}-méthanol
1.40 [1-(2-{4-[5-(4-chlorophényl)-4-méthyloxazol-2-yl]-pipéridin-1-yl}-5-oxo-6,7-dihydro-5H-5λ4-thiéno[3,2-d]pyrimidin-4-ylamino)-cyclopropyl]-méthanol
1.41 4-(4-chlorophényl)-1-[5-oxo-4-(tétrahydropyran-4-ylamino)-6,7-dihydro-5H-5λ4-thiéno[3,2-d]pyrimidin-2-yl]-pipéridin-4-ol
1.42 {2-[4-(4-chlorophényl)-4-methoxypipéridin-1-yl]-5-oxo-6,7-dihydro-5H-5λ4-thiéno[3,2-d]pyrimidin-4-yl}-(tétrahydropyran-4-yl)-amine
1.43 4-{1-[4-(1-hydroxyméthylcyclopropylamino)-5-oxo-6,7-dihydro-5H-5λ4-thiéno[3,2-d]pyrimidin-2-yl]-pipéridin-4-yloxy}-benzonitrile
1.44 5-oxo-2-[4-(4,5,6,7-tétrahydrobenzoxazol-2-yl)-pipéridin-1-yl]-6,7-dihydro-5H-5λ4-thiéno[3,2-d]pyrimidin-4-yl}-(tétrahydropyran-4-yl)-amine
1.45 (S)-5-{2-[4-(4-chlorophényl)-pipéridin-1-yl]-5,5-dioxo-6,7-dihydro-5H-5λ6-thiéno[3,2-d]pyrimidin-4-ylamino}-1-méthylpipéridin-2-one
au moins un AINS (**2**) choisi dans le groupe constitué du celecoxib (2.25), étoricoxib (2.41), lumiracoxib (2.74), parecoxib (2.88), rofecoxib (2.101) et valdecoxib (2.125), acide acétylsalicylique (2.3), méloxicam (2.77), naproxène (2.82) et piroxicam (2.93).

19. Combinaison de médicaments selon l'une des revendications 1 à 19, **caractérisée en ce qu'**elle comprend, outre un inhibiteur de PDE4 de formule générale **1,**un AINS **(2)** choisi dans le groupe constitué du celecoxib (2.25), étoricoxib (2.41), lumiracoxib (2.74), parécoxib (2.88), rofecoxib (2.101) et valdecoxib (2.125).

20. Combinaison de médicaments selon l'une des revendications 1 à 19, **caractérisée en ce que** l'on utilise comme AINS **(2),** soit l'acide acétylsalicylique (2.3) en une dose unitaire de 50 à 2000 mg, le méloxicam (2.77) en une dose unitaire de 7,5 mg à 30 mg et le naproxène en une dose unitaire de 250 à 1000 mg.

21. Combinaison de médicaments selon l'une des revendications 1 à 20,
pour son utilisation dans le traitement d'une maladie choisie dans le groupe consistant en les maladies des voies respiratoires, maladies pulmonaires, les troubles gastro-intestinaux, maladies telles qu'également les maladies inflammatoires des articulations, de la peau ou des yeux, les cancers et les maladies du système nerveux périphérique ou central.

22. Combinaison de médicaments selon la revendication 21, pour son utilisation dans le traitement d'une maladie choisie parmi la BPCO, la sinusite chronique, l'asthme, la maladie de Crohn, la fibrose pulmonaire idiopathique et la rectocolite hémorragique.
